# EUROPEAN PATENT APPLICATION

(11) **EP 4 382 524 A1**
(43) Date of publication of application: **12.06.2024**
(21) Application number: 22853374.1
(22) Date of filing: 29.07.2022
(51) Int. Cl.: C07D 405/12, A61K 31/404, A61K 31/454, C07D 209/08, C07D 409/12, C07D 401/12

(54) **INDOLE DERIVATIVE, METHOD FOR PREPARING SAME, AND USE THEREOF**

(30) Priority: 02.08.2021 KR 20210101379
(71) Applicant: MitoImmune Therapeutics Inc., Seoul 06123 (KR)
(72) Inventor: KIM, Soon Ha, Seoul 06123 (KR); CHANG, Hye Kyung, Seoul 06123 (KR); YOO, Eun Kyung, Seoul (KR); JANG, Kyung Kuk, Seoul 06123 (KR); SEO, Mooyoung, Seoul 06123 (KR); PARK, Jin Sung, Seoul 06123 (KR); YANG, Jeong-Hee, Seoul 06123 (KR); PARK, Hyunjun, Seoul 06123 (KR); PARK, Jeong Hyang, Seoul 06123 (KR); PARK, Yun Min, Seoul 06123 (KR); KIM, Jae Young, Seoul 06123 (KR); YUN, Yewon, Seoul 06123 (KR)
(74) Representative: Plasseraud IP
(86) International application number: PCT/KR2022/011213
(87) International publication number: WO 2023/013996

(57) **Abstract**

The present invention relates to a compound of Formula 1, a preparation method thereof, a pharmaceutical composition comprising the same as an active ingredient, and a use thereof. The compound of Formula 1 according to the present invention can exhibit cell necrosis and ferroptosis inhibitory efficacy in various cells such as heart, kidney, nerve, retina, liver, or lung cells, and accordingly, can be usefully used for prevention or treatment of cell necrosis or ferroptosis-related diseases

## Description

### [Technical Field]

The present invention relates to a compound of Formula 1, an isomer thereof, a solvate thereof, a hydrate thereof, or a pharmaceutically acceptable salt thereof, a method for preparing the same, a pharmaceutical composition comprising the same, and a use thereof.

### [Background Art]

Cell death contributes to the maintenance and change of cellular functions for the survival of living organisms, or contributes to maintaining the number of cells in balance with the proliferation of cells for embryonic development, and is also used as a method to effectively and quickly inform the immune system of various danger signals generated from outside, such as invasion of viruses or bacteria in the immune system, and local injury.

However, cell death due to external hazards or local injury can cause local or systemic inflammation due to excessive activation of the immune system, leading to a situation in which living organisms are harmed. Cell death in which this phenomenon occurs is mainly observed in necrotic cell death (or necrosis), and researchers have conducted many studies for disease treatment and improvement by preventing cell necrosis.

The cell necrosis is usually the cell death characterized by oxygen and energy depletion, cell membrane collapse, and the like due to sudden physical and chemical shocks applied to cells, and simultaneously, injury-related substances (DAMPs, damage associated with molecular patterns) such as ATP (adenosine triphosphate), Ca²⁺ (calcium ion), ROS (reactive oxygen species) and HMGB1 (high mobility group box 1) are also released, where these substances are known to induce chained inflammatory reactions and cell death by stimulating or damaging surrounding cells. This cell necrosis is non-regulated cell death (NRCD), and is distinguished from apoptosis, which is regulated cell death (RCD) regulated by caspases.

In the past, a lot of research had been done on apoptosis, which is regulated cell death, in the study of cell death for the treatment of diseases, and pharmaceutical companies had also made great efforts to develop caspase inhibitors, but few drugs have been approved by the FDA. Apoptosis is highly likely to be cell death that occurs to maintain homeostasis in the body, whereas cell necrosis is cell death that occurs mainly in pathological conditions. Accordingly, research on inhibition of cell necrosis is very important in disease prevention and treatment.

Recently, as research on cell death has been actively conducted, regulated necrotic cell death (RNCD), which is distinct from cell necrosis, has been newly identified as ferroptosis.

Ferroptosis is characterized by the accumulation of lipid peroxides and iron involvement. Cells normally receive cystine through system Xc⁻ to maintain the concentration of glutathione, and activate GPX4 (glutathione peroxidase 4) to activate an antioxidation system for removing intracellular lipid peroxidation substances. However, when the glutathione concentration is low or the GPX4 does not function, the cell death occurs by inducing damage to lipids, proteins, nucleic acids, and the like, and cell membrane collapse due to the accumulation of lipid peroxides. It is known that as another factor in the accumulation of lipid peroxides, the ROS generated by the binding of iron ions with intracellular free radicals by the Fenton reaction are involved. The cell death by the lipid peroxide has also an aspect similar to cell necrosis, where a large number of DAMPs are released to the outside of the cells and cause inflammation and death of surrounding cells.

Therefore, it has been frequently reported that ferroptosis is observed in pathological conditions similar to cell necrosis, and inhibition of ferroptosis is very important for disease prevention and treatment.

As representative diseases that cell necrosis, which is non-regulated cell death, and ferroptosis, which is regulated necrotic cell death, appear, ischemic disease (e.g., myocardial infarction, stroke, renal infarction), neurodegenerative disease, and inflammatory disease, aging, macular degeneration and lung disease, and the like have been reported. Accordingly, the discovery and the development of substances that inhibit cell necrosis and ferroptosis in disease research and treatment are also very urgent.

Meanwhile, 5-[(1,1-dioxido-4-thiomorpholinyl)methyl]-2-phenyl-*N*-(tetrahydro-2*H-*pyran-4-yl)-1*H*-indol-7-amine is a compound disclosed through International Patent Publication No. WO2009-025478, which is a material known to exhibit preventive or therapeutic and ameliorating effects on cell necrosis and related diseases. Indole derivatives such as the above compound have pharmaceutically very useful structures, and many studies on these structures are in progress.

The present inventors conducted a study on whether ferroptosis, which is regulated necrotic cell death similar to cell necrosis, shows pharmaceutically similar efficacy to indole derivatives such as the above compound, and confirmed their effects. Accordingly, the present inventors have continuously conducted research on various compounds exhibiting the effects of preventing or treating and ameliorating diseases related to non-regulated cell necrosis, and ferroptosis, which is regulated necrotic cell death, thereby synthesizing novel compounds, and completing the present invention by confirming their effects.

### [Prior Art Documents]

### [Patent Documents]

International Patent Publication No. WO2009-025478

### [Disclosure]

### [Technical Problem]

It is an object of the present invention to provide a novel compound of Formula 1, an isomer thereof, a solvate thereof, a hydrate thereof, or a pharmaceutically acceptable salt thereof.

Also, it is an object of the present invention to provide a pharmaceutical composition for preventing or treating cell necrosis or ferroptosis-related diseases comprising a compound of Formula 1, an isomer thereof, a solvate thereof, a hydrate thereof, or a pharmaceutically acceptable salt thereof as an active ingredient together with a pharmaceutically acceptable carrier.

In addition, it is an object of the present invention to provide a method for preparing a compound of Formula 1, and a method for preventing or treating cell necrosis or ferroptosis-related diseases.

Furthermore, it is an object of the present invention to provide a method for inhibiting ferroptosis, and a method for inhibiting reactive oxygen species.

### [Technical Solution]

The present invention provides a compound of Formula 1 below, an isomer thereof, a solvate thereof, a hydrate thereof, or a pharmaceutically acceptable salt thereof.

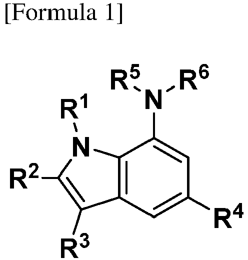

In Formula 1 above,
R¹ is hydrogen or C₁₋₈ alkyl,
R² is hydrogen, aryl with 5 to 10 atoms, heteroaryl with 5 to 10 atoms, C₁₋₈ alkyl, or C₁₋₈ alkoxy, and R² is unsubstituted or substituted with halogen,
R³ is hydrogen, nitrile, aryl with 5 to 10 atoms, heteroaryl with 5 to 10 atoms, C₁₋₈ alkyl, C₁₋₈ alkoxy, -C(=O)-X, -CH(OH)-X, -CH=CH-C(=O)-X, -CH₂CH₂-C(=O)-X, or - CH=N-X, where the aryl with 5 to 10 atoms, heteroaryl with 5 to 10 atoms, or C₁₋₈ alkyl in R³ is unsubstituted or substituted with a substituent Y,
the substituents X and Y are each independently one or more selected from the group consisting of halogen, hydroxy, C₁₋₈ alkyl, C₁₋₈ haloalkyl, C₁₋₈ alkoxy, C₁₋₃ ester, aryl with 5 to 10 atoms, and heteroaryl with 5 to 10 atoms,
R⁴ is -C₁₋₈ alkylene-O-R⁷, where
R⁷ is C₁₋₈ alkyl or -(CH₂)ₘ-C₃₋₁₀ cycloalkyl, and m is an integer of 1 to 4,
R⁵ and R⁶ are each independently hydrogen, C₁₋₈ alkyl, C₁₋₈ alkoxy, C₃₋₁₀ cycloalkyl, - (CH₂)ₚ-C₃₋₁₀ cycloalkyl, C₃₋₁₀ heterocycloalkyl, -(CH₂)ₚ-C₃₋₁₀ heterocycloalkyl, aryl with 5 to 10 atoms, benzyl or heteroaryl with 5 to 10 atoms, where p is an integer of 1 to 4, and
R⁵ and R⁶ are unsubstituted or substituted with one or more substituents R⁸ selected from the group consisting of halogen, C₁₋₈ alkyl, C₁₋₈ alkoxy, C₁₋₈ haloalkyl, oxo (=O), -C(=O)-C₁₋₈ alkyl, -C(=O)OC₁₋₈ alkyl and -S(=O)₂C₁₋₈ alkyl.

In the definition of a substituent according to the present invention, the term 'alkyl' refers to an aliphatic hydrocarbon radical. An alkyl may be a "saturated alkyl" which does not include an alkenyl or alkynyl region, or may be an "unsaturated alkyl" which includes at least one alkenyl or alkynyl region.

"Alkenyl" refers to a group including at least one carbon-carbon double bond, and "alkynyl" refers to a group including at least one carbon-carbon triple bond. The alkenyl and alkynyl may be a branched or straight chain type, respectively.

An alkyl group, unless otherwise defined, may have 1 to 20 carbon atoms. An alkyl group may be a medium-sized alkyl having 1 to 10 carbon atoms. An alkyl group may be a lower alkyl having 1 to 6 carbon atoms. Whereas typical alkyl groups include methyl, ethyl, propyl, isopropyl, butyl, isobutyl, t-butyl, pentyl, hexyl, ethenyl, propenyl and butenyl, and the like, an alkyl group is not limited to these. For example, a C₁₋₄alkyl has 1 to 4 carbon atoms in its alkyl chain, and is selected from a group comprised of methyl, ethyl, propyl, iso-propyl, n-butyl, iso-butyl, sec-butyl and t-butyl.

The term 'alkylene' refers to a hydrocarbon bivalent group wherein radicals are additionally formed in the alkyl described above, and non-limiting examples include methylene, ethylene, propylene, butylene and isobutylene.

The term 'alkoxy', unless otherwise defined, refers to an alkyloxy, and non-limiting examples include methoxy, ethoxy and propoxy.

The term "haloalkyl" may mean -RX(X is one or more halogen atoms(F, Cl, Br and I)), that is, "haloalkyl" may be an alkyl form in which one or more halogens are substituted. For example, non-limiting examples of "C₁₋₈ haloalkyl" may include trifluoromethyl, or difluoromethyl.

The term 'cycloalkyl', unless otherwise defined, refers to a saturated or unsaturated aliphatic ring. Further, the number of atoms forming the ring may be 3 to 12. Non-limiting examples of typical cycloalkyl groups include cyclopropyl, cyclobutyl, cyclopentyl and cyclohexyl.

The term `heterocycloalkyl', unless otherwise defined, refers to a cycloalkyl as defined above which includes 1 to 3 heteroatoms selected from a group comprised of N, O and S. A heterocycloalkyl may be monocyclic, or may be multicyclic, such as spirocyclic, bridged cyclic or fused cyclic. Non-limiting examples of heterocycloalkyl include pyrrolidine, piperidine, tetrahydropyran, oxetane, thiopyran and groups similar to these.

The term 'aryl' includes at least one ring having a covalent π electron system, for example, monocyclic or fused polycyclic (i.e., cycles that share the adjacent carbon atom pairs) groups. That is, in the present specification, aryl means an aromatic 4-10 membered, preferably 6-10 membered, monocyclic or multicyclic ring including phenyl, naphthyl, and the like, unless otherwise defined.

The term 'heteroaryl', unless otherwise defined, refers to an aromatic 3-10 membered, preferably 4-8 membered, more preferably 5-6 membered cycle that has 1 to 3 hetero atoms selected from N, O and S, and may be fused with benzo or C₃₋₈ cycloalkyl. The monocyclic heteroaryl includes, but not limited to, thiazole, oxazole, thiophene, furan, pyrrole, imidazole, isoxazole, isothiazole, pyrazole, triazole, triazine, thiadiazole, tetrazole, oxadiazole, pyridine, pyridazine, pyrimidine, pyrazine and the like. Non-limiting examples of bicyclic heteroaryl include indole, indoline, benzothiophene, benzofuran, benzimidazole, benzoxazole, benzisoxazole, benzthiazole, benzthiadiazole, benztriazole, quinoline, isoquinoline, purine, puropyridine and groups similar thereto.

The term 'heterocycle', unless otherwise defined, refers to a 3-10 membered, preferably 4-8 membered, more preferably 5-6 membered cycle that has 1 to 3 hetero atoms selected from a group comprised of N, O and S, which may be fused with benzo or C₃₋₈ cycloalkyl, and is saturated or contains 1 or 2 double bonds. Non-limiting examples of heterocycle include pyrroline, pyrrolidine, imidazoline, imidazolidine, pyrazoline, pyrazolidine, pyran, piperidine, morpholine, thiomorpholine, piperazine, and hydrofuran.

Other terms and abbreviations used in the present specification, unless defined otherwise, may be interpreted to have the meanings ordinarily understood by a person having ordinary skill in the art.

In this specification, the "hydrate" may mean a compound of the present invention containing a stoichiometric or non-stoichiometric amount of water bound by non-covalent intermolecular forces, or a salt thereof. The hydrate of the compound of the present invention represented by Formula 1 may include a stoichiometric or non-stoichiometric amount of water that is bound by non-covalent intermolecular forces. The hydrate may contain 1 equivalent or more, preferably, 1 to 5 equivalents of water. Such a hydrate may be prepared by crystallizing the compound of the present invention represented by Formula 1, an isomer thereof, or a pharmaceutically acceptable salt thereof from water or a solvent containing water.

In this specification, the "solvate" may mean a compound of the present invention containing a stoichiometric or non-stoichiometric amount of a solvent bound by non-covalent intermolecular forces, or a salt thereof. Preferred solvents therefor include solvents that are volatile, non-toxic, and/or suitable for administration to humans.

In this specification, the "isomer" may mean a compound of the present invention that has the same chemical formula or molecular formula but is structurally or sterically different, or a salt thereof. Such isomers include all structural isomers such as tautomers, R or S isomers having an asymmetric carbon center, stereoisomers such as geometric isomers (trans, cis), and optical isomers (enantiomers). All these isomers and mixtures thereof are also included within the scope of the present invention.

The compound of Formula 1 may be a compound represented by Formula 2 below.

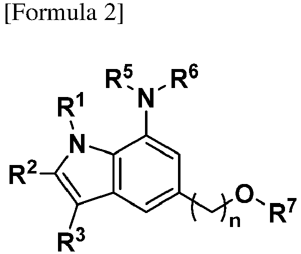

In the above formula, R¹ to R³, and R⁵ to R⁷ are each as defined herein, and n may be an integer of 1 to 4.

According to one aspect of the compound of Formula 1,
R¹ may be hydrogen or C₁₋₆ alkyl,
R² may be hydrogen, aryl with 5 to 8 atoms, heteroaryl with 5 to 8 atoms containing one or more heteroatoms of N, O, and S, C₁₋₆ alkyl, or C₁₋₆ alkoxy, and R² may be unsubstituted or substituted with halogen,
R³ may be hydrogen, nitrile, aryl with 5 to 8 atoms, heteroaryl with 5 to 8 atoms containing one or more heteroatoms of N, O, and S, C₁₋₆ alkyl, C₁₋₆ alkoxy, -C(=O)-X, - CH(OH)-X, -CH=CH-C(=O)-X, -CH₂CH₂-C(=O)-X, or -CH=N-X, where the aryl with 5 to 8 atoms, heteroaryl with 5 to 8 atoms, or C₁₋₆ alkyl in R³ may be unsubstituted or substituted with a substituent Y,
the substituents X and Y may be each independently one or more selected from the group consisting of halogen, hydroxy, C₁₋₈ alkyl, C₁₋₆ haloalkyl, C₁₋₆ alkoxy, C₁₋₃ ester, aryl with 5 to 8 atoms, and heteroaryl with 5 to 8 atoms containing one or more heteroatoms of N, O, and S,
R⁴ may be -C₁₋₈ alkylene-O-R⁷, where
R⁷ may be C₁₋₈ alkyl or -(CH₂)ₘ-C₃₋₇ cycloalkyl, and m may be an integer of 1 to 4,
R⁵ and R⁶ may be each independently hydrogen, C₁₋₆ alkyl, C₁₋₆ alkoxy, C₃₋₇ cycloalkyl, -(CH₂)ₚ-C₃₋₇ cycloalkyl, C₃₋₇ heterocycloalkyl, -(CH₂)ₚ-C₃₋₇ heterocycloalkyl, aryl with 5 to 8 atoms, benzyl, or heteroaryl with 5 to 8 atoms containing one or more heteroatoms of N, O, and S, where p may be an integer of 1 to 4, and
R⁵ and R⁶ may be unsubstituted or substituted with one or more substituents R⁸ selected from the group consisting of halogen, C₁₋₆ alkyl, C₁₋₆ alkoxy, C₁₋₆ haloalkyl, oxo (=O), -C(=O)-C₁₋₆ alkyl, -C(=O)OC₁₋₆ alkyl, and -S(=O)₂C₁₋₆ alkyl.

In the compound of Formula 1, R¹ may be hydrogen or C₁₋₆ alkyl, and may be, preferably, hydrogen or C₁₋₃ alkyl.

In one embodiment, when R¹ is C₁₋₈ alkyl, R³ may be C₁₋₈ alkyl substituted with C₁₋₈ alkoxy.

In the compound of Formula 1, R² may be hydrogen, aryl with 5 to 10 atoms, heteroaryl with 5 to 10 atoms, C₁₋₈ alkyl, or C₁₋₈ alkoxy, and R² may be unsubstituted or substituted with halogen. Specifically, R² may be unsubstituted aryl with 5 to 10 atoms, and more specifically, may be phenyl, particularly, unsubstituted phenyl.

In one embodiment, R² may be hydrogen, and R³ may be aryl with 5 to 10 atoms.

In the compound of Formula 1, R³ may be hydrogen, nitrile, aryl with 5 to 10 atoms, heteroaryl with 5 to 10 atoms, C₁₋₈ alkyl, C₁₋₈ alkoxy, -C(=O)-X, -CH(OH)-X, -CH=CH-C(=O)-X, -CH₂CH₂-C(=O)-X, or -CH=N-X, where the aryl with 5 to 10 atoms, heteroaryl with 5 to 10 atoms, or C₁₋₈ alkyl in R³ may be unsubstituted or substituted with the substituent Y.

In one embodiment, R³ may be hydrogen, nitrile, aryl with 5 to 10 atoms, heteroaryl with 5 to 10 atoms containing one or more heteroatoms of N, O, and S, C₁₋₆ alkyl, C₁₋₆ alkoxy, -C(=O)-X, -CH(OH)-X, -CH=CH-C(=O)-X, -CHCH₂-C(=O)-X, or -CH=N-X, where the aryl with 5 to 10 atoms, heteroaryl with 5 to 10 atoms containing one or more heteroatoms of N, O, and S, or C₁₋₆ alkyl in R³ may be unsubstituted or substituted with the substituent Y, and
the substituents X and Y may be each independently one or more selected from the group consisting of halogen, hydroxy, C₁₋₆ alkyl, C₁₋₆ haloalkyl, C₁₋₆ alkoxy, C₁₋₃ ester, aryl with 5 to 10 atoms, and heteroaryl with 5 to 10 atoms containing one or more heteroatoms of N, O, and S.

In one embodiment, when R³ is aryl with 5 to 10 atoms or heteroaryl with 5 to 10 atoms, R³ may be unsubstituted or substituted with the substituent Y of C₁₋₃ ester.

In one embodiment, when R³ is C₁₋₈ alkyl, R³ may be unsubstituted or substituted with one or more substituents Y selected from hydroxy, C₁₋₈ haloalkyl, and C₁₋₈ alkoxy.

In one embodiment, when R³ is -C(=O)-X, X may be C₁₋₈ haloalkyl.

In one embodiment, when R³ is -CH=CH-C(=O)-X, X may be C₁₋₈ alkoxy, or aryl with 5 to 10 atoms.

In one embodiment, when R³ is -CH₂CH₂-C(=O)-X, X may be C₁₋₈ alkoxy, or hydroxy.

In one embodiment, when R³ is -CH=N-X, X may be C₁₋₈ alkoxy.

In the compound of Formula 1, R⁴ may be -C₁₋₈ alkylene-O-R⁷, and R⁷ may be C₁₋₈ alkyl or -(CH₂)ₘ-C₃₋₁₀ cycloalkyl. Here, m may be an integer of 1 to 4.

In one embodiment, R⁴ may be -C₁₋₈ alkylene-O-R⁷, and R⁷ may be C₁₋₈ alkyl or - (CH₂)ₘ-C₃₋₁₀ cycloalkyl, and more specifically, may be C₁₋₈ alkyl or -CH₂-cyclopropyl.

In the compound of Formula 1, R⁵ and R⁶ may be each independently hydrogen, C₁₋₈ alkyl, C₁₋₈ alkoxy, C₃₋₁₀ cycloalkyl, C₃₋₁₀ heterocycloalkyl, -(CH₂)ₚ-C₃₋₁₀ heterocycloalkyl, aryl with 5 to 10 atoms, benzyl or heteroaryl with 5 to 10 atoms, and p may be an integer of 1 to 4.

In one embodiment, R⁵ and R⁶ may be each independently hydrogen, C₁₋₆ alkyl, C₁₋₆ alkoxy, C₃₋₇ cycloalkyl, C₃₋₇ heterocycloalkyl, -(CH₂)ₚ-C₃₋₇ heterocycloalkyl, aryl with 5 to 8 atoms, benzyl or heteroaryl with 5 to 8 atoms, where p may be an integer of 1 to 4, and
more specifically, R⁵ and R⁶ may be each independently hydrogen, C₁₋₃ alkyl, C₁₋₃ alkoxy, C₃₋₇ cycloalkyl, C₃₋₇ heterocycloalkyl, -(CH₂)ₚ-C₃₋₇ heterocycloalkyl, aryl with 5 to 8 atoms, benzyl or heteroaryl with 5 to 8 atoms, where p may be an integer of 1 to 4, and the heterocycloalkyl and heteroaryl may contain one or more heteroatoms of N, O, and S.

In one embodiment, any one of R⁵ and R⁶ may be hydrogen, and the other may be C₁₋₈ alkyl, C₁₋₈ alkoxy, C₃₋₁₀ cycloalkyl, C₃₋₁₀ heterocycloalkyl, -(CH₂)ₚ-C₃₋₁₀ heterocycloalkyl, aryl with 5 to 10 atoms, benzyl, or heteroaryl with 5 to 10 atoms. Here, the heterocycloalkyl and heteroaryl may contain one or more heteroatoms of N, O and S.

In another embodiment of R⁵ and R⁶, R⁵ and R⁶ may simultaneously be C₃₋₇ cycloalkyl, particularly, unsubstituted cyclopentyl.

In one embodiment, R⁵ and R⁶ may be each independently hydrogen, tetrahydropyran, cyclopentyl, -(CH₂)ₚ-tetrahydropyranyl, C₁₋₈ alkyl, -(CH₂)ₚ-cyclopentyl, -(CH₂)ₚ-cyclopropyl, cyclohexyl, benzyl, piperidinyl, or thianyl.

In the compound of Formula 1, R⁵ and R⁶ may be unsubstituted or substituted with one or more substituents R⁸ selected from the group consisting of halogen, C₁₋₈ alkyl, C₁₋₈ alkoxy, C₁₋₈ haloalkyl, oxo (=O), -C(=O)-C₁₋₈ alkyl, -C(= O)OC₁₋₈ alkyl and -S(=O)₂C₁₋₈ alkyl.

In one embodiment, the substituent R⁸ may be selected from the group consisting of halogen, C₁₋₆ alkyl, C₁₋₆ alkoxy, C₁₋₆ haloalkyl, oxo (=O), -C(=O)-C₁₋₆ alkyl, -C(=O)OC₁₋₆ alkyl and -S(=O)₂C₁₋₆ alkyl, and more specifically, may be selected from the group consisting of halogen, C₁₋₃ alkyl, C₁₋₃ alkoxy, C₁₋₃ haloalkyl, oxo(=O), -C(=O)-C₁₋₃ alkyl, -C(=O)OC₁₋₃ alkyl and -S(=O)₂C₁₋₃ alkyl.

In one embodiment, when one or more of R⁵ and R⁶ are C₃₋₁₀ cycloalkyl or C₃₋₁₀ heterocycloalkyl, the substituent R⁸ may be selected from the group consisting of halogen, C₁₋₈ alkyl, oxo (=O), -C(=O)-C₁₋₈ alkyl, -C(=O)OC₁₋₈ alkyl and -S(=O)₂C₁₋₈ alkyl; or when one or more of R⁵ and R⁶ is C₁₋₈ alkyl, the substituent R⁸ may be C₁₋₈ alkoxy.

In one embodiment, when one or more of R⁵ and R⁶ are C₃₋₁₀ heterocycloalkyl containing a heteroatom of N, the substituent R⁸ may be selected from the group consisting of -C(=O)-C₁₋₆ alkyl, -C(=O)OC₁₋₆ alkyl and -S(=O)₂C₁₋₆ alkyl, or when one or more of R⁵ and R⁶ are C₃₋₁₀ heterocycloalkyl containing a heteroatom of S, the substituent R⁸ may be oxo (=O).

In one embodiment, R⁵ or R⁶ may be C₁₋₈ alkyl, or C₁₋₈ alkoxy, R² may be aryl with 5 to 10 atoms, and R¹ may be hydrogen.

In one embodiment, R¹ may be C₁₋₈ alkyl, R² may be aryl with 5 to 10 atoms, and R³ may be C₁₋₆ alkyl substituted with the substituent Y of C₁₋₈ alkoxy.

The compound of Formula 1 according to the present invention may be any one selected from the flowing compound group:
<1>5-(methoxymethyl)-2-phenyl-N-tetrahydropyran-4-yl-1H-indol-7-amine; <2>N-cyclopentyl-5-(methoxymethyl)-2-phenyl-1H-indol-7-amine; <3>5-(methoxymethyl)-2-phenyl-N-(tetrahydropyran-4-ylmethyl)-1H-indol-7-amine; <4>N-(1,1-dioxothian-4-yl)-5-(methoxymethyl)-2-phenyl-1H-indol-7-amine; <5>5-(methoxymethyl)-N-(1-methylsulfonyl-4-piperidyl)-2-phenyl-1H-indol-7-amine; <6>1-[4-[[5-(methoxymethyl)-2-phenyl-1H-indol-7-yl]amino]-1-piperidyl]ethanone; <7>N-(4,4-difluorocyclohexyl)-5-(methoxymethyl)-2-phenyl-1H-indol-7-amine; <8>N-benzyl-5-(methoxymethyl)-2-phenyl-1H-indol-7-amine; <9>tert-butyl 4-[[5-(methoxymethyl)-2-phenyl-1H-indol-7-yl]amino-piperidine-1-carboxylate; <10> N-isopropyl-5-(methoxymethyl)-2-phenyl-1H-indol-7-amine; <11>5-(methoxymethyl)-N-(1-methyl-4-piperidyl)-2-phenyl-1H-indol-7-amine; <12>N-(2-methoxyethyl)-5-(methoxymethyl)-2-phenyl-1H-indol-7-amine; <13>5-(methoxymethyl)-N-(3-methoxypropyl)-2-phenyl-1H-indol-7-amine; <14>5-(methoxymethyl)-N-(1-oxothian-4-yl)-2-phenyl-1H-indol-7-amine; <15>5-(tert-butoxymethyl)-2-phenyl-N-tetrahydropyran-4-yl-1H-indol-7-amine; <16>5-(tert-butoxymethyl)-N-cyclopentyl-2-phenyl-1H-indol-7-amine; <17>5-(tert-butoxymethyl)-N,N-dicyclopentyl-2-phenyl-1H-indol-7-amine; < 18 > 5 - (methoxymethyl)-3-phenyl-N-tetrahydropyran-4-yl-1H-indol-7-amine; <19>N-cyclopentyl-5-(ethoxymethyl)-2-phenyl-1H-indol-7-amine; <20>5-(ethoxymethyl)-2-phenyl-N-(tetrahydro-2H-pyran-4-yl)-1H-indol-7-amine; <21>N-cyclopentyl-5-(isopropoxymethyl)-2-phenyl-1H-indol-7-amine; <22>5-(isopropoxymethyl)-2-phenyl-N-(tetrahydro-2H-pyran-4-yl)-1H-indol-7-amine; <23>7-(cyclopentylamino)-5-(ethoxymethyl)-2-phenyl-1H-indole-3-carbonitrile; <24>5-(ethoxymethyl)-2-phenyl-7-((tetrahydro-2H-pyran-4-yl)amino)-1H-indole-3-carbonitrile; <25>(E)-7-(cyclopentylamino)-5-(ethoxymethyl)-2-phenyl-1H-indole-3-carbaldehyde O-methyl oxime; <26>(E)-7-(bis(tetrahydro-2H-pyran-4-yl)amino)-5-(ethoxymethyl)-2-phenyl-1H-indole-3-carbaldehyde O-methyl oxime; <27>(E)-5-(ethoxymethyl)-2-phenyl-7-(tetrahydro-2H-pyran-4-yl)amino)-1H-indole-3-carbaldehyde O-methyl oxime; <28>N-cyclopentyl-5-(ethoxymethyl)-3-(methoxymethyl)-1-methyl-2-phenyl-1H-indol-7-amine; <29>5-(ethoxymethyl)-3-(methoxymethyl)-1-methyl-2-phenyl-N-(tetrahydro-2H-pyran-4-yl)-1H-indol-7-amine; <30>methyl (E)-3-(7-(cyclopentylamino)-5-(ethoxymethyl)-2-phenyl-1H-indol-3-yl)acrylate; <31>methyl (E)-3-(5-(ethoxymethyl)-2-phenyl-7-((tetrahydro-2H-pyran-4-yl)amino)-1H-indol-3-yl)acrylate; <32>1-(7-(cyclopentylamino)-5-(ethoxymethyl)-2-phenyl-1H-indol-3-yl)-2,2,2-trifluoroethan-1-one; <33>1-(5-(ethoxymethyl)-2-phenyl-7-((tetrahydro-2H-pyran-4-yl)amino)-1H-indol-3-yl)-2,2,2-trifluoro-1-one; <34>(E)-3-(7-(cyclopentylamino)-5-(ethoxymethyl)-2-phenyl-1H-indol-3-yl)-1-phenylprop-2-en-1-one; <35>(E)-3-(5-(ethoxymethyl)-2-phenyl-7-((tetrahydro-2H-pyran-4-yl)amino)-1H-indol-3-yl)-1-phenylprop-2-en-1-one; <36>1-(7-(cyclopentylamino)-5-(ethoxymethyl)-2-phenyl-1H-indol-3-yl)-2,2,2-trifluoroethan-1-ol; <37>1-(5-(ethoxymethyl)-2-phenyl-7-((tetrahydro-2H-pyran-4-yl)amino)-1H-indol-3-yl)-2,2,2-trifluoro-1-ol; <38>methyl 3-(7-(cyclopentylamino)-5-(ethoxymethyl)-2-phenyl-1H-indol-3-yl)propanoate; <39>methyl 3-(5-(ethoxymethyl)-2-phenyl-7-((tetrahydro-2H-pyran-4-yl)amino)-1H-indol-3-yl)propanoate; <40>3-(7-(cyclopentylamino)-5-(ethoxymethyl)-2-phenyl-1H-indol-3-yl)propanoic acid; <41>5-(2-methoxyethyl)-2-phenyl-N-(tetrahydro-2H-pyran-4-yl)-1H-indol-7-amine; <42> N-cyclopentyl-5-(2-methoxyethyl)-2-phenyl-1H-indol-7-amine; <43>ethyl 2-(7-(cyclopentylamino)-5-(ethoxymethyl)-2-phenyl-1H-indol-3-yl)-1H-imidazole-1-carboxylate; <44>ethyl 2-(5-(ethoxymethyl)-2-phenyl-7-((tetrahydro-2H-pyran-4-yl)amino)-1H-indol-3-yl)-1H-imidazole-1-carboxylate; <45>N-cyclopentyl-5-(ethoxymethyl)-3-(1H-imidazol-2-yl)-2-phenyl-1H-indol-7-amine; <46>5-(ethoxymethyl)-3-(1H-imidazol-2-yl)-2-phenyl-N-(tetrahydro-2H-pyran-4-yl)-1H-indol-7-amine; <47>2-phenyl-5-(propoxymethyl)-N-(tetrahydro-2H-pyran-4-yl)-1H-indol-7-amine; <48>5-((cyclopropylmethoxy)methyl)-2-phenyl-N-(tetrahydro-2H-pyran-4-yl)-1H-indol-7-amine; <49>5-(ethoxymethyl)-2-phenyl-N-((tetrahydro-2H-pyran-4-yl)methyl)-1H-indol-7-amine; <50>5-(3-methoxypropyl)-2-phenyl-N-(tetrahydro-2H-pyran-4-yl)-1H-indol-7-amine; and <51>5-(3-ethoxypropyl)-2-phenyl-N-(tetrahydro-2H-pyran-4-yl)-1H-indol-7-amine.

The present invention further provides a method for preparing the compound of Formula 1. In the following, to help understanding of the present invention, a method for preparing the compound of Formula 1 will be described on the basis of an exemplary reaction formula. However, a person having ordinary skill in the art may prepare the compound of Formula 1 by means of various methods based on the structure of Formula 1, and all such methods shall be interpreted as being included in the scope of the present invention. That is, any combination of various synthesis methods stated in the present specification or disclosed in prior art may be used to prepare the compound of Formula 1, such combinations understood as belonging to the scope of the present invention, and the compound of Formula 1 not limited to that which is described in the following.

First, the method for preparing a compound of Formula 1 may comprise steps of: reacting a compound of Formula 3 and HO-R₇ to prepare a compound of Formula 4; reducing the compound of Formula 4; and preparing the compound of Formula 1 from the reduced compound of Formula 4.

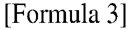

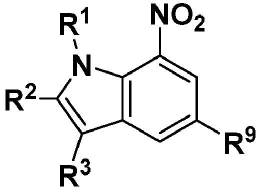

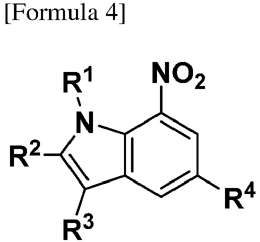

In Formulas above, R¹ to R⁷ are each the same as defined herein, R⁹ is -C₁₋₈ alkylene-LG, and LG is a leaving group. Here, the leaving group may be a functional group such as halogen, sulfonic acid ester, or alkoxy, and is not limited as long as the leaving group may be released from the compound of Formula 3 to prepare the compound of Formula 4.

The step of reacting a compound of Formula 3 and HO-R₇ to prepare a compound of Formula 4 may be a step in which the leaving group of the compound of Formula 3 is substituted by HO-R₇ while using a base to prepare a compound of Formula 4. Here, the base may be Et₃N, DIPEA, DBU, NMP, or K₂CO₃, but is not limited thereto.

The step of reducing the compound of Formula 4 is a step of reducing the nitro group of the compound of Formula 4 to an amine group, which may be carried out using an acid catalyst and a metal, or using a metal catalyst in the presence of hydrogen gas. As the metal, iron, zinc, lithium, sodium, and tin (typically, tin chloride) may be used, and as the acid catalyst, an inorganic acid such as hydrochloric acid, sulfuric acid, nitric acid and phosphoric acid, and the like may be used. Also, in a reduction reaction using a metal catalyst in the presence of hydrogen gas, as the usable metal catalyst, palladium, nickel, platinum, ruthenium, rhodium, and the like may be mentioned, where the pressure of hydrogen gas may be usually 1 to 3 atmospheres.

The step of preparing the compound of Formula 1 from the reduced compound of Formula 4 is a step in which the compound of Formula 4 and a ketone compound or an aldehyde compound are subjected to reductive amination reaction to prepare the compound of Formula 1, where in the reductive amination reaction, NaBH(OAc)₃ or NaBH₃CN may be used, without being limited thereto.

Here, the ketone compound or the aldehyde compound is not limited as long as it is a ketone or aldehyde compound capable of reacting with the amine group of the compound of Formula 4 to form R⁵ and R⁶ substituents as in the compound of Formula 1, and having an oxo (=O) group.

For example, when cyclopentanone is used as the ketone compound, the compound of Formula 1 in which one or more of R⁵ and R⁶ are cyclopentyl may be prepared.

In addition, as long as the reaction proceeds smoothly in each manufacturing step, the conditions may not be limited.

Meanwhile, a method for preparing the compound of Formula 3 is described in WO 2009-025478 A1. For example, the compound of Formula 3 in which the leaving group is a halogen may be prepared by reduction of a carboxylic acid or ester to form a hydroxyalkyl, followed by halogenation of the hydroxy group.

The present invention further provides a pharmaceutical composition comprising the compound of Formula 1, an isomer thereof, a solvate thereof, a hydrate thereof, or a pharmaceutically acceptable salt thereof; and a pharmaceutically acceptable carrier.

A pharmaceutical composition for prevention or treatment of cell necrosis or ferroptosis-related diseases selected from the following group, the composition comprising the compound of Formula 1 of Claim 1, an isomer thereof, a hydrate thereof, a solvate thereof, or a pharmaceutically acceptable salt thereof as an active ingredient, and a pharmaceutically acceptable carrier:
The cell necrosis and ferroptosis-related diseases include acute/chronic hepatic disease (e.g. hepatitis, hepatic fibrosis, hepatocirrhosis), neurodegenerative disease (e.g. dementia, Parkinson's disease, Huntington's disease), ischemic cardiac disease, reperfusion injury, ischemic stroke or ischemic injury, pancreatitis, bacterial/viral sepsis, diabetes mellitus or diabetic complications, diabetic vascular disease [in particular, these diabetes are caused by pancreatic cell destroying substances, and mediated by virus, hyperglycemia, fatty acid, diet, toxin, streptozotocin and the like], necrotizing procolitis, cystic fibrosis, rheumatoid arthritis, degenerative arthritis, nephropathy, bacterial infection, viral infection (e.g. HIV), multiple sclerosis, leukemia, lymphoma, neonatal respiratory distress syndrome, asphyxia, tuberculosis, endometriosis, angiasthenia, psoriasis, chilblain, steroid treatment complications, gangrene, pressure sores, hemoglobinuria, burns, hyperthermia, Crohn's disease, celiac disease, compartment syndrome, spinal cord injury, glomerulonephritis, renal failure, muscular dystrophy, metabolic inherited disease, mycoplasmal disease, anthrax, Andersen's disease, congenital mitochondrial disease, phenylketonuria, placental infarction, syphilis, osteonecrosis and the like. In addition, necrosis and associated diseases caused by drugs and toxic substances are selected from the group consisting of the necrosis associated with alcoholism, the exposure to, and/or administration and/or self-administration of, cocaine, drugs (e.g., paracetamol), antibiotics, anti-cancer agent, Adriamycin, puromycin, bleomycin, NSAID, cyclosporine, chemical toxins (e.g., carbon tetrachloride, cyanide, methanol, ethylene glycol), poison gas, agrochemicals, heavy metals (e.g., lead, mercury, cadmium), or injury due to the exposure to radioactivity/UV and associated necrosis thereof.

Further, the compound of Formula 1 is additionally expected to exhibit preventive or therapeutic and improvement effects in cell necrosis and ferroptosis-related diseases of acute/chronic renal disease, traumatic brain damage, the neurogenerative disease of amyotrophic lateral sclerosis, necrotizing colitis, viral infection (e.g. SARS-CoV), skin disease including psoriasis and allergic dermatitis, and organ preservation / organ transplant (see Korean Registered Patents 10-1098583 and 10-1941004).

Further, a pharmaceutical composition comprising the compound of Formula 1 is able to regulate intracellular calcium, and is able to improve ER stress and mitochondrial dysfunction due to abnormal intracellular calcium levels.

In addition, the compound of Formula 1 can inhibit cell death through ferroptosis caused by accumulation of lipid peroxides, which is indicated by the Fenton reaction, and GPX4 pathway interference by Erastin, glutamate, or RSL3, and the like, which are ferroptosis-inducing substances.

Accordingly, a pharmaceutical composition comprising the compound of Formula 1 is expected to exhibit preventive or therapeutic and improvement effects with regard to cell necrosis and ferroptosis-related diseases. Associated diseases are as follow.

Chronic inflammatory pulmonary disease including acute lung injury syndrome / acute pulmonary disease, pneumonia, tuberculosis, asthma, pulmonary hypertension, chronic obstructive pulmonary disease, idiopathic pulmonary fibrosis and cystic fibrosis (See Mitochondrial dysfunction in fibrotic diseases. Cell Death Discov. 2020 Sep 5;6:80*.* Mitochondrial dysfunction in lung aging and diseases. Eur Respir Rev. 2020 Oct 15;29(157):200165*,* see Korean registered patent 10-1636563)

Demyelinating disease including demyelination and amyotrophic lateral sclerosis (ALS), hypertension including pulmonary hypertension, stroke, prion disease, epilepsy, ataxia, migraine, reduced cognitive skill, seizure, tremors, psychiatric disorders (e.g. depression) (See Neuronal and glial calcium signaling in Alzheimer's disease. Cell Calcium. Oct-Nov 2003;34(4-5):385-97. Mitochondrial disorders: challenges in diagnosis & treatment. Indian J Med Res. 2015 Jan;141(1):13-26.)

Insulin resistance, hyperlipidemia, atherosclerosis, inflammatory bowel disease (IBD) including Crohn's Disease and ulcerative colitis, various cancers and metastasis of cancer (See reticulum stress and oxidative stress in cell fate decision and human disease. Antioxid Redox Signal. 2014 Jul 20;21(3):396-413*.)*

Visual impairment-associated disease (e.g. macular degeneration, retinitis pigmentosa, optic neuropathy, cataracts, glaucoma), anemia, cholestasis, hypoparathyroidism, pancytopenia, pancreatic disorder, lactic acidosis, lactacidaemia, hearing loss, short stature, intestinal obstruction, cardiac conduction defect, cardiomyopathy, endometriosis, infertility, early menopause (See Mitochondrial diseases: the contribution of organelle stress responses to pathology. Nat Rev Mol Cell Biol. 2018 Feb;19(2):77-92*.* Seminars in medicine of the Beth Israel Hospital, Boston. Mitochondrial DNA and disease. N Engl J Med. 1995 Sep 7; 333(10): 638-44*.* Mitochondrial injury and dysfunction in hypertension-induced cardiac damage. Eur Heart J. 2014 Dec 7; 35(46): 3258-3266*.)*

Muscular atrophy diseases including limb girdle/Becker muscular dystrophy (GGMD/BMD) and Duchenne muscular dystrophy (DMD) (Duchenne muscular dystrophy is associated with the inhibition of calcium uniport in mitochondria and an increased sensitivity of the organelles to the calcium-induced permeability transition. See Biochim Biophys Acta Mol Basis Dis. 2020 May 1;1866(5):165674*.)*

Aging and aging-related diseases (See Interrelation between ROS and Ca2+ in aging and age-related diseases. Redox Biology. 2020 ; 6:101678*.*)*.*

A pharmaceutical composition comprising the compound of Formula 1 exhibits not only liver protection and liver function improvement effects, but also preventive and therapeutic effects against acute and chronic liver diseases such as fatty liver, hepatic fibrosis, liver cirrhosis, and virus or drug-induced hepatitis. Further, as a result [the pharmaceutical composition] may prevent or treat liver disease complications such as portal hypertension, but is not limited to such. Even further, the pharmaceutical composition according to the present invention is effective in preventing and treating liver disease selected from among liver transplant, alcoholic or non-alcoholic fatty liver disease (See Korean Registered Patent 10-2006247), hepatic fibrosis, liver cirrhosis, and virus or drug-induced hepatitis, and is effective against acute and chronic alcoholic liver disease. Further, the composition according to the present invention is effective in treating or preventing fatty liver caused by fatty acids or acute or chronic liver disease caused by fatty liver.

The compound of Formula 1 may include a stem cell culturing step to improve the differentiation efficiency and maturity of stem cell-derived cardiomyocytes.

Further, the compound of Formula 1 can be used to prevent and treat mucositis.

More specifically, the compound of Formula 1 may be used for preventing or treating heart disease such as ischemic heart disease, cardiac conduction defect, cardiomyopathy, myocardial infarction and heart failure, neurodegenerative disease such as dementia, Parkinson's disease and amyotrophic lateral sclerosis, renal ischemia-reperfusion injury and fibrosis inhibition, acute and chronic respiratory disease and macular degeneration, and the like.

In this specification, the "treatment" means stopping or delaying progression of a disease or reversing or alleviating its symptoms when used in an object showing symptoms of onset, and the "prevention" means stopping or delaying indication of a disease when used in an object that does not show symptoms of onset, but is at high risk.

In the present invention, the "administration" means providing a given compound of the present invention to a subject by any suitable method.

In the present invention, "pharmaceutical composition" may comprise pharmaceutically acceptable carriers, together with the compounds of the present invention.

The compound of Formula 1 according to the present invention may be administered in various oral and non-oral dosage forms for clinical administration, and when formulated, is prepared using diluents or excipients such as commonly used fillers, bulking agents, binding agents, wetting agents, disintegrating agents, surfactants.

Solid formulations for oral administration include tablets, pills, powders, granules, capsules and troches, and such solid formulations are prepared by mixing at least one of the compound of the present invention with at least one excipient, for example, starch, calcium carbonate, sucrose, lactose or gelatin. Further, in addition to simple excipients, lubricating agents such as magnesium stearate talc are used. Liquid formulations for oral administration include suspensions, liquids for internal use, emulsions or syrups, and in addition to the commonly used simple diluents water and liquid paraffin, various excipients, for example wetting agents, sweeteners, aromatics and preservatives may be included.

Formulations for non-oral administration include sterile aqueous solutions, nonaqueous solvents, suspensions, emulsions, lyophilized preparations, suppositories. As the nonaqueous solvent and the suspension solvent, propylene glycol, polyethylene glycol, vegetable oil such as olive oil, injectable ester such as ethyl oleate, and the like can be used. As the base of the suppository, witepsol, macrogol, tween 61, cacao butter, laurin butter, glycerol, gelatin and the like can be used.

Further, the effective human dose for the compound of Formula 1 according to the present invention may vary depending on the age, body weight, sex, mode of administration, health status and severity of illness of the patients, and is normally approximately 0.001-100mg/kg/day, preferably 0.01-35 mg/kg/day. For an adult patient with a body weight of 70kg, it is normally 0.07-7000mg/day, preferably 0.7-2500mg/day, and depending on the judgment of a physician or pharmacist [the compound of Formula 1] may be administered once a day or divisionally across multiple administrations at certain time intervals.

A pharmaceutical composition comprising the compound of Formula 1, an isomer thereof, a hydrate thereof, a solvate thereof, or a pharmaceutically acceptable salt thereof as an active ingredient may be used to be administered as a single therapeutic agent, or to be administered in combination with other therapeutic agents in use.

Another aspect of the present invention provides a method for preventing or treating cell necrosis or ferroptosis-related diseases comprising a step of administering the compound of Formula 1, an isomer thereof, a hydrate thereof, a solvate thereof, or a pharmaceutically acceptable salt thereof to a subject in need thereof in a pharmaceutically effective amount.

In the present invention, the cell necrosis or ferroptosis-related disease may involve lipid peroxidation.

In the present invention, the "lipid peroxidation" means oxidative degradation of fats, oils, waxes, sterols, triglycerides, and the like, and the lipid peroxidation is considered as one of the main causes of the development of various degenerative diseases.

In the present invention, the cell necrosis or ferroptosis-related disease may be, specifically, neurodegenerative disease, liver disease, kidney disease, stroke, myocardial infarction, ocular disease or lung disease.

The neurodegenerative disease may be one or more selected from the group consisting of Alzheimer's disease, Parkinson's disease, epilepsy, Huntington's disease, amyotrophic lateral sclerosis, Friedreich's ataxia, multiple sclerosis, CMT (Charcot-Marie-Tooth) disease, dementia with Lewy bodies, and traumatic brain Injury.

Another aspect of the present invention provides a method for suppressing ferroptosis comprising a step of administering the compound of Formula 1, an isomer thereof, a hydrate thereof, a solvate thereof, or a pharmaceutically acceptable salt thereof, to a subject in need thereof in a pharmaceutically effective amount.

In the present invention, the compound of Formula 1 may interfere or inhibit ferroptosis by acting on Erastin, glutamate, or RSL3, and the like, which are ferroptosis-inducing substances, thereby inhibiting cell death.

Another aspect of the present invention provides a method for reducing reactive oxygen species (ROS) in cells comprising a step of contacting the compound of Formula 1, an isomer thereof, a hydrate thereof, a solvate thereof, or a pharmaceutically acceptable salt thereof with the cells.

In this specification, the "reactive oxygen species (ROS)" mean chemically active molecules, such as free radicals, containing oxygen, and an example of the reactive oxygen species may include oxygen ions and peroxides. The ferroptosis is characterized by rapid accumulation of reactive oxygen species in an iron-dependent manner, and the compound of Formula 1 can inhibit ferroptosis by inhibiting reactive oxygen species.

In the present invention, the "subject" in need of administration may include both mammals and non-mammals. Here, an example of the mammal may include humans, non-human primates such as chimpanzees or monkey species, livestock animals such as cattle, horse, sheep, and the like, but is not limited thereto.

Another aspect of the present invention provides a use of the compound of Formula 1, an isomer, a solvate thereof, or a pharmaceutically acceptable salt thereof, for prevention or treatment of cell necrosis or ferroptosis-related diseases.

The use and the prevention or treatment method of the present invention may be applied mutatis mutandis to the contents of the pharmaceutical composition.

### [Effects of Invention]

The compound of Formula 1 according to the present invention can exhibit cell necrosis inhibitory efficacy in various cells such as heart, kidney, nerve, retina, liver, or lung cells, and in particular can effectively inhibit ferroptosis.

In addition, the compound of Formula 1 according to the present invention can exhibit excellent pharmacokinetic values in plasma and brain.

Accordingly, the compound of Formula 1 according to the present invention can be usefully used for prevention or treatment of cell necrosis or ferroptosis-related diseases in various cells.

### [Brief Description of Drawings]

FIG. 1 is a graph of the calcium concentration regulation effect of the compound of Example 2 under the t-BHP treatment conditions of Experimental Example 2.
FIG. 2 is a time v. average plasma and brain concentration graph following oral administration of the compound of Example 2 according to Experimental Example 9 (ICR mouse, 10 mg/kg).
FIG. 3 is a time v. average plasma and brain concentration graph following oral administration of the compound of the Comparative Example according to Experimental Example 9 (ICR mouse, 10 mg/kg).

### [Best Modes of the Invention]

The advantages and characteristics of the present invention, and method for achieving the same, shall become evident with reference to the embodiments which are described in detail in the following. However, the present invention is not limited to the embodiments disclosed in the following, and may be realized in various different forms. The present embodiments are solely intended to complete the disclosure o the present invention, and to inform persons having ordinary skill in the art of the full scope of the invention; the present invention is defined solely by the appended claims.

### [Example]

The following preparation examples describe in further detail the preparation of intermediates necessary for synthesis of the compounds according to embodiments of the present invention. The abbreviations used in the following preparation examples and embodiments are as follow.
Ac: acetyl
ACN: acetonitrile
BOC: t-butoxycarbonyl
Bn: benzyl
Bu: butyl
DBU: 1,8-diazabicyclo(5,4,0)undec-7-en
DCM: dichloromethane
DIBAL-H: diisobutylaluminum hydride
DIPEA: diisopropylethylamine
DMF: N,N-dimethylformamide
DMSO: dimethylsulfoxide
EA: ethyl acetate
EtOH: ethyl alcohol
Et: ethyl
HATU: (1-[bis(dimethylamino)methylene]-1H-1,2,3-triazolo[4,5-b]pyridinium 3-oxide hexafluorophosphate
Hex: hexane
EDC: 1-ehtyl-3-(3-dimethylaminopropyl)carbodiimide
Me: methyl
NMM: N-methylmorpholine
NBS: N-bromosuccinimide
NIS: N-iodosuccimide
PE: petroleum ether
Pd(dppf)Cl₂: [1,1' -bis(diphenylphosphino)ferrocene]dichloropalladium(II)
Ph: phenyl
TBAF: tetrabutylammonium fluoride
TEA: triethylamine
TFA: trifluoroacetic acid
THF: tetrahydrofuran
TMS: trimethylsilyl

The example compounds of the present invention may be prepared in accordance with Reaction Formula 1 or 2 below. Reaction Formula 1 illustrates a case where R² is a substituent other than hydrogen, and Reaction Formula 2 illustrates a case where R³ is a substituent other than hydrogen.

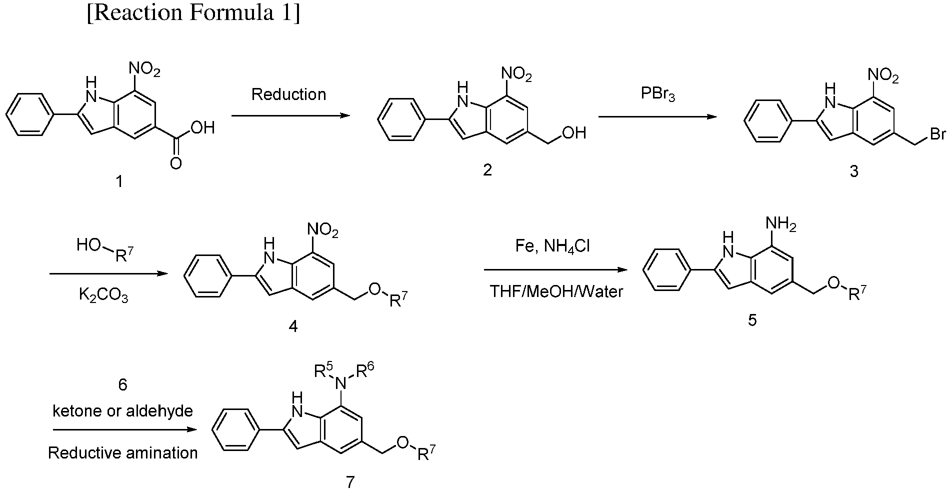

In Reaction Formula 1 above, Compounds 1 and 2 were synthesized according to the method described in Korean Registered Patent 10-1511771.

Using Intermediate 3 obtained in Reaction Formula 1, a suitable alcohol (R⁴-OH) and a base, Compound 4 can be synthesized. As the base, TEA, DIPEA, DBU, NMM or K₂CO₃, or commercially available NaOEt or NaOMe may be used.

Using Compound 4 with a metal and acid catalyst, or by carrying out a reduction reaction using a metal catalyst in the presence of hydrogen gas, Compound 5 can be obtained. As the metal, Fe, Zn, Li, Na and the like may be used, and as the acid catalyst, an inorganic acid such as hydrochloric acid, sulfuric acid or nitric acid, an organic acid such as AcOH or TFA, or NH₄Cl, etc. may be used. A reduction reaction may be carried out in the presence of hydrogen gas using Pd, Ni, Pt, Rt or Rh and the like as the metal catalyst.

Using Compound 5 with a ketone compound or the aldehyde compound Compound 6 with NaBH(OAc)₃ or NaBH₃CN and the like, Compound 7 can be obtained through a reductive amination reaction.

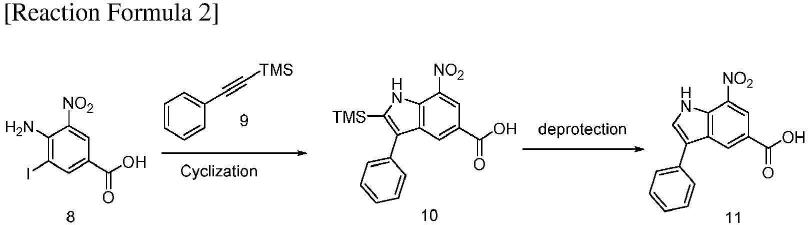

In Reaction Formula 2 above, Compound 8, 10 and 11 were synthesized in accordance with the method described in Korean Registered Patent 10-1511771.

Compound 10 can be prepared through indole ring synthesis using Compound 8 and the TMS compound Compound 9 in the presence of DBU, K₂CO₃ or a commercially available base such as KOtBu, and a metal catalyst such as CuI or Pd(II).

Compound 11 can be prepared by removing the protective group of Compound 10 through a suitable deprotection reaction.

Preparation Examples 1 to 27 below show the intermediate compounds for preparing the compounds of the example of the present invention, and the methods for synthesis of the same.

### Preparation Example 1: (7-nitro-2-phenyl-1H-indol-5-yl)methanol {(7-nitro-2-phenyl-1H-indol-5-yl)methanol}

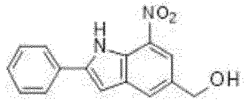

7-nitro-2-phenyl-1H-indol-5-carboxylic acid (1.0 g, 3.45 mmol) was added to THF (10 mL) and cooled to 0°C. 2M BH₃ · SMe₂/THF (5.3 mL, 10.6 mmol) was slowly added dropwise, followed by stirring for 30 minutes at the same temperature. The reaction mixture was heated to room temperature and stirred for 5 hours. The reaction mixture was cooled to 0°C, and 1N NaOH aqueous solution (10 mL) was slowly added dropwise over 30 minutes. After 1 hour of additional stirring, EA was added, and after 2 extractions the organic layer was collected and washed with brine. The organic layer was dried with MgSO₄ and filtered. The filtrate was concentrated under reduced pressure, then EtOH was added to the concentration residue to crystallize, followed by filtration and vacuum drying to obtain the title compound (770 mg, 71%) as a yellow solid.

¹H NMR (400MHz, CDCl₃); δ 10.08 (br, 1H), 8.17 (s, 1H), 7.97 (s, 1H), 7.76 (d, 2H), 7.51 (t, 2H), 7.49 (t, 1H), 6.94 (s, 1H), 4.87 (s, 2H), 1.81 (t, 1H).

### Preparation Example 2: 5-(bromomethyl)7-nitro-2-phenyl)-1H-indole

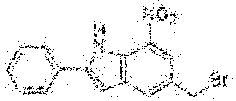

The (7-nitro-2-phenyl-1H-indol-5-yl)methanol (7.6 g, 28.33 mmol) was added to THF (76 mL) and cooled to 0°C. PBr₃ (1.6 mL, 17.0 mmol) was slowly added dropwise, and the mixture was heated to room temperature and stirred for 2 hours. The reaction mixture was poured into ice water to stop the reaction, and EA was added to extract. The organic layer was dried with MgSO₄ and filtered. The filtrate was concentrated under reduced pressure, then diethyl ether was added to the concentration residue to crystallize, followed by filtration and vacuum drying to obtain the title compound (7.5 g, 80%) as a yellow solid.

¹H NMR (400MHz, CDCl₃); δ 10.04 (br, 1H), 8.13 (s, 1H), 7.94 (s, 1H), 7.72 (d, 2H), 7.50 (t, 2H), 7.42 (t, 1H), 6.90 (s, 1H), 4.84 (s, 2H)

### Preparation Example 3: 5-(methoxymethyl)-7-nitro-2-phenyl-1H-indole

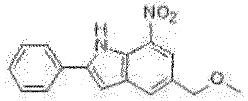

5-(bromomethyl)-7-nitro-2-phenyl-1H-indole (7.4 g, 22.36 mmol) was dissolved in a mixture of THF (150 mL) and MeOH (74 mL), then cooled to 0°C. NaOMe 25 wt% in MeOH (10.2 mL, 44.7 mmol) was added dropwise, then stirred for 1 hour at 0°C. The reaction mixture was poured into ice to stop the reaction, the extracted twice with EA. The organic layer was collected and washed with saturated NH₄Cl aqueous solution and saturated NaCl aqueous solution, then dried with MgSO₄, and filtered. The filtrate was concentrated under reduced pressure, and the concentration residue was purified by silica gel column chromatography (Hex/EA, 20/1 - 15/1) to obtain the title compound (3.1 g, 49%) as a yellow solid.

¹H NMR (400MHz, CDCl₃); δ 10.06 (br, 1H), 8.12 (s, 1H), 7.92 (s, 1H), 7.74 (d, 2H), 7.49 (t, 2H), 7.42 (t, 1H), 6.90 (s, 1H), 4.59 (s, 2H), 3.43 (s, 3H)

### Preparation Example 4: 5-(2-methoxymethyl)-2-phenyl-1H-indol-7-amine

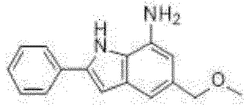

5-(methoxymethyl)-7-nitro-2-phenyl-1H-indole (2.6 g, 9.2 mmol) was added to a mixture of THF (26 mL), MeOH (26 mL) and H₂O (26 mL), and Fe powder (2.83 g, 50.65 mmol) and NH₄Cl (1.48 g, 27.6 mmol) were added. The mixture was heated to 60°C and stirred for 2 hours (in a brown suspension.) The reaction mixture was cooled to room temperature and diluted with DHF, then filtered through a celite pad, and washed with THF before concentrating the filtrate. EA and saturated NaHCO₃ aqueous solution was added to the concentration residue to extract, then the organic layer was washed with saturated NaCl aqueous solution. After

drying with MgSO₄ and filtering, the filtrate was concentrated under reduced pressure. Hexane was added to the concentration residue, and after stirring into a suspension the mixture was filtered to obtain the title compound (1.67 g, 72%) as a brown solid.

¹H NMR (400MHz, DMSO-d₆); δ 10.89 (br, 1H), 7.81 (d, 2H), 7.64 (d, 2H), 7.31 (t, 1H), 6.76 (s, 1H), 6.73 (s, 1H), 5.16 (br, 2H), 4.32 (s, 2H), 3.23 (s, 3H)).

### Preparation Example 5: 2-methoxy-N-[5-methoxymethyl]-2-phenyl-1H-indol-7-yl]acetamide

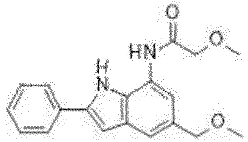

Methoxyacetic acid (33 µL, 0.436 mmol) was diluted in ACN (1.5 mL), then DIPEA (207 µL, 1.188 mmol) and HATU (226 mg, 0.594 mmol) were added. 5-(methoxymethyl)-2-phenyl-1H-indol-7-amine (100 mg, 0.396 mmol) was added, followed by stirring for 15 hours at room temperature. After confirming completion of the reaction with TLC, the reaction mixture was purified by silica gel column chromatography (Hex/EA, 1/2) to obtain the title compound (99 mg, 77%) as a yellow solid.

¹H NMR (400MHz, CDCl₃); δ 10.83 (br, 1H), 8.58 (br, 1H), 7.69 (d, 2H), 7.39 (d, 2H), 7.28 (t, 1H), 6.79 (s, 1H), 6.76 (s, 1H), 4.49 (s, 2H), 4.09 (s, 2H), 3.53 (s, 3H), 3.36 (s, 3H).

### Preparation Example 6: 3-methoxy-N-[5-(methoxymethyl)-2-phenyl-1H-indol-7-yl]propenamide

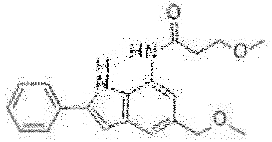

3-methoxypropionic acid (41 µL, 0.436 mmol) was diluted in ACN (1.5 mL), then DIPEA (207 µL, 1.188 mmol) and HATU (226 mg, 0.594 mmol) were added. The 5-(2-methoxymethyl)-2-phenyl-1H-indol-7-amine obtained in Preparation Example 4 (100 mg, 0.396 mmol) was added, followed by stirring for 15 hours at room temperature. After confirming completion of the reaction with TLC, the reaction mixture was purified by silica gel column chromatography (Hex/EA, 1/2) to obtain the title compound (150 mg, quantitative yield) as a yellow solid.

¹H NMR (400MHz, CDCl₃); δ 10.83 (br, 1H), 8.58 (br, 1H), 7.69 (d, 2H), 7.39 (d, 2H), 7.28 (t, 1H), 6.79 (s, 1H), 6.76 (s, 1H), 4.09 (s, 2H), 3.70 (t, 3H), 3.53 (s, 3H), 3.33 (t, 2H), 3.36 (s, 3H)

### Preparation Example 7: 5-(tert-butoxymethyl)-7-nitro-2-phenyl-1H-indole

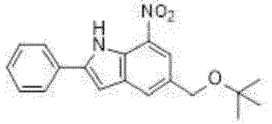

The (7-nitro-2-phenyl-1H-indol-5-yl)methanol obtained in Preparation Example 1 (500 mg, 1.86 mmol) was added to DCM (75 mL), then cooled to -70°C. After bubbling for 3 minutes with 2-methylprop-1-ene gas for 3 minutes, the mixture was additionally stirred for 30 minutes at -70°C and was heated to room temperature and stirred for 15 hours. After confirming completion of the reaction with TLC, the reaction mixture was poured into a saturated NaHCO₃ aqueous solution and stirred for 30 minutes. After layer separation, the organic layer was washed with saturated NaCl aqueous solution and dried with MgSO₄, then filtered. The filtrate was concentrated under reduced pressure and the concentration residue was purified by silica gel column chromatography (Hex/EA, 9/1) to obtain the title compound (150 mg, 25%) as a yellow solid.

¹H NMR (400MHz, CDCl₃); δ 10.03 (br, 1H), 8.12 (s, 1H), 7.92 (s, 1H), 7.71 (d, 2H), 7.48 (d, 2H), 7.28 (t, 1H), 6.89 (s, 1H), 4.57 (s, 2H), 1.33 (s, 9H)

### Preparation Example 8: 5-(tert-butoxymethyl)-2-phenyl-1H-indol-7-amine

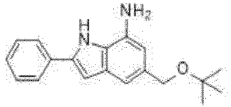

The 5-(tert-butoxymethyl)7-nitro-2-phenyl-1H-indole (150 mg, 0.462 mmol) obtained in Preparation Example 7 was added to a mixture of THF (30 mL), MeOH (30 mL) and H₂O (30 mL), then Fe powder (258 mg, 4.62 mmol) and NH₄Cl (247 mg, 4.62 mmol) were added. The mixture was heated to 60°C, then stirred for 2 hours (brown suspension). The reaction mixture was cooled to room temperature, diluted with THF, then filtered through a celite pad. After washing with THF, the filtrate was concentrated. EA and saturated NaHCO₃ aqueous solution were added to extract, and the organic layer was washed with saturated NaCl aqueous solution. After drying with MgSO₄ and filtering, the filtrate was concentrated under reduced pressure. The concentration residue was purified by silica gel column chromatography (Hex/EA, 1/1) to obtain the title compound as a yellow solid (60 mg, 44%).

Mass [M+H] = 295.4 (M+1)

### Preparation Example 9: 4-amino-3-iodo-5-nitrobenzoic acid

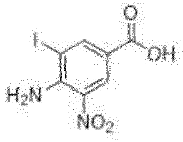

Commercially available 4-amino-3-nitrobenzoic acid (5 g, 27.45 mmol) was added to THF (50 mL), then cooled to 0°C. Keeping the temperature below 10°C, c-H₂SO₄ was added, and then the mixture was heated to room temperature. NIS (9.26 g, 41.18 mmol) was added, followed by heating to 70°C and refluxing for 18 hours. After confirming completion of the reaction with TLC, the mixture was cooled to room temperature. DCM (100 mL) was added to the reaction mixture, which was then stirred for 1 hour. The solid was filtered, then washed with DCM (50 mL) and EtOH (20 mL), and vacuum dried to obtain the title compound (5.2 g, 62%) as a yellow solid.

¹H NMR (400MHz, DMSO-d₆); δ 8.52 (s, 1H), 8.37 (s, 1H), 7.50 (br, 2H).

### Preparation Example 10: 7-nitro-3-phenyl-1H-indol-5-carboxylic acid

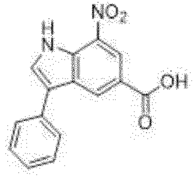

The 4-amino-3-iodo-5-nitrobenzoicacid obtained in Preparation Example 9 (500 mg, 1.623 mmol) was dissolved in DMF (20 mL). TEA (1.13 mL0, 8.115 mmol), trimethyl(phenylethynyl)silane (1.57 mL, 8.115 mmol), Pd(OAc)₂ (36.4 mg, 0.1623 mmol) and LiCl (68.7 mg, 1.623 mmol) were added, followed by degasification with argon gas for 30 minutes. The mixture was heated to 100°C under an argon atmosphere, then stirred for 4 hours. After confirming completion of the reaction with TLC, the mixture was cooled to room temperature. EA (200 mL) and saturated NH₄Cl aqueous solution (100 mL) were added to the reaction mixture, which was then extracted. The water layer was additionally extracted twice with EA (100 mL), and the organic layer was collected, dried with MgSO₄, and purified. The filtrate was concentrated under reduced pressure and purified by silica gel column chromatography (DCM/MeOH, 95/5) to obtain crude 7-nitro-3-phenyl-2-(trimethylsilyl)-1H-indol-5-carboxylic acid (1.6 g, quantitative yield) as a red wind colored liquid, which was used in the subsequent reaction without further purification.

The crude 7-nitro-3-phenyl-2-(trimethylsilyl)-1H-indol-5-carboxylic acid obtained in the previous step was diluted by adding THF (20 mL), then cooled to 0°C. TBAF 1M in THF (3.26 mL, 3.26 mmol) was added, then the mixture was stirred for 1 hour at 0°C (a yellow solid was formed.) After heating to room temperature and stirring additionally for 4 hours, completion of the reaction was confirmed using TLC, and H₂O was added to terminate the reaction. Concentration under reduced pressure was performed to remove volatile substances, and EA was added to the concentration residue to extract. The organic layer was dried with MgSO₄ and filtered, and the filtrate was concentrated under reduced pressure. Diethyl ether was added to the concentration residue to crystallize, then filter to obtain the title compound (220 mg, 48%) as a dark brown solid.

Mass [M+H] = 283.3 (M+1)

### Preparation Example 11: (7-nitro-3-phenyl-1H-indol-5-yl)methanol

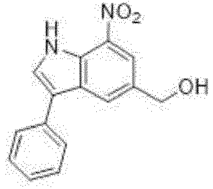

The 7-nitro-3-phenyl-1H-indol-5-carboxylic acid obtained in Preparation Example 10 (220 mg, 0.779 mmol) was dissolved in THF (3 mL), then cooled to 0°C. BH₃ · SMe₂ 2M in THF (1.17 mL, 2.34 mmol) was added, and the mixture was heated to room temperature and stirred for 2 hours. Completion of the reaction was confirmed with TLC, then the mixture was cooled to 0°C. 1N NaOH aqueous solution (5 mL) was slowly added dropwise, then the mixture was slowly heated to room temperature, and stirred for 1 hour. After extracting 2 times with EA, the organic layer was collected and washed with saturated NaCl aqueous solution. The organic layer was dried with MgSO₄ and filtered, then concentrated under reduced pressure. The concentration residue was purified by silica gel column chromatography (Hex/EA, 3/1) to obtain the title compound (130 mg, 62%) as an orange solid.

¹H NMR (400MHz, CDCl₃); δ 9.99 (br, 1H), 8.24 (d, 2H), 7.62 (s, 2H), 7.50 (s, 1H), 7.48 (d, 2H), 7.31 (t, 2H), 4.88 (d, 2H).

### Preparation Example 12: 5(methoxymethyl)-7-nitro-3-phenyl-1H-indole

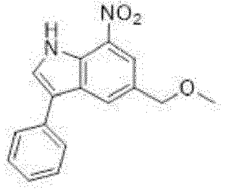

The (7-nitro-3-phenyl-1H-indol-5-yl)methanol (130 mg, 0.485 mmol) was dissolved in THF (3 mL), then cooled to 0°C. PBr₃ (27.4 µL, 0.291 mmol) was added. The mixture was heated to room temperature and stirred for 2 hours. Completion of the reaction was confirmed with TLC, then the reaction mixture was poured into ice water to terminate the reaction. EA was added to extract, and the organic layer was dried with MgSO₄ and filtered. The filtrate was concentrated under reduced pressure to obtain crude 5-(bromomethyl)-7-nitro-3-phenyl-1H-indole as an orange liquid, which was used in the subsequent reaction without additional purification.

The 5-(bromomethyl)-7-nitro-3-phenyl-1H-indole obtained in the previous step was dissolved in THF (1.3 mL) and MeOH (2.6 mL), then cooled to 0°C. NaOMe 25% in MeOH (76 mg, 1.455 mmol) was added. The mixture was heated to room temperature and stirred for 30 minutes. After confirming completion of the reaction, the reaction mixture was poured into ice water to terminate the reaction. After extracting twice with EA, the organic layer was collected and washed with saturated NaCl aqueous solution. After drying with MgSO₄ and filtering, the filtrate was concentrated under reduced pressure. The concentration residue was purified by silica gel column chromatography (Hex/EA, 9/1) to obtain the title compound (33 mg, 17%) as a yellow liquid.

¹H NMR (400MHz, CDCl₃); δ 10.00 (br, 1H), 8.22 (d, 2H), 7.62 (d, 2H), 7.50 (d. 2H), 7.49 (s, 1H), 7.47 (t, 1H), 4.62 (s, 2H), 3.43 (s, 3H).

### Preparation Example 13: 5-(methoxymethyl)-3-phenyl-1H-indol-7-amine

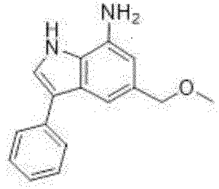

To the 5(methoxymethyl)-7-nitro-3-phenyl-1H-indole obtained in Preparation Example 12 (33 mg, 0.117 mmol), a mixed solution of THF (2 mL), MeOH (2 mL) and H₂O (2 mL) was added. Then Fe powder (65 mg, 1.17 mmol) and NH₄Cl (63 mg, 1.17 mmol) were added. The mixture was heated to 60°C, then stirred for 1 hour (brown suspension). The reaction mixture was cooled to room temperature and diluted with THF, then filtered through a celite pad. After washing, the filtrate was concentrated. EA and saturated NaHCO₃ aqueous solution was added to extract, and the organic layer was washed with saturated NaCl aqueous solution. After drying with MgSO₄ and filtering, the filtrate was concentrated under reduced pressure. The concentration residue was purified by silica gel column chromatography (Hex/EA, ½) to obtain the title compound as a brown liquid (13 mg, 44%).

Mass [M+H] = 253.3 (M+1)

### Preparation Example 14: 5-(ethoxymethyl)-2-phenyl-1H-indol-7-amine

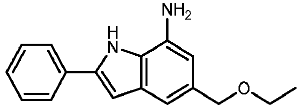

### Step 1: 5-(ethoxymethyl)-7-nitro-2-phenyl-1H-indole

5-(bromomethyl)-7-nitro-2-phenyl-1H-indole (2.0 g, 6.04 mmol) obtained in Preparation Example 2 was added to ethanol (50 mL), and the suspension was dissolved by adding a minimum amount of THF thereto while heating it to reflux. After heating it to reflux and stirring overnight, it was distilled under reduced pressure, whereby the concentrated residue was purified by column chromatography to obtain 5-(ethoxymethyl)-7-nitro-2-phenyl-1H-indole (1.7 g, yield 92%).

¹H NMR (400 MHz, DMSO-d₆); δ 11.62 (bs, 1H), 8.05 (s, 1H), 8.01-7.99 (m, 3H), 7.52-7.41 (m, 3H), 7.16 (s, 1H), 3.54 (q, 2H), 1.19 (t, 3H).

### Step 2: 5-(ethoxymethyl)-2-phenyl-1H-indol-7-amine

From the compound 5-(ethoxymethyl)-7-nitro-2-phenyl-1H-indole (1.7 g, 5.57 mmol) obtained in Step 1, 5-(ethoxymethyl)-2-phenyl-1H-indol-7-amine (1.5 g, yield 100%) was obtained, using the same method as in Preparation Example 4. This compound was used in the next reaction without purification.

¹H NMR (400 MHz, DMSO-d₆); δ 10.88 (bs, 1H), 7.80 (d, 2H), 7.46 (t, 2H), 7.29 (t, 1H), 6.75 (s, 1H), 6.73 (s, 1H), 6.33 (s, 1H), 5.16 (bs, 2H), 4.36 (s, 2H), 3.43 (q, 2H), 1.13 (t, 3H).

### Preparation Example 15: 5-(isopropoxymethyl)-2-phenyl-1H-indol-7-amine

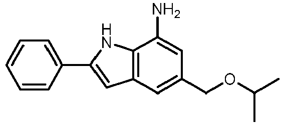

### Step 1: 5-(isopropoxymethyl)-7-nitro-2-phenyl-1H-indole

5-(bromomethyl)-7-nitro-2-phenyl-1H-indole (1.0 g, 3.02 mmol) obtained in Preparation Example 2 was added to isopropanol (50 mL), and the suspension was melted by adding a minimum amount of THF thereto while heating it to reflux. After heating it to reflux and stirring overnight, it was distilled under reduced pressure, whereby the concentrated residue was purified by column chromatography to obtain 5-isopropoxymethyl-7-nitro-2-phenyl-1H-indole (0.9 g, yield 96%).

¹H NMR (400 MHz, DMSO-d₆); δ 11.60 (bs, 1H), 8.10-7.95 (m, 4H), 7.56-7.38 (m, 4H), 7.15 (s, 1H), 4.63 (s, 2H), 3.80-3.62 (m, 1H), 1.18 (d, 2H).

### Step 2: 5-(isopropoxymethyl)-2-phenyl-1H-indol-7-amine

From 0.9 g of 5-(isopropoxymethyl)-7-nitro-2-phenyl-1H-indole as obtained above, 5-(isopropoxymethyl)-2-phenyl-1H-indole-7-amine (0.58 g, yield 72%) was obtained, using the same method as in Preparation Example 4.

¹H NMR (400 MHz, DMSO-d₆); δ 10.87 (bs, 1H), 7.80 (d, 2H), 7.46 (t, 2H), 7.29 (t, 1H), 6.75 (s, 1H), 6.73 (s, 1H), 6.33 (s, 1H), 5.15 (s, 2H), 4.37 (s, 2H), 3.70-3.56 (m, 1H), 1.12 (d, 2H).

### Preparation Example 16: 5-(ethoxymethyl)-7-nitro-2-phenyl-1H-indole-3-carbaldehyde

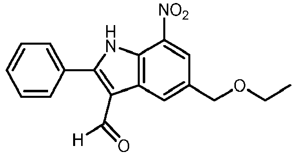

The intermediate 5-(ethoxymethyl)-7-nitro-2-phenyl-1H-indole (500 mg, 1.69 mmol) obtained in Step 1 of Preparation Example 14 was dissolved in DMF (5 mL), and POCl₃ (285 mg, 1.86 mmol) was slowly added dropwise thereto at 10°C. After stirring at 50°C for 1 hour, the reaction mixture was poured into water and extracted with EA. The organic layer was washed with a saturated NaHCO₃ solution and water, dried over MgSO₄, and the filtered filtrate was distilled under reduced pressure. 5-(ethoxymethyl)-7-nitro-2-phenyl-1H-indole-3-carbaldehyde (530 mg, yield 96%) obtained by concentration was used in the next reaction without purification.

¹H NMR (400 MHz, DMSO-d₆); δ 12.70 (bs, 1H), 9.23 (s, 1H), 8.64 (s, 1H), 8.17 (s, 1H), 7.82 (d, 1H), 7.63-7.56 (m, 3H), 4.69 (s, 2H), 3.56 (q, 2H), 1.20 (t, 3H).

### Preparation Example 17: 7-amino-5-(ethoxymethyl)-2-phenyl-1H-indole-3-carbonitrile

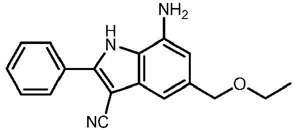

### Step 1: 5-(ethoxymethyl)-7-nitro-2-phenyl-1H-indole-3-carbaldehyde oxime

530 mg (1.63 mmol) of the compound obtained in Preparation Example 16 and hydroxylamine hydrochloride (180 mg, 2.45 mmol) were dissolved in DMF (2 mL) and stirred at 110°C for 2 hours. At room temperature, the reaction mixture was placed in water and extracted with EA. The organic layer was washed with water and a saturated NaCl solution, dried over MgSO₄, and then the filtered filtrate was distilled under reduced pressure. 5-(ethoxymethyl)-7-nitro-2-phenyl-1H-indole-3-carbaldehyde oxime (530 mg, yield 96%) obtained by concentration was used in the next reaction without purification.

¹H NMR (400 MHz, DMSO-d₆); δ 12.14 (bs, 1H), 11.04 (s, 1H), 8.54 (s, 1H), 8.17 (s, 1H), 8.15 (s, 1H), 7.70-7.50 (m, 5H), 4.65 (s, 2H), 3.54 (q, 2H), 1.19 (t, 3H).

### Step 2: 5-(ethoxymethyl)-7-nitro-2-phenyl-1H-indole-3-carbonitrile

DMF (15 mL) was added to the oxime compound (530 mg, 1.56 mmol) obtained in Step 1, KF (4.5 g), and aluminum oxide (6.8 g), and the mixture was stirred at 110°C for 3 hours, and then stirred at room temperature overnight. The reaction mixture was diluted with EA and filtered. The organic layer was washed with water and a saturated NaCl solution, and then dried over MgSO₄, and the filtered filtrate was distilled under reduced pressure. The concentrated residue was purified by column chromatography to obtain 5-(ethoxymethyl)-7-nitro-2-phenyl-1H-indole-3-carbonitrile (200 mg, yield 40%).

¹H NMR (400 MHz, DMSO-d₆); δ 12.79 (bs, 1H), 8.21 (s, 1H), 8.08 (s, 1H), 8.00-7.93 (m, 2H), 7.69-7.56 (m, 3H), 4.70 (s, 2H), 3.57 (q, 2H), 1.20 (t, 3H).

### Step 3: 7-amino-5-(ethoxymethyl)-2-phenyl-1H-indole-3-carbonitrile

From the compound (200 mg) obtained in Step 2, 7-amino-5-(ethoxymethyl)-2-phenyl-1H-indole-3-carbonitrile (140 mg, yield 77%) was obtained using the same method as in Preparation Example 4.

¹H NMR (400 MHz, DMSO-d₆); δ 11.91 (bs, 1H), 7.94 (d, 2H), 7.63 (t, 2H), 7.54 (d, 1H), 6.77 (s, 1H), 6.50 (s, 1H), 5.46 (bs, 2H), 4.43 (s, 2H), 3.46 (q, 2H), 1.15 (t, 3H).

### Preparation Example 18: (E)-7-amino-5-(ethoxymethyl)-2-phenyl-1H-indole-3-carbaldehyde O-methyl-oxime

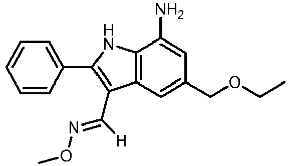

### Step 1: (E)-5-(ethoxymethyl)-7-nitro-2-phenyl-1H-indole-3-carbaldehyde O-methyl-oxime

The compound (1 g, 3.1 mmol) obtained in Preparation Example 16, methylhydroxylamine hydrogen chloride (0.31 g, 3.7 mmol), and pyridine (1 g, 12.3 mmol) were added to ethanol (10 mL) and stirred at room temperature for 4 hours. The reaction mixture was distilled under reduced pressure, and then EA was added thereto, and the mixture was washed with water and a saturated NaCl solution. After drying over MgSO₄, the filtered filtrate was distilled under reduced pressure. The concentrated residue was purified by column chromatography to obtain (E)-5-(ethoxymethyl)-7-nitro-2-phenyl-1H-indole-3-carbaldehyde O-methyl-oxime (0.95 g, yield 87%).

¹H NMR (400 MHz, DMSO-d₆); δ 12.25 (bs, 1H), 8.55 (s, 1H), 8.20 (s, 1H), 8.17 (s, 1H), 7.70-7.53 (m, 5H), 4.69 (s, 2H), 3.94 (s, 3H), 3.57 (q, 2H), 1.21 (t, 3H).

### Step 2: (E)-7-amino-5-(ethoxymethyl)-2-phenyl-1H-indole-3-carbaldehyde O-methyl-oxime

From the compound (0.95 g, 2.7 mmol) obtained in Step 1, (E)-7-amino-5-(ethoxymethyl)-2-phenyl-1H-indole-3-carbaldehyde O-methyl-oxime (0.79 g, yield 91%) was obtained using the same method as in Preparation Example 4.

¹H NMR (400 MHz, DMSO-d₆); δ 11.32 (bs, 1H), 8.25 (s, 1H), 7.67-7.40 (m, 5H), 7.31 (s, 1H), 6.47 (s, 1H), 5.22 (bs, 2H), 4.40 (s, 2H), 3.88 (s, 3H), 3.46 (q, 2H), 1.15 (t, 3H).

### Preparation Example 19: 5-(ethoxymethyl)-3-(methoxymethyl)-1-methyl-2-phenyl-1H-indol-7-amine

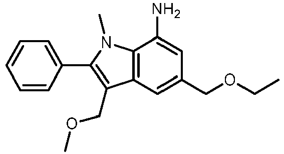

### Step 1: (5-(Ethoxymethyl)-1-methyl-7-nitro-2-phenyl-1H-indol-3-yl)-methanol

The compound (1 g, 3.08 mmol) obtained in Preparation Example 16 and NaBH₄ (0.24 g, 6.16 mmol) were added to ethanol (30 mL) and stirred at 45°C for 1 hour. 30 mL of 1% sodium hydroxide solution was added dropwise thereto at 10°C, and then water was added thereto, and the mixture was extracted with EA. The organic layer was washed with water and a saturated NaCl solution, dried over MgSO₄ and filtered. The filtrate was concentrated under reduced pressure, and the concentrated residue was purified by column chromatography to obtain (5-ethoxymethyl-1-methyl-7-nitro-2-phenyl-1H-indol-3-yl)-methanol (0.9 g, yield 89%).

¹H NMR (400 MHz, DMSO-d₆); δ 11.67 (bs, 1H), 8.15 (s, 1H), 8.09 (s, 1H), 7.81 (d, 2H), 7.55 (t, 2H), 7.48 (t, 1H), 5.14 (t, 1H), 4.69 (d, 2H), 4.66 (s, 2H), 3.57 (q, 2H), 1.21 (t, 3H).

### Step 2: 5-(ethoxymethyl)-3-(methoxymethyl)-1-methyl-7-nitro-2-phenyl-1H-indole

(5-(ethoxymethyl)-1-methyl-7-nitro-2-phenyl-1H-indol-3-yl)-methanol (1.23 g, 3.77 mmol) obtained in Step 1 was dissolved in DMF (30 mL), and 60% NaH (0.45 g, 11.3 mmol) was slowly added thereto at 0°C. After stirring at room temperature for 1 hour, MeI (1.4 g, 9.79 mmol) was added dropwise thereto at 0°C. After stirring at room temperature for 1 hour, the reaction was terminated with a saturated NH₄Cl solution, water was added thereto, and the mixture was extracted with EA. The organic layer was washed with water, dried over MgSO₄, and then the filtered filtrate was distilled under reduced pressure. The concentrated residue was purified by column chromatography to obtain 5-(ethoxymethyl)-3-(methoxymethyl)-1-methyl-7-nitro-2-phenyl-1H-indole (1.23 g, yield 91%).

¹H NMR (400 MHz, DMSO-d₆); δ 8.02 (s, 1H), 7.84 (s, 1H), 7.65-7.53 (m, 5H), 4.64 (s, 2H), 4.47 (s, 2H), 3.53 (q, 2H), 3.49 (s, 3H), 3.23 (s, 3H), 1.20 (t, 3H).

### Step 3: 5-(ethoxymethyl)-3-(methoxymethyl)-1-methyl-2-phenyl-1H-indol-7-amine

From 5-(ethoxymethyl)-3-(methoxymethyl)-1-methyl-7-nitro-2-phenyl-1H-indole (1.23 g, 3.47 mmol) obtained in Step 2, 5-(ethoxymethyl)-3-(methoxymethyl)-1-methyl-2-phenyl-1H-indol-7-amine (0.8 g, yield 70%) was obtained using the same method as in Preparation Example 4.

¹H NMR (400 MHz, DMSO-d₆); δ 7.60-7.40 (m, 5H), 6.89 (s, 1H), 6.50 (s, 1H), 4.93 (bs, 2H), 4.40 (s, 2H), 4.31 (s, 2H), 3.82 (s, 3H), 3.45 (q, 2H), 3.17 (s, 3H), 1.15 (t, 3H).

### Preparation Example 20: Methyl (E)-3-(7-amino-5-(ethoxymethyl)-2-phenyl-1H-indol-3-yl)-acrylate

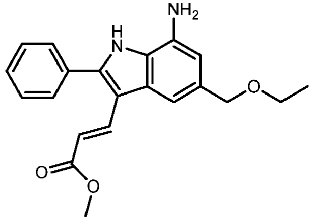

### Step 1: Methyl (E)-3-(5-(ethoxymethyl)-7-nitro-2-phenyl-1H-indol-3-yl)-acrylate

The compound (1.0 g, 3.08 mmol) obtained in Preparation Example 16 and methyl (triphenylphosphoranylidene)acetate (1.1 g, 3.24 mmol) were added to ACN (30 mL) and heated to reflux overnight. Water was added thereto at room temperature, and the mixture was extracted with EA. The organic layer was washed with water and a saturated NaCl solution, dried over MgSO₄, and the filtered filtrate was distilled under reduced pressure. The concentrated residue was purified by column chromatography to obtain methyl (E)-3-(5-(ethoxymethyl)-7-nitro-2-phenyl-1H-indol-3-yl)-acrylate (1.1 g, yield 94%).

¹H NMR (400 MHz, DMSO-d₆); δ 12.45 (bs, 1H), 8.43 (s, 1H), 8.19 (s, 1H), 7.72 (d, 1H), 7.65-7.54 (m, 5H), 6.62 (d, 1H), 4.72 (s, 2H), 3.71 (s, 3H), 3.57 (q, 2H), 1.21 (t, 3H).

### Step 2: Methyl (E)-3-(7-amino-5-(ethoxymethyl)-2-phenyl-1H-indol-3-yl)-acrylate

The compound (0.6 g, 1.58 mmol) obtained in Step 1 was dissolved in a 2:5:1 mixed solution (20 mL) of THF, methanol and water. Iron powder (1.1 g, 18.9 mmol) and NH₄Cl (1.7 g, 31.5 mmol) were added thereto, and the mixture was stirred at 40°C for 1 hour. After filtering with Celite at room temperature, water was added to the filtrate, and the mixture was extracted with EA. The organic layer was washed with water, dried over MgSO₄, and the filtered filtrate was distilled under reduced pressure. The concentrated residue was purified by column chromatography to obtain methyl (E)-3-(7-amino-5-(ethoxymethyl)-2-phenyl-1H-indol-3-yl)-acrylate (0.45 g, yield 80%).

¹H NMR (400 MHz, DMSO-d₆); δ 11.60 (bs, 1H), 7.82 (d, 1H), 7.69-7.50 (m, 5H), 7.08 (s, 1H), 6.52 (s, 1H), 6.38 (d, 1H), 5.31 (bs, 2H), 4.47 (s, 2H), 3.68 (s, 3H), 3.47 (q, 2H), 1.16 (t, 3H).

### Preparation Example 21: 1-(7-amino-5-(ethoxymethyl)-2-phenyl-1H-indol-3-yl)-2,2,2-trifluoro-ethan-1 -one

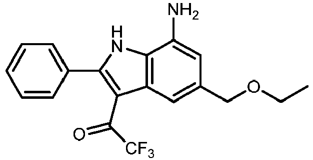

### Step 1: 1-(5-(ethoxymethyl)-7-nitro-2-phenyl-1H-indol-3-yl)-2,2,2-trifluoro-ethan-1-one

The intermediate 5-(ethoxymethyl)-7-nitro-2-phenyl-1H-indole (1.0 g, 3.37 mmol) obtained in Step 1 of Preparation Example 14, and trifluoroacetic anhydride (0.94 g, 6.75 mmol) were dissolved in DMF (15 mL) and stirred at room temperature overnight. Water was added to the reaction mixture, and the mixture was extracted with methyl t-butyl ether. The organic layer was washed with water and a saturated NaCl solution, dried over MgSO₄, and the filtered filtrate was distilled under reduced pressure. The concentrated residue was purified by column chromatography to obtain 1-(5-(ethoxymethyl)-7-nitro-2-phenyl-1H-indol-3-yl)-2,2,2-trifluoro-ethane-1-one (1.1 g, yield 82%).

¹H NMR (400 MHz, DMSO-d₆); δ 13.12 (bs, 1H), 8.48 (s, 1H), 8.23 (s, 1H), 7.66-7.50 (m, 5H), 4.72 (s, 2H), 3.58 (q, 2H), 1.22 (t, 3H).

### Step 2: 1-(7-amino-5-(ethoxymethyl)-2-phenyl-1H-indol-3-yl)-2,2,2-trifluoro-ethan-1-one

From the compound (0.6 g, 1.53 mmol) obtained in Step 1, 1-(7-amino-5-(ethoxymethyl)-2-phenyl-1H-indol-3-yl)-2,2,2-trifluoro-ethanone (0.36 g, yield 65%) was obtained using the same method as in Preparation Example 4.

¹H NMR (400 MHz, DMSO-d₆); δ 12.28 (bs, 1H), 7.65-7.50 (m, 5H), 7.25 (s, 1H), 6.54 (s, 1H), 5.39 (bs, 2H), 4.44 (s, 2H), 3.46 (q, 2H), 1.16 (t, 3H).

### Preparation Example 22: (E)-3-(7-amino-5-(ethoxymethyl)-2-phenyl-1H-indol-3-yl)-1-phenylprop-2-en-1-one

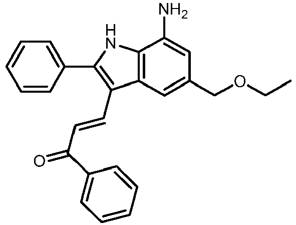

### Step 1: 3-(5-ethoxymethyl-7-nitro-2-phenyl-1H-indol-3-yl)-1-phenyl-propenone

5-(ethoxymethyl)-7-nitro-2-phenyl-1H-indole-3-carbaldehyde (500 mg, 1.54 mmol) obtained in Preparation Example 16, acetophenone (185 mg, 1.54 mmol), and piperidine (73 mg, 0.85 mmol) were added to ethanol (10 mL) and heated to reflux overnight. Water was added thereto at room temperature and the mixture was extracted with EA. The organic layer was washed with water and a saturated NaCl solution, dried over MgSO₄, and then the filtered filtrate was distilled under reduced pressure. The concentrated residue was stirred with methanol, filtered and then dried to 3-(5-ethoxymethyl-7-nitro-2-phenyl-1H-indol-3-yl)-1-phenyl-propenone (370 mg, yield 61%), which was used in the next reaction without purification.

¹H NMR (400 MHz, DMSO-d₆); δ 12.54 (bs, 1H), 8.60 (s, 1H), 8.22 (s, 1H), 8.10 (d, 1H), 7.94-7.55 (m, 10H), 4.79 (s, 2H), 3.57 (q, 2H), 1.22 (t, 3H).

### Step 2: (E)-3-(7-amino-5-(ethoxymethyl)-2-phenyl-1H-indol-3-yl)-1-phenylprop-2-en-1-one

The title compound (110 mg, yield 26%) was obtained from the compound (370 mg, 1.06 mmol) obtained in Step 1 using the same method as in Preparation Example 4.

¹H NMR (400 MHz, DMSO-d₆); δ 11.47 (bs, 1H), 8.08-7.98 (m, 3H), 7.70-7.52 (m, 9H), 7.28 (s, 1H), 6.56 (s, 1H), 5.35 (bs, 1H), 4.54 (s, 2H), 3.52 (q, 2H), 1.18 (t, 3H).

### Preparation Example 23: Ethyl 2-(7-amino-5-(ethoxymethyl)-2-phenyl-1H-indol-3-yl)-1H-imidazole-1-carboxylate

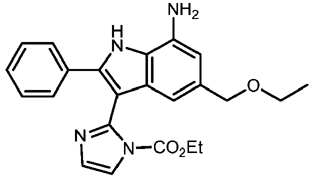

### Step 1: 2-(5-ethoxymethyl-7-nitro-2-phenyl-1H-indol-3-yl)-imidazole-1,3-dicarboxylic acid diethyl ester

Imidazole (0.82 g, 12 mmol) and TEA (3.64 g, 36 mmol) were dissolved in dichloroethane (60 mL), and ethyl chloroformate (2.86 g, 26.4 mmol) was slowly added dropwise thereto at 0°C. The intermediate 5-(ethoxymethyl)-7-nitro-2-phenyl-1H-indole (1.18 g, 4 mmol) obtained in Step 1 of Preparation Example 14 was added thereto under the same conditions, and TEA (3.64 g, 36 mmol) dissolved in dichloroethane was added dropwise thereto, and the mixture was stirred at room temperature for 1 hour. The reaction mixture was diluted with DCM, and washed sequentially with a 1N hydrochloric acid solution, a 3% Na₂CO₃ solution, and a saturated NaCl solution. The organic layer was dried over MgSO₄, and the filtered filtrate was distilled under reduced pressure. The concentrated residue was purified by column chromatography to obtain 2-(5-ethoxymethyl-7-nitro-2-phenyl-1H-indol-3-yl)-imidazole-1,3-dicarboxylic acid diethyl ester (0.3 g, yield 15%).

### Step 2: 2-(5-ethoxymethyl-7-nitro-2-phenyl-1H-indol-3-yl)-imidazole-1-carboxylic acid ethyl ester

The compound (300 mg, 0.59 mmol) obtained in Step 1 and o-chloranyl (148 mg, 0.59 mmol) were dissolved in ACN (10 mL) and stirred at room temperature for 4 hours. The reaction mixture was distilled under reduced pressure, and then the concentrated residue was purified by column chromatography to obtain 2-(5-ethoxymethyl-7-nitro-2-phenyl-1H-indol-3-yl)-imidazole-1-carboxylic acid ethyl ester (100 mg, yield 39%).

¹H NMR (400 MHz, DMSO-d₆); δ 12.06 (bs, 1H), 8.12 (s, 1H), 7.79 (s, 1H), 7.72 (d, 1H), 7.48-7.33 (m, 5H), 7.24 (d, 1H), 4.61 (s, 2H), 3.92 (q, 2H), 3.52 (q, 2H), 1.16 (t, 3H), 0.88 (t, 3H).

### Step 3: Ethyl 2-(7-amino-5-(ethoxymethyl)-2-phenyl-1H-indol-3-yl)-1H-imidazole-1 -carboxylate

From the compound (100 mg, 0.23 mmol) obtained in Step 2, ethyl 2-(7-amino-5-(ethoxymethyl)-2-phenyl-1H-indol-3-yl)-1H-imidazole-1-carboxylate (60 mg, yield 64%) was obtained using the same method as in Preparation Example 4.

¹H NMR (400 MHz, DMSO-d₆); δ 11.21 (bs, 1H), 7.72 (d, 1H), 7.52-7.33 (m, 5H), 7.24 (d, 1H), 6.57 (s, 1H), 6.45 (s, 1H), 5.31 (bs, 2H), 4.40 (s, 2H), 3.96 (q, 2H), 3.47 (q, 2H), 1.17 (t, 3H), 0.92 (t, 3H).

### Preparation Example 24: 2-phenyl-5-(propoxymethyl)-1H-indol-7-amine

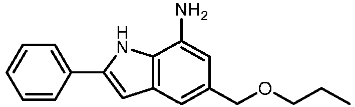

### Step 1: 7-nitro-2-phenyl-5-(propoxymethyl)-1H-indole

5-(bromomethyl)-7-nitro-2-phenyl-1H-indole (1.0 g, 3.02 mmol) obtained in Preparation Example 2 was added to propanol (20 mL), and the suspension was dissolved by adding a minimum amount of THF thereto while heating it to reflux. After heating it to reflux and stirring overnight, it was distilled under reduced pressure, whereby the concentrated residue was purified by column chromatography to obtain 7-nitro-2-phenyl-5-propoxymethyl-1H-indole (0.82 g, yield 87%).

¹H NMR (400 MHz, DMSO-d₆); δ 11.62 (bs, 1H), 8.10-7.96 (m, 4H), 7.51 (t, 2H), 7.46-7.35 (m,1H), 7.16 (s, 1H), 4.63 (s, 2H), 3.45 (t, 2H), 1.69-1.53 (m, 2H), 0.92 (t, 3H).

### Step 2: 2-phenyl-5-(propoxymethyl)-1H-indol-7-amine

From the compound (0.82 g, 2.64 mmol) obtained in Step 1, 2-phenyl-5-(propoxymethyl)-1H-indol-7-amine (0.6 g, yield 81%) was obtained using the same method as in Preparation Example 4.

¹H NMR (400 MHz, DMSO-d₆); δ 10.90 (bs, 1H), 7.81 (d, 2H), 7.47 (t, 2H), 7.31 (t, 1H), 6.77 (d, 1H), 6.74 (s, 1H), 6.34 (s, 1H), 5.17 (bs, 2H), 4.38 (s, 2H), 3.45-3.30 (m, 2H), 1.60-1.48 (m, 2H), 0.88 (t, 3H).

### Preparation Example 25: 5-((cyclopropylmethoxy)methyl)-2-phenyl-1H-indol-7-amine

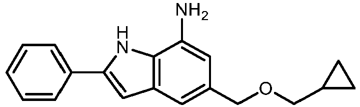

### Step 1: 5-((cyclopropylmethoxy)methyl)-7-nitro-2-phenyl-1H-indole

The compound 5-(bromomethyl)-7-nitro-2-phenyl-1H-indole (0.5 g, 1.51 mmol) obtained in Preparation Example 2 was added to cyclopropylmethanol (10 mL) and heated to reflux overnight. After the reaction mixture was distilled under reduced pressure, the concentrated residue was purified by column chromatography to obtain 5-cyclopropylmethoxymethyl-7-nitro-2-phenyl-1H-indole (400 mg, yield 82%).

¹H NMR (400 MHz, DMSO-d₆); δ 11.61 (bs, 1H), 8.06-7.96 (m, 4H), 7.49 (t, 2H), 7.40 (m, 1H), 7.15 (s, 1H), 4.64 (s, 2H), 3.33 (d, 1H) 1.15-1.02 (m, 1H), 0.52-0.44 (m,2H), 0.23-0.15 (m, 2H).

### Step 2: 5-((cyclopropylmethoxy)methyl)-2-phenyl-1H-indol-7-amine

From the compound (400 mg, 1.24 mmol) obtained in Step 1, 5-((cyclopropylmethoxy)methyl)-2-phenyl-1H-indol-7-amine (245 mg, yield 68%) was obtained using the same method as in Preparation Example 4.

¹H NMR (400 MHz, DMSO-d₆); δ 10.88 (bs, 1H), 7.81 (d, 2H), 7.47 (t, 2H), 7.30 (t, 1H), 6.76 (s, 1H), 6.73 (s, 1H), 6.34 (s, 1H), 5.16 (bs, 2H), 4.39 (s, 2H), 3.23 (d, 1H) 1.08-0.95 (m, 1H), 0.50-0.40 (m,2H), 0.20-0.09 (m, 2H).

### Preparation Example 26: 5-(2-methoxyethyl)-2-phenyl-1H-indol-7-amine

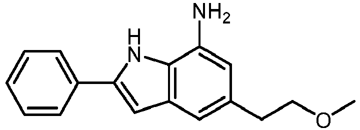

### Step 1: Ethyl 2-(4-aminophenyl)acetate

Commercially available ethyl 2-(4-nitrophenyl)acetate (20 g, 95.6 mmol) was dissolved in a 1:1:1 mixed solution (300 mL) of THF, methanol and water. Iron powder (26.7 g, 478 mmol) and NH₄Cl (25.6 g, 478 mmol) were added thereto, and the mixture was stirred at 60°C for 2 hours. The completion of the reaction was confirmed by TLC, and the mixture was filtered through Celite at room temperature, and then concentrated under reduced pressure. The organic layer was diluted with EA, washed with a saturated NH₄Cl solution, extracted with EA, and then dried over MgSO₄, and then the filtered filtrate was concentrated under reduced pressure. Ethyl 2-(4-aminophenyl)acetate (17 g, yield 99%) was obtained.

Mass [M + H] = 180.09 (M +1)

### Step 2: Ethyl 2-(4-acetamidophenyl)acetate

Ethyl 2-(4-aminophenyl)acetate (17 g, 94.87 mmol) obtained in Step 1 was dissolved in DCM (170 mL), TEA (19.8 mL, 142.3 mmol) was introduced thereto, and then acetic anhydride (11.6 g, 113.84 mmol) was slowly added dropwise thereto at 0°C. The mixture was stirred at room temperature for 1 hour. After confirming the completion of the reaction by TLC, the reaction mixture was diluted with DCM and washed with a saturated sodium carbonate solution and a saturated NaCl solution in this order. The organic layer was dried over MgSO₄, and then the filtered filtrate was concentrated under reduced pressure. The concentrated residue was recrystallized from hexane and then filtered to obtain ethyl 2-(4-acetamidophenyl)acetate (20.6 g, yield 98%) as a pink solid.

¹H NMR (400MHz, CDCl₃); δ 7.48 (d, 2H), 7.32 (br, 1H), 7.26 (d, 2H), 4.17 (q, 2H), 3.59 (s, 2H), 2.18 (s, 3H), 1.27 (t, 3H).

Mass [M + H] = 222.11 (M + 1)

### Step 3: Ethyl 2-(4-acetamido-3-nitrophenyl)acetate

Ethyl 2-(4-acetamidophenyl)acetate (20.6 g, 93.1 mmol) obtained in Step 2 was dissolved in acetic anhydride (309 mL), and red fuming nitric acid was slowly added dropwise thereto at 0°C. The mixture was heated to room temperature and stirred for 4 hours. The completion of the reaction was confirmed by TLC, water was added, and the resulting solid was filtered and dried. Ethyl 2-(4-acetamido-3-nitrophenyl)acetate (24 g, 92%) was obtained.

¹H NMR (400MHz, CDCl₃); δ 10.31 (br, 1H), 8.76 (d, 1H), 8.17 (s, 1H), 7.60 (d, 1H), 4.20 (q, 2H), 3.67 (s, 2H), 2.31 (s, 3H), 1.29 (t, 3H).

Mass [M + H] = 267.09 (M +1)

### Step 4: Ethyl 2-(4-amino-3-nitrophenyl)acetate

Ethyl 2-(4-acetamido-3-nitrophenyl)acetate (24 g, 86 mmol) obtained in Step 3 was dissolved in ethanol (300 mL) and 1N HCl (160 mL) was added dropwise thereto. After stirring at room temperature overnight, the mixture was further heated to reflux overnight. The completion of the reaction was confirmed by TLC, the reaction solution was concentrated, and then the organic layer was washed with saturated NaHCO₃, and then dried over MgSO₄, and then the filtered filtrate was concentrated. Ethyl 2-(4-amino-3-nitrophenyl)acetate (16 g, 82%) was obtained as an orange solid.

¹H NMR (400MHz, CDCl₃); δ 8.05 (s, 1H), 7.35 (d, 1H), 6.82 (d, 1H), 6.06 (br, 1H), 4.19 (q, 2H), 3.56 (s, 2H), 1.29 (t, 3H).

Mass [M + H] = 225.08 (M +1)

### Step 5: Ethyl 2-(4-amino-3-iodo-5-nitrophenyl)acetate

Ethyl 2-(4-amino-3-nitrophenyl)acetate (16 g, 71.27 mmol) obtained in Step 4 was dissolved in ethanol (480 mL), silver nitrate (14.5 g, 85.52 mmol), iodine (21.7 g), 85.52 mmol) and stirred at room temperature overnight. After confirming the completion of the reaction by TLC, the reaction solution was concentrated and then diluted with EA. The organic layer was washed three times with saturated sodium thiosulfate, dried over MgSO₄, and then the filtered filtrate was concentrated. The concentrated residue was purified by column chromatography (Hex/EA, 5/1) to obtain ethyl 2-(4-amino-3-iodo-5-nitrophenyl)acetate (17 g, yield 68%).

¹H NMR (400MHz, CDCl₃); δ 8.11 (s, 1H), 7.91 (s, 1H), 6.65 (br, 1H), 4.20 (q, 2H), 3.53 (s, 2H), 1.30 (t, 3H).

Mass [M + H] = 350.98 (M +1)

### Step 6: Ethyl-2-(7-nitro-2-phenyl-1H-indol-5-yl)acetate

Ethyl-2-(4-amino-3-iodo-5-nitrophenyl)acetate (5.67 g, 16.2 mmol) obtained in Step 5 was dissolved in anhydrous dioxane (100 mL), and TEA (6.77 mL, 48.6 mmol), phenylacetylene (2.14 mL, 19.44 mmol), bistriphenyl phosphine palladium dichloride (114 mg, 0.162 mmol), and copper iodide (30.85 mg, 0.162 mmol) were introduced thereto in this order. The mixture was reacted at 60°C for 18 hours, and 1,8-diazabicyclo[5,4,0]undec-7-ene (18.3 mL, 129.6 mmol) was added to the reaction liquid, which was reacted at 110°C for 48 hours. It was filtered through Celite at room temperature, and the filtrate was distilled under reduced pressure. Ethyl acetate was added to the concentrated residue, which was washed with water. The aqueous layer was re-extracted with EA, and the combined organic layers were dried over MgSO₄ and filtered. The filtrate was concentrated under reduced pressure, and the concentrated residue was purified by silica column chromatography to obtain ethyl-2-(7-nitro-2-phenyl-1H-indol-5-yl)acetate (3.3 g, yield 63%) as an orange solid.

¹H NMR (400MHz, CDCl₃); δ 10.08 (br, 1H), 8.10 (s, 1H), 7.90 (s, 1H), 7.77 (d, 2H), 7.52 (t, 2H), 7.42 (t, 1H), 6.93 (s, 1H), 4.22 (q, 2H), 3.80 (2, 1H), 1.30 (t, 3H).

### Step 7: tert-Butyl 5-(2-ethoxy-2-oxoethyl)-7-nitro-2-phenyl-1H-indole-1-carboxylate

Ethyl-2-(7-nitro-2-phenyl-1H-indol-5-yl)acetate (2.33 g, 7.18 mmol) obtained in Step 6 was dissolved in DCM (50 mL), and TEA (2.0 mL, 14.37 mmol), DMAP (88 mg, 0.718 mmol) and di-tert-butyl dicarbonate (2.35 g, 10.77 mmol) were added, and the mixture was reacted at room temperature for 16 hours. A saturated NH₄Cl aqueous solution was added thereto, and the mixture was extracted with DCM. The organic layer was dried over MgSO₄ and filtered. The filtrate was concentrated under reduced pressure, and the concentrated residue was purified by silica column chromatography to obtain tert-butyl 5-(2-ethoxy-2-oxoethyl)-7-nitro-2-phenyl-1H-indole-1-carboxylate. (2.53 g, yield 83%) as a yellow liquid.

¹H NMR (400MHz, CDCl₃); δ 7.85 (s, 1H), 7.78 (s, 1H), 7.51 (m, 2H), 7.46 (m, 3H), 6.61 (s, 1H), 4.22 (q, 2H), 3.79 (s, 2H), 1.30 (m, 12H).

### Step 8: 2-(1-(tert-butoxycarbonyl)-7-nitro-2-phenyl-1H-indol-5-yl)acetic acid

tert-Butyl 5-(2-ethoxy-2-oxoethyl)-7-nitro-2-phenyl-1H-indole-1-carboxylate (2.53 g, 5.96 mmol) obtained in Step 7 was introduced to a 1:1:1 mixed solution (90 mL) of THF, methanol, and water, and lithium hydroxide hydrate (500 mg, 11.92 mmol) was added thereto. The mixture was reacted for 15 hours, and concentrated under reduced pressure. The pH was adjusted to 6-7 using a 2N aqueous hydrochloric acid solution and a saturated aqueous NH₄Cl solution at 0°C. The mixture was extracted with EA, dried over MgSO₄, and filtered. The filtrate was concentrated under reduced pressure, and the concentrated residue was purified by silica column chromatography to obtain 2-(1-(tert-butoxycarbonyl)-7-nitro-2-phenyl-1H-indol-5-yl)acetic acid (1.1 g, yield 47%) as a yellow solid.

¹H NMR (400MHz, CDCl₃); δ 7.85 (s, 1H), 7.78 (s, 1H), 7.51 (m, 2H), 7.46 (m, 3H), 6.62 (s, 1H), 3.85 (s, 2H), 1.30 (s, 9H).

### Step 9: tert-Butyl 5-(2-hydroxyethyl)-7-nitro-2-phenyl-1H-indole-1-carboxylate

2-(1-(tert-butoxycarbonyl)-7-nitro-2-phenyl-1H-indol-5-yl)acetic acid (1.1 g, 2.78 mmol) obtained in Step 8 was dissolved in THF (100 mL) and cooled to 5°C. 2M BH₃·SMe₂ in THF (4.16 mL, 8.325 mmol) was slowly added dropwise thereto, and the mixture was reacted at room temperature for 18 hours. A 1N aqueous sodium hydroxide solution (20 mL) was slowly added dropwise thereto at -5°C, and the mixture was stirred at room temperature for 1 hour. The mixture was diluted with EA, and washed with water. The organic layer was dried over MgSO₄, and filtered. The filtrate was concentrated under reduced pressure, and the concentrated residue was purified by silica column chromatography to obtain tert-butyl 5-(2-hydroxyethyl)-7-nitro-2-phenyl-1H-indole-1-carboxylate (990 mg, yield 93%) as a yellow solid.

¹H NMR (400MHz, CDCl₃); δ 7.80 (s, 1H), 7.72 (s, 1H), 7.54 (m, 2H), 7.46 (m, 3H), 6.60 (s, 1H), 3.98 (t, 2H), 3.05 (t, 2H), 1.44 (t, 1H), 1.30 (s, 9H).

### Step 10: tert-Butyl 5-(2-methoxyethyl)-7-nitro-2-phenyl-1H-indole-1-carboxylate

tert-Butyl 5-(2-hydroxyethyl)-7-nitro-2-phenyl-1H-indole-1-carboxylate (700 mg, 1.83 mmol) obtained in Step 9 was dissolved in THF (20 mL) and cooled to 0°C. NaH 55%/mineral oil (160 mg, 3.66 mmol) was introduced thereto, and after 30 minutes, MeI (171 µL, 2.745 mmol) was added dropwise thereto. The mixture was reacted at room temperature for 20 hours, and the reaction liquid was diluted with water, and extracted twice with EA. The organic layer was dried over anhydrous MgSO₄, and filtered. The filtrate was concentrated under reduced pressure, and the concentrated residue was purified by silica column chromatography to obtain tert-butyl 5-(2-methoxyethyl)-7-nitro-2-phenyl-1H-indole-1-carboxylate (306 mg, yield 42%) as a yellow solid.

¹H NMR (400MHz, CDCl₃); δ 7.79 (s, 1H), 7.70 (s, 1H), 7.54 (m, 2H), 7.46 (m, 3H), 6.59 (s, 1H), 3.69 (t, 2H), 3.39 (s, 3H), 3.05 (t, 2H), 1.30 (s, 9H).

### Step 11: 5-(2-methoxyethyl)-7-nitro-2-phenyl-1H-indole

tert-Butyl 5-(2-methoxyethyl)-7-nitro-2-phenyl-1H-indole-1-carboxylate obtained in Sep 10 (306 mg, 0.828 mmol) was dissolved in DCM (10 mL), and trifluoroacetic acid (2 mL) was added dropwise thereto. The mixture was reacted at room temperature for 16 hours, and a saturated aqueous NaHCO₃ solution (20 mL) was added dropwise thereto. The organic layer was dried over MgSO₄, and filtered. The filtrate was concentrated under reduced pressure, and the concentrated residue was purified by silica column chromatography to obtain 5-(2-methoxyethyl)-7-nitro-2-phenyl-1H-indole (264 mg, yield 99%) as a yellow solid.

¹H NMR (400MHz, CDCl₃); δ 10.04 (br, 1H), 8.05 (s, 1H), 7.84 (s, 1H), 7.77 (d, 2H), 7.53 (t, 2H), 7.41 (t, 1H), 6.91 (s, 1H), 3.71 (t, 2H), 3.40 (s, 3H), 3.07 (t, 2H).

### Step 12: 5-(2-methoxyethyl)-2-phenyl-1H-indol-7-amine

From the compound 5-(2-methoxyethyl)-7-nitro-2-phenyl-1H-indole (287 mg, 1.066 mmol) as obtained above, 5-(2-methoxyethyl)-2-phenyl-1H-indol-7-amine (100 mg, yield 35%) was obtained using the same method as in Preparation Example 4.

¹H NMR (400MHz, CDCl₃); δ 8.05 (br, 1H), 7.70 (d, 2H), 7.45 (t, 2H), 7.30 (t, 1H), 6.70 (s, 1H), 6.77 (s, 1H), 6.42 (s, 1H), 3.68 (t, 2H), 3.42 (s, 3H), 3.20 (br, 2H), 2.98 (t, 2H).

Mass [M + H] = 267.1 (M +1)

### Preparation Example 27: 5-(3-methoxypropyl)-2-phenyl-1H-indol-7-amine

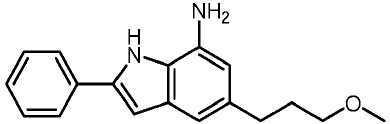

### Step 1: 7-nitro-2-phenyl-1H-indole-carbaldehyde

(7-nitro-2-phenyl-1H-indol-5-yl)methanol (5 g, 18.6 mmol) obtained in Preparation Example 1 was dissolved in DCM (300 mL) and acetone (100 mL), and pyridinium chlorochromate (5.63 g, 26.12 mmol) was introduced thereto, and the mixture was heated to reflux overnight. After confirming the completion of the reaction by TLC, it was filtered through silica gel, and then concentrated to obtain 7-nitro-2-phenyl-1H-indole-carbaldehyde (5 g, yield 100%) as a yellow solid.

¹H NMR (400MHz, CDCl₃); δ 10.34 (br, 1H), 10.15 (s, 1H), 8.68 (s, 1H), 8.51 (s, 1H), 7.79 (d, 2H), 7.57 (t, 2H), 7.49 (t, 1H), 7.12 (s, 1H).

Mass [M + H] = 267.07 (M +1)

### Step 2: tert-Butyl 5-formyl-7-nitro-2-phenyl-1H-indole-1-carboxylate

7-Nitro-2-phenyl-1H-indole-carbaldehyde (5 g, 18.7 mmol) obtained in Step 1 was dissolved in DCM (250 mL), and TEA (6.54 mL, 46.95 mmol), (Boc)₂O (8.19 g, 37.56 mmol) and DMAP (0.229 g, 1.878 mmol) were introduced thereto. The mixture was stirred at room temperature for two days. The completion of the reaction was confirmed by TLC, and the concentrated residue was purified by column chromatography (Hex/EA, 10/1) to obtain tert-butyl 5-formyl-7-nitro-2-phenyl-1H-indole-1-carboxylate (5.2 g, yield 75%).

¹H NMR (400MHz, CDCl₃); δ 10.12 (s, 1H), 8.38 (d, 2H), 7.49 (m, 5H), 6.77 (s, 1H), 1.31 (s, 9H).

Mass [M + H] = 367.12 (M +1)

### Step 3: 3-(1-(tert-butoxycarbonyl)-7-nitro-2-phenyl-1H-indol-5-yl)propionic acid

Formic acid (1.63 mL, 42.57 mmol), TEA (2.57 mL, 18.45 mmol), and 2,2-dimethyl-1,3-dioxane-4,6-dione (2.045 g, 14.19 mmol) were introduced to DMF (20 mL) at 10°C. tert-Butyl 5-formyl-7-nitro-2-phenyl-1H-indole-1-carboxylate (5.2 g, 14.19 mmol) obtained in Step 2 was added, followed by stirring at 80°C overnight. After confirming the completion of the reaction by TLC, a saturated NH₄Cl solution was added to the reaction solution at 10°C, which was stirred for 30 minutes. The organic layer was washed three times with a saturated NH₄Cl solution, and then washed again with water and washed with a saturated NaCl solution. After drying over MgSO₄, the filtered filtrate was concentrated. 3-(1-(tert-butoxycarbonyl)-7-nitro-2-phenyl-1H-indol-5-yl)propionic acid (6 g, yield 104%) was obtained as orange oil.

¹H NMR (400MHz, CDCl₃); δ 7.85 (s, 1H), 7.78 (s, 1H), 7.51 (m, 2H), 7.46 (m, 2H), 6.61 (s, 1H), 3.84 (s, 2H), 1.30 (s, 9H).

Mass [M + H] = 410.15 (M +1)

### Step 4: tert-Butyl 5-(3-hydroxypropyl)-7-nitro-2-phenyl-1H-indole-1-carboxylate

3-(1-(t-butoxycarbonyl)-7-nitro-2-phenyl-1H-indol-5-yl)propionic acid (6 g, 14.76 mmol) obtained in Step 3 was dissolved in THF (120 mL) and cooled to 0°C. BH₃·SMe₂ 2M in THF (22.05 mL, 44.28 mmol) was added, and the mixture was heated to room temperature, and stirred for 5 hours. The completion of the reaction was confirmed by TLC, and the mixture was cooled to 0°C. A 1 N NaOH aqueous solution was slowly added dropwise thereto, and the mixture was stirred for 1 hour. It was extracted twice with EA, and the organic layer was dried over MgSO₄, filtered, and concentrated under reduced pressure. The concentrated residue was purified by silica gel column chromatography (Hex/EA, 2/1) to obtain tert-butyl 5-(3-hydroxypropyl)-7-nitro-2-phenyl-1H-indole-1-carboxylate (3.2 g, yield 55%) as a yellow oil.

¹H NMR (400MHz, CDCl₃); δ 7.76 (s, 1H), 7.67 (s, 1H), 7.54 (m, 2H), 7.44 (m, 3H), 6.58 (s, 1H), 3.75 (t, 2H), 2.91 (t, 2H), 1.99 (m, 2H), 1.30 (s, 9H).

Mass [M + H] = 397.17 (M +1)

### Step 5: tert-Butyl 5-(3-methoxypropyl)-7-nitro-2-phenyl-1H-indole-1-carboxylate

tert-Butyl 5-(3-hydroxypropyl)-7-nitro-2-phenyl-1H-indole-1-carboxylate (300 mg, 0.757 mmol) obtained in Step 4 was dissolved in THF (5 mL), and cooled to 0°C. NaH (55%, dispersion in paraffin liquid, 36 mg, 1.513 mmol) was added thereto, and the mixture was stirred at the same temperature for 30 minutes. MeI (0.47 mL, 7.57 mmol) was added dropwise thereto, and the mixture was stirred at room temperature overnight. After confirming the completion of the reaction by TLC, the reaction solution was poured into ice water and stirred for 10 minutes. It was extracted twice with EA and washed with a saturated NaCl solution. The organic layer was dried over MgSO₄, filtered, and concentrated under reduced pressure. The concentrated residue was purified by silica gel column chromatography (Hex/EA, 7/1) to obtain tert-butyl 5-(3-methoxypropyl)-7-nitro-2-phenyl-1H-indole-1-carboxylate (146 mg, yield 54%) as a yellow solid.

¹H NMR (400MHz, CDCl₃); δ 7.75 (s, 1H), 7.66 (s, 1H), 7.54 (m, 2H), 7.45 (m, 3H), 6.58 (s, 1H), 3.45 (t, 2H), 3.38 (s, 3H), 2.88 (t, 2H), 2.00 (m, 2H), 1.30 (s, 9H).

Mass [M + H] = 411.47 (M +1)

### Step 6: 5-(3-methoxypropyl)-7-nitro-2-phenyl-1H-indole

tert-Butyl 5-(3-methoxypropyl)-7-nitro-2-phenyl-1H-indole-1-carboxylate (146 mg, 0.368 mmol) obtained in Step 5 was dissolved in DCM (10 mL), and trifluoroacetic acid (0.137 mL, 1.84 mmol) was added dropwise thereto, and the mixture was stirred at room temperature overnight. The completion of the reaction was confirmed by TLC, and the mixture was cooled to 0°C. It was extracted twice with DCM, and washed with a saturated NaHCOa solution. The organic layer was dried over MgSO₄, filtered, and concentrated under reduced pressure. 5-(3-methoxypropyl)-7-nitro-2-phenyl-1H-indole (103 mg, yield 90%) was obtained as a yellow solid.

¹H NMR (400MHz, CDCl₃); δ 10.03 (br, 1H), 8.02 (s, 1H), 7.80 (s, 1H), 7.75 (d, 1H), 7.53 (t, 2H), 7.43 (t, 1H), 6.91 (s, 1H), 3.46 (t, 2H), 3.39 (s, 3H), 2.89 (t, 2H), 2.01 (m, 2H).

Mass [M + H] = 311.35 (M +1)

### Step 7: 5-(3-methoxypropyl)-7-nitro-2-phenyl-1H-indol-7-amine

5-(3-methoxypropyl)-7-nitro-2-phenyl-1H-indole (103 mg, 0.332 mmol) obtained in Step 6 was dissolved in THF (2 mL), EA (1 mL), and palladium carbon 50%/water (39 mg, 30 wt%) was introduced thereto. A hydrogen balloon was connected, and the mixture was stirred for 1 hour. The reaction solution was filtered through Celite, and then concentrated under reduced pressure, filtered through silica gel, and then concentrated under reduced pressure to obtain the title compound 5-(3-methoxypropyl)-7-nitro-2-phenyl-1H-indol-7-amine (88 mg, yield 80%) as a yellow solid.

¹H NMR (400MHz, CDCl₃); δ 8.37 (br, 1H), 7.68 (d, 2H), 7.43 (t, 2H), 7.31 (t, 1H), 7.02 (s, 2H), 6.75 (s, 1H), 6.51 (s, 1H), 3.42 (t, 2H), 3.36 (s, 3H), 2.71 (t, 2H), 1.93 (m, 2H).

Mass [M + H] = 311.35 (M +1)

In the following, the compounds of the examples of the present invention were prepared using the compounds prepared in Preparation Examples 1 through 27 above.

### Example 1: 5-(methoxymethyl)-2-phenyl-N-tetrahydropyran-4-yl-1H-indol-7-amine

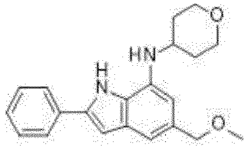

The 5-(methoxymethyl-2-phenyl-1H-indol-7-amine obtained in Preparation Example 4 (100 mg, 0.396 mmol) was dissolved in DCM (12 mL), then tetrahydro-4H-pyran-4-one (60 mg, 0.594 mmol) was added. After stirring for 30 minutes, NaBH(OAc)₃ (336 mg, 1.584 mmol) was added. After stirring for 4 hours at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 2/1) to obtain the title compound (60 mg, 45%) as a yellow solid.

¹H NMR (400MHz, DMSO-d₆); δ 10.97 (s, 1H), 7.81 (d, 2H), 7.48 (t, 2H), 7.31 (t, 1H), 6.76 (s, 2H), 6.28 (s, 1H), 5.38 (m, 1H), 4.36 (s, 2H), 3.94 (m, 2H), 3.53 (v, 1H), 3.50 (m, 2H), 3.24 (s, 3H), 2.04 (m, 2H), 1.48 (m, 2H).

Mass [M+H] = 337.0 (M+1)

### Example 2: N-cyclopentyl-5-(methoxymethyl)-2-phenyl-1H-indol-7-amine

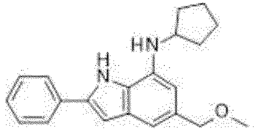

The 5-(methoxymethyl-2-phenyl-1H-indol-7-amine obtained in Preparation Example 4 (100 mg, 0.396 mmol) was dissolved in DCM (12 mL), then cyclopentanone (50 mg, 0.594 mmol) was added. After stirring for 30 minutes, NaBH(OAc)₃ (336 mg, 1.584 mmol) was added. After stirring for 3 hours at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 4/1) to obtain the title compound (90 mg, 71%) as a yellow solid.

¹H NMR (400MHz, DMSO-d₆); δ 10.90 (s, 1H), 7.80 (d, 2H), 7.46 (t, 2H), 7.30 (t, 1H), 6.75 (s, 2H), 6.22 (s, 1H), 5.39 (d, 1H), 4.37 (s, 2H), 3.90 (v, 1H), 3.25 (s, 3H), 2.05 (v, 2H), 1.76 (v, 2H), 1.61 (v, 4H).

Mass [M+H] = 320.9 (M+1)

### Example 3: 5-(methoxymethyl)-2-phenyl-N-(tetrahydropyran-4-ylmethyl)-1H-indol-7-amine

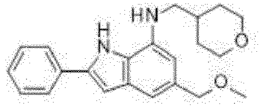

The 5-(methoxymethyl-2-phenyl-1H-indol-7-amine obtained in Preparation Example 4 (100 mg, 0.396 mmol) was dissolved in DCM (12 mL), then tetrahydropyran-4-carbaldehyde (68 mg, 0.596 mmol) was added. After stirring for 30 minutes, NaBH(OAc)₃ (336 mg, 1.584 mmol) was added. After stirring for 1 hour at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 2/1) to obtain the title compound (55 mg, 40%) as a light brown solid.

¹H NMR (400MHz, DMSO-d₆); δ 10.97 (br, 1H), 7.79 (d, 2H), 7.47 (t, 2H), 7.31 (t, 1H), 6.77 (s, 2H), 6.20 (s, 1H), 5.44 (m, 1H), 4.37 (s, 2H), 3.90 (m, 1H), 3.25 (s, 3H), 3.10 (m, 2H), 1.92 (m, 2H), 1.77 (m, 2H), 1.32 (m, 2H).

Mass [M+H] = 351.1 (M+1)

### Example 4: N-(1,1-dioxothian-4-yl)-5-(methoxymethyl)-2-phenyl-1H-indol-7-amine {N-(1,1-dioxothian-4-yl)-5-(methoxymethyl)-2-phenyl-1H-indol-7-amine}

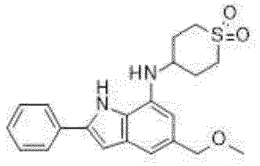

The 5-(methoxymethyl-2-phenyl-1H-indol-7-amine obtained in Preparation Example 4 (170 mg, 0.674 mmol) was dissolved in DCM (30 mL), then tetrahydro-4H-thiopyran-4-on 1,1-dioxide (150 mg, 1.011 mmol) was added. After stirring for 30 minutes, NaBH(OAc)₃ (571 mg, 2.696 mmol) was added. After stirring for 2 hours at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 1/1) to obtain the title compound (130 mg, 50%) as a light brown solid.

¹H NMR (400MHz, DMSO-d₆); δ 10.92 (br, 1H), 7.80 (d, 2H), 7.48 (t, 2H), 7.30 (t, 1H), 6.80 (s, 2H), 6.31 (s, 1H), 5.45 (d, 1H), 4.37 (s, 2H), 3.90 (m, 1H), 3.24 (s, 3H), 3.30-3.10 (m, 4H), 2.35 (m, 2H), 1.99 (m, 2H).

Mass [M+H] = 385.0 (M+1)

### Example 5: 5-(methoxymethyl)-N-(1-methylsulfonyl-4-piperidyl)-2-phenyl-1H-indol-7-amine

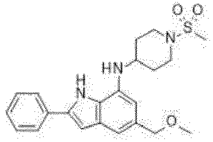

The 5-(methoxymethyl-2-phenyl-1H-indol-7-amine obtained in Preparation Example 4 (176 mg, 0.698 mmol) was dissolved in DCM (50 mL), then 1-(methylsulfonyl)piperidine-4-one (185 mg, 1.046 mmol) was added. After stirring for 30 minutes, NaBH(OAc)₃ (591 mg, 2.79 mmol) was added. After stirring for 4 hours at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 1/1) to obtain the title compound (170 mg, 59%) as a light brown solid.

¹H NMR (400MHz, DMSO-d₆); δ 10.96 (br, 1H), 7.80 (d, 2H), 7.47 (t, 2H), 7.33 (t, 1H), 6.78 (s, 2H), 6.28 (s, 1H), 5.40 (d, 1H), 4.37 (s, 2H), 3.60 (v, 3H), 3.24 (s, 3H), 2.99 (m, 2H), 2.91 (s, 3H), 2.16 (m, 2H), 1.50 (m, 2H).

Mass [M+H] = 413.9 (M+1)

### Example 6: 1-[4-[[5-(methoxymethyl)-2-phenyl-1H-indol-7-yl]amino]-1-piperidyl]ethanone

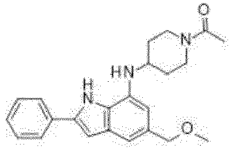

The 5-(methoxymethyl-2-phenyl-1H-indol-7-amine obtained in Preparation Example 4 (100 mg, 0.396 mmol) was dissolved in DCM (10 mL), then 1-acetylpiperidine-4-one (84 mg, 0.594 mmol) was added. After stirring for 30 minutes, NaBH(OAc)₃ (336 mg, 1.584 mmol) was added. After stirring for 15 hours at room temperature, completion of the reaction was confirmed using TLC, and 1N NaOH₃ aqueous solution was added, followed by stirring for 1 hour. 100 mL DCM was additionally added before layer separation, after which the organic layer was dried with MgSO₄ and filtered. The filtrate was concentrated under reduced pressure, and the concentration residue was purified by silica gel column chromatography (DCM/MeOH, 95/5) to obtain the title compound (26 mg, 17%) as a light brown solid.

¹H NMR (400MHz, DMSO-d₆); δ 10.91 (br, 1H), 7.76 (d, 2H), 7.43 (t, 2H), 7.41 (t, 1H), 6.73 (s, 2H), 6.26 (s, 1H), 5.32 (d, 1H), 4.33 (s, 2H), 3.66 (m, 2H), 3.25(m, 2H), 3.20 (s, 3H), 2.39 (m, 2H), 2.28 (m, 2H), 2.04 (s, 3H).

Mass [M+H] = 378.2 (M+1)

### Example 7: N-(4,4-difluorocyclohexyl)-5-(methoxymethyl)-2-phenyl-1H-indol-7-amine

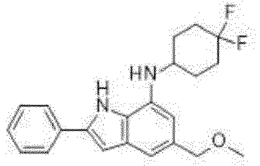

The 5-(methoxymethyl-2-phenyl-1H-indol-7-amine obtained in Preparation Example 4 (150 mg, 0.594 mmol) was dissolved in DCM (10 mL), then 4,4-difluorocyclohexane-1-one (120mg, 0.891 mmol) was added. After stirring for 30 minutes, NaBH(OAc)₃ (504 mg, 2.38 mmol) was added. After stirring for 3 hours at room temperature, it was confirmed using TLC that the starting materials had disappeared, then 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 4/1) to obtain the title compound (160 mg, 73%) as a yellow solid.

¹H NMR (400MHz, CDCl₃); δ 8.12 (br, 1H), 7.66 (d, 2H), 7.42 (t, 2H), 7.30 (t, 1H), 7.08 (s, 1H), 6.76 (s, 1H), 6.54 (s, 1H), 4.50 (s, 2H), 3.58 (m, 1H), 3.37 (s, 3H), 2.16 (m, 4H), 2.00-1.58 (m, 4H).

Mass [M+H] = 371.2 (M+1)

### Example 8: N-benzyl-5-(methoxymethyl)-2-phenyl-1H-indol-7-amine

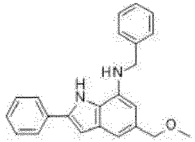

The 5-(methoxymethyl-2-phenyl-1H-indol-7-amine obtained in Preparation Example 4 (50 mg, 0.198 mmol) was dissolved in DCM (2.5 mL), then benzaldehyde (20 mg, 0.198 mmol) was added. After stirring for 30 minutes, NaBH(OAc)₃ (126 mg, 0.594 mmol) was added. After stirring for 1 hour at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 4/1) to obtain the title compound (30 mg, 44%) as a light brown solid.

¹H NMR (400MHz, CDCl₃); δ 8.21 (br, 1H), 7.59 (d, 2H), 7.45 (d, 2H), 7.40 -7.26 (m, 6H), 7.11 (s, 1H), 6.76 (s, 1H), 6.62(s, 1H), 4.49 (s, 2H), 4.46 (s, 2H), 3.35 (s, 3H).

Mass [M+H] = 343 (M+1)

### Example 9: tert-butyl 4-[[5-(methoxymethyl)-2-phenyl-1H-indol-7-yl]amino]-piperidine-1-carboxylate

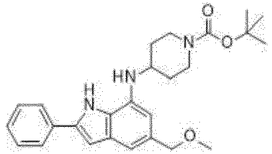

The 5-(methoxymethyl-2-phenyl-1H-indol-7-amine obtained in Preparation Example 4 (50 mg, 0.198 mmol) was dissolved in DCM (10 mL), then tert-butyl 4-oxopiperidine-1-carboxylate (39 mg, 0.198 mmol) was added. After stirring for 30 minutes, NaBH(OAc)₃ (126 mg, 0.594 mmol) was added. After stirring for 15 hours at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 2/1) to obtain the title compound (81 mg, 95%) as a light brown solid.

¹H NMR (400MHz, CDCl₃); δ 9.40 (br, 1H), 7.67 (d, 2H), 7.37 (t, 2H), 7.25 (m, 1H), 6.99 (s, 1H), 6.74 (s, 1H), 6.47 (s, 1H), 4.50 (s, 2H), 4.11 (m, 2H), 3.61 (m, 1H), 3.36 (s, 3H). 2.92 (m, 2H), 2.11 (m, 2H), 1.43 (m, 9H), 1.43-1.22 (m, 2H).

Mass [M+H] = 436 (M+1)

### Example 10: N-isopropyl-5-(methoxymethyl)-2-phenyl-1H-indol-7-amine

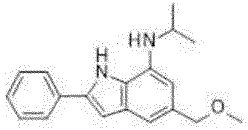

The 5-(methoxymethyl-2-phenyl-1H-indol-7-amine obtained in Preparation Example 4 (50 mg, 0.198 mmol) was dissolved in DCM (2.5 mL), then acetone (12 mg, 0.198 mmol) was added. After stirring for 30 minutes, NaBH(OAc)₃ (126 mg, 0.594 mmol) was added. After stirring for 15 hours at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 4/1) to obtain the title compound (40 mg, 70%) as a light brown solid.

¹H NMR (400MHz, CDCl₃); δ 8.17 (br, 1H), 7.69 (d, 2H), 7.45 (t, 2H), 7.30 (t, 1H), 7.09 (s, 1H), 6.80 (s, 1H), 6.58 (s, 1H), 4.55 (s, 2H), 3.84 (m, 1H), 3.40 (s, 3H), 1.33 (d, 6H).

Mass [M+H] = 295.2 (M+1)

### Example 11: 5-(methoxymethyl)-N-(1-methyl-4-piperidyl)-2-phenyl-1H-indol-7-amine

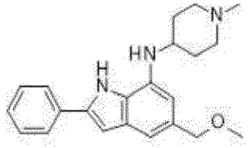

The 5-(methoxymethyl-2-phenyl-1H-indol-7-amine obtained in Preparation Example 4 (50 mg, 0.198 mmol) was dissolved in DCM (2.5 mL), then acetone1-methylpiperidine-4-one (22 mg, 0.198 mmol) was added. After stirring for 30 minutes, NaBH(OAc)₃ (126 mg, 0.594 mmol) was added. After stirring for 15 hours at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (DCM/MeOH, 90/10) to obtain the title compound (7 mg, 10%) as a light brown solid.

¹H NMR (400MHz, CDCl₃); δ 8.39 (br, 1H), 7.69 (d, 2H), 7.43 (t, 2H), 7.30 (t, 1H), 7.08 (s, 1H), 6.78 (s, 1H), 6.55 (s, 1H), 4.54 (s, 2H), 3.45 (m, 1H), 3.39 (s, 3H), 2.88 (m, 2H), 2.33 (s, 3H), 2.17 (m, 4H), 1.63 (v, 2H).

Mass [M+H] = 350.3 (M+1)

### Example 12: N-(2-methoxyethyl)-5-(methoxymethyl)-2-phenyl-1H-indol-7-amine

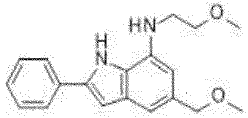

The 2-methoxy-N-[5-(methoxymethyl)-2-phenyl-1H-indol-7-yl]acetamide obtained in Preparation Example 5 (124 mg, 0.337 mmol) was dissolved in THF (1.2 mL) and cooled to 0°C, then BH₃ · SMe₂ 2M in THF (0.5 mL, 1.011 mmol) was added. After heating to room temperature, the mixture was stirred for 1 hour. Completion of the reaction was confirmed, then the mixture was cooled to 0°C. A 1N NaOH aqueous solution (5 mL) was slowly added dropwise (heat and gas generated). The mixture was heated to room temperature and stirred for 1 hour, then EA and H₂O were added to the reaction mixture for extraction. The organic layer was dried with MgSO₄ and filtered. The filtrate was concentrated under reduced pressure, and the concentration residue was purified by silica gel column chromatography (Hex/EA, 1/1) to obtain the title compound (96 mg, 80%) as a light brown solid.

¹H NMR (400MHz, CDCl₃); δ 9.31 (br, 1H), 7.67 (d, 2H), 7.43 (t, 2H), 7.30 (t, 1H), 7.14 (s, 1H), 6.78 (s, 1H), 6.59 (s, 1H), 4.54 (s, 2H), 3.76 (t, 2H), 3.53 (t, 2H), 3.50 (s, 3H), 3.41 (s, 3H).

Mass [M+H] = 311.2 (M+1)

### Example 13: 5-(methoxymethyl)-N-(3-methoxypropyl)-2-phenyl-1H-indol-7-amine

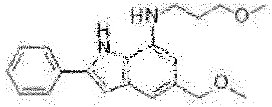

The 3-methoxy-N-[5-(methoxymethyl)-2-phenyl-1H-indol-7-yl]propenamide (133 mg, 0.396 mmol) obtained in Preparation Example 6 was dissolved in THF (1.3 mL) and cooled to 0°C, then BH₃ · SMe₂ 2M in THF (0.6 mL, 1.188 mmol) was added. After bringing to room temperature, the mixture was stirred for 1 hour. After confirming the starting materials had disappeared with TLC, the mixture was cooled to 0°C. A 1N NaOH aqueous solution (5 mL) was slowly added dropwise (caution: heat and gas generated). The mixture was heated to room temperature and stirred for 1 hour, then EA and H₂O were added to the reaction mixture for extraction. The organic layer was dried with MgSO₄ and filtered. The filtrate was concentrated under reduced pressure, and the concentration residue was purified by silica gel column chromatography (Hex/EA, 2/1) to obtain the title compound (67 mg, 65%) as a light brown solid.

¹H NMR (400MHz, CDCl₃); δ 9.86 (br, 1H), 7.70 (d, 2H), 7.45 (t, 2H), 7.30 (t, 1H), 7.11 (s, 1H), 6.79 (s, 1H), 6.57 (s, 1H), 4.54 (s, 2H), 3.70 (t, 2H), 3.45 (t, 2H), 3.42 (s, 3H), 3.41 (s, 3H), 1.99 (m, 2H).

Mass [M+H] = 325.2 (M+1)

### Example 14: 5-(methoxymethyl)-N-(1-oxothian-4-yl)-2-phenyl-1H-indol-7-amine

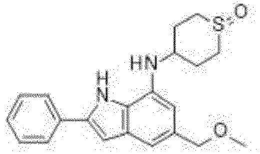

The 5-(methoxymethyl-2-phenyl-1H-indol-7-amine obtained in Preparation Example 4 (60 mg, 0.24 mmol) was dissolved in DCM (10 mL), then tetrahydro-4H-thiopyrane-4-on 1-oxide (38 mg, 0.287 mmol) was added. After stirring for 30 minutes, NaBH(OAc)₃ (203 mg, 0.96 mmol) was added. After stirring for 15 hours at room temperature, completion of the reaction was confirmed using TLC, and 1N NaOH aqueous solution was added, followed by stirring for 1 hour. After layer separation, the organic layer was dried with MgSO₄ and filtered. The filtrate was concentrated under reduced pressure, and the concentration residue was purified by silica gel column chromatography (Hex/EA, 1/1 → DCM/MeOH, 90/10) to obtain the title compound (22 mg, 25%) as a brown solid.

¹H NMR (400MHz, CDCl₃); δ 10.67 (br, 1H), 7.81 (d, 2H), 7.46 (t, 2H), 7.30 (t, 1H), 7.00 (s, 1H), 6.77 (s, 1H), 6.40 (s, 1H), 4.52 (s, 2H), 3.70 (m, 1H), 3.38 (s, 3H), 3.28 (m, 2H), 2.74 (m, 2H), 2.47 (m, 2H), 2.32 (m, 2H).

Mass [M+H] = 369.2 (M+1)

### Example 15: 5-(tert-butoxymethyl)-2-phenyl-N-tetrahydropyran-4-yl-1H-indol-7-amine

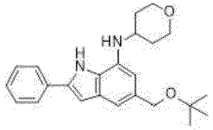

The 5-(tert-butoxymethyl)-2-phenyl-1H-indol-7-amine (40 mg, 0.123 mmol) obtained in Preparation Example 8 was dissolved in DCM (5 mL), then tetrahydro-4H-pyran-4-one (25 mg, 0.247 mmol) was added. After stirring for 30 minutes, NaBH(OAc)₃ (104 mg, 0.493 mmol) was added. The mixture was stirred for 15 hours at room temperature, and TLC was used to confirm that the starting materials had disappeared. Saturated NaHCO₃ aqueous solution was added, followed by stirring for 1 hour. The layers were separated, and the organic layer was dried with MgSO₄ and filtered. The filtrate was concentrated under reduced pressure, and the concentration residue was purified by silica gel column chromatography (Hex/EA, 1/1) to obtain the title compound (7 mg, 15%) as a brown solid.

¹H NMR (400MHz, CDCl₃); δ 7.67 (d, 2H), 7.42 (t, 2H), 7.30 (t, 1H), 7.11 (s, 1H), 6.75 (s, 1H), 6.56 (s, 1H), 4.46 (s, 2H), 4.02 (m, 2H), 3.64 (m, 1H), 3.52 (m, 2H), 2.10 (m, 2H), 1.61 (m, 2H), 1.30 (s, 9H).

Mass [M+H] = 379.3 (M+1)

### Example 16: 5-(tert-butoxymethyl)-N-cyclopentyl-2-phenyl-1H-indol-7-amine

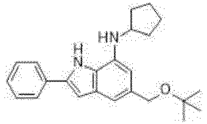

The 5-(tert-butoxymethyl)-2-phenyl-1H-indol-7-amine (20 mg, 0.062 mmol) obtained in Preparation Example 8 was dissolved in DCM (5 mL), then cyclopentanone (5.2 mg, 0.062 mmol) was added. After stirring for 30 minutes, NaBH(OAc)₃ (52 mg, 0.247 mmol) was added. The mixture was stirred for 15 hours at room temperature, and TLC was used to confirm completion of the reaction. Saturated NaHCO₃ aqueous solution was added, followed by stirring for 1 hour. The layers were separated, and the organic layer was dried with MgSO₄ and filtered. The filtrate was concentrated under reduced pressure, and the concentration residue was purified by silica gel column chromatography (Hex/EA, 3/1) to obtain the title compound (9 mg, 41%) as a brown solid.

¹H NMR (400MHz, CDCl₃); δ 8.15 (br, 1H), 7.70 (d, 2H), 7.44 (t, 2H), 7.30 (t, 1H), 7.11 (s, 1H), 6.79 (s, 1H), 6.57 (s, 1H), 4.53 (s, 2H), 4.02 (m, 1H), 2.15 (m, 2H), 1.82 (m, 2H), 1.72 (m, 4H), 1.35 (s, 9H).

Mass [M+H] = 363.3 (M+1)

### Example 17: 5-(tert-butoxymethyl)-N,N-dicyclopentyl-2-phenyl-1H-indol-7-amine

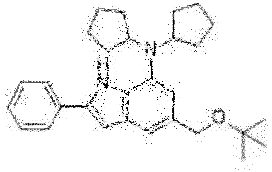

The 5-(tert-butoxymethyl)-2-phenyl-1H-indol-7-amine (20 mg, 0.062 mmol) obtained in Preparation Example 8 was dissolved in DCM (5 mL), then cyclopentanone (10.4 mg, 0.124 mmol) was added. After stirring for 30 minutes, NaBH(OAc)₃ (52 mg, 0.247 mmol) was added. The mixture was stirred for 15 hours at room temperature, and TLC was used to confirm completion of the reaction. Saturated NaHCO₃ aqueous solution was added, followed by stirring for 1 hour. The layers were separated, and the organic layer was dried with MgSO₄ and filtered. The filtrate was concentrated under reduced pressure, and the concentration residue was purified by silica gel column chromatography (Hex/EA, 3/1) to obtain the title compound (14 mg, 53%) as a brown solid.

¹H NMR (400MHz, CDCl₃); δ 8.62 (br, 1H), 7.66 (d, 2H), 7.42 (t, 2H), 7.38 (s, 1H), 7.30 (t, 1H), 7.00 (s, 1H), 6.73 (s, 1H), 4.51 (s, 2H), 3.69 (m, 2H), 1.78 (m, 4H), 1.48 (m, 8H), 1.33 (m, 4H), 1.30 (s, 9H).

Mass [M+H] = 431.3 (M+1)

### Example 18: 5-(methoxymethyl)-3-phenyl-N-tetrahydropyran-4-yl-1H-indol-7-amine

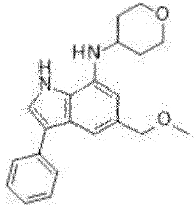

The 5-(methoxymethyl)-3-phenyl-1H-indol-7-amine (13 mg, 0.052 mmol) obtained in Preparation Example 13 was diluted in DCM (2mL), then tetrahydro-4H-pyran-4-one (7.7 mg, 0.077 mmol) was added followed by stirring for 30 minutes. NaBH(OAc)₃ (44 mg, 0.21 mmol) was added, and the mixture was stirred for 2 hours. After confirming completion of the reaction with TLC, a 1N NaOH aqueous solution was added. After 1 hour stirring, the layers were separated. The organic layer was dried with MgSO₄ and filtered. The filtrate was concentrated under reduced pressure, and the concentration residue was purified by silica gel column chromatography (Hex/EA, 1/1) to obtain the title compound (5mg, 30%) as a brown liquid.

¹H NMR (400MHz, CDCl₃); δ 8.30 (br, 1H), 7.63 (d, 2H), 7.42 (t, 2H), 7.35 (s, 1H), 7.27 (m, 2H), 6.60 (s, 1H), 4.53 (s, 2H), 4.02 (m, 2H), 3.65 (m, 1H), 3.52 (m, 2H), 3.36 (s, 3H), 2.07 (m, 2H), 1.55 (m, 2H).

Mass [M+H] = 337.0 (M+1)

### Example 19: N-cyclopentyl-5-(ethoxymethyl)-2-phenyl-1H-indol-7-amine

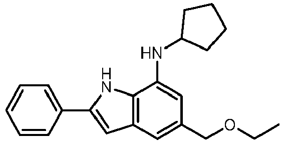

The desired title compound was obtained in the same method as in Example 2 using 5-(ethoxymethyl)-2-phenyl-1H-indol-7-amine obtained in Preparation Example 14 and cyclopentanone.

¹H NMR (400 MHz, DMSO-d₆); δ 10.89 (bs, 1H), 7.79 (d, 2H), 7.46 (t, 2H), 7.34-7.29 (m, 1H), 6.75 (s, 1H), 6.74 (s, 1H), 6.22 (s, 1H), 5.41 (d, 1H), 4.41 (s, 2H), 3.90-3.80 (m, 1H), 3.45 (q, 2H), 2.15-1.95 (m, 2H), 1.80-1.50 (m, 6H), 1.13 (t, 3H).
Mass [M + H] = 334.7 (M +1)

### Example 20: 5-(ethoxymethyl)-2-phenyl-N-(tetrahydro-2H-pyran-4-yl)-1H-indol-7-amine

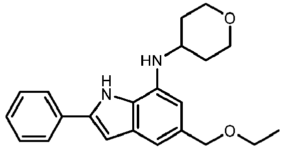

The desired title compound was obtained in the same method as in Example 1 using 5-(ethoxymethyl)-2-phenyl-1H-indol-7-amine obtained in Preparation Example 14 and tetrahydro-4H-pyran-4-one.

¹H NMR (400 MHz, DMSO-d₆); δ 10.92 (bs, 1H), 7.80 (d, 2H), 7.47 (t, 2H), 7.30 (t, 1H), 6.77 (s, 2H), 6.29 (s, 1H), 5.35 (d, 1H), 4.41 (s, 2H), 3.96-3.90 (m, 2H), 3.70-3.55 (m, 1H), 3.54-3.38 (m, 4H), 2.10-2.00 (m, 2H), 1.60-1.38 (m, 2H), 1.12 (t, 3H).

Mass [M + H] = 350.7 (M +1)

### Example 21: N-cyclopentyl-5-(isopropoxymethyl)-2-phenyl-1H-indol-7-amine

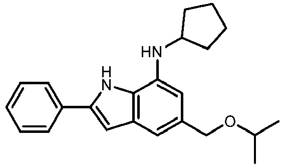

The desired title compound was obtained in the same method as in Example 2 using 5-(isopropoxymethyl)-2-phenyl-1H-indol-7-amine obtained in Preparation Example 15 and cyclopentanone.

¹H NMR (400 MHz, DMSO-d₆); δ 10.88 (bs, 1H), 7.79 (d, 2H), 7.46 (t, 2H), 7.31-7.25 (m, 1H), 6.75 (s, 1H), 6.74 (s, 1H), 6.22 (s, 1H), 5.40 (d, 1H), 4.42 (s, 2H), 3.94-3.70 (m, 1H), 3.69-3.56 (m, 1H), 2.10-1.94 (m, 2H), 1.81-1.50 (m, 6H), 1.12 (d, 2H).

Mass [M + H] = 348.8 (M +1)

### Example 22: 5-(isopropoxymethyl)-2-phenyl-N-(tetrahydro-2H-pyran-4-yl)-1H-indol-7-amine

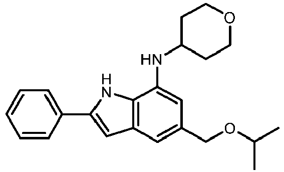

The desired title compound was obtained in the same method as in Example 1 using 5-(isopropoxymethyl)-2-phenyl-1H-indol-7-amine obtained in Preparation Example 15 and tetrahydro-4H-pyran-4-one.

¹H NMR (400 MHz, DMSO-d₆); δ 10.91 (bs, 1H), 7.80 (d, 2H), 7.47 (t, 2H), 7.30 (t, 1H), 6.76 (s, 2H), 6.29 (s, 1H), 5.34 (d, 1H), 4.42 (s, 2H), 4.00-3.88 (m, 2H), 3.70-3.56 (m, 2H), 3.49 (t, 2H), 2.12-2.00 (m, 2H), 1.60-1.38 (m, 2H), 1.11 (t, 3H).

Mass [M + H] = 364.8 (M +1)

### Example 23: 7-(cyclopentylamino)-5-(ethoxymethyl)-2-phenyl-1H-indole-3-carbonitrile

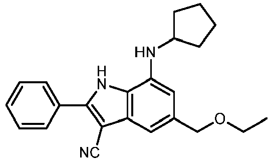

The desired title compound was obtained in the same method as in Example 2 using 7-amino-5-(ethoxymethyl)-2-phenyl-1H-indole-3-carbonitrile obtained in Preparation Example 17 and cyclopentanone.

¹H NMR (400 MHz, DMSO-d₆); δ 11.90 (bs, 1H), 7.93 (d, 2H), 7.63 (t, 2H), 7.58-7.50 (m, 1H), 6.81 (s, 1H), 6.38 (s, 1H), 5.67 (d, 1H), 4.48 (s, 2H), 3.95-3.85 (m, 1H), 3.47 (q, 2H), 2.12-1.98 (m, 2H), 1.85-1.50 (m, 6H), 1.16 (t, 3H).

Mass [M + H] = 359.8 (M +1)

### Example 24: 5-(ethoxymethyl)-2-phenyl-7-((tetrahydro-2H-pyran-4-yl)amino)-1H-indole-3-carbonitrile

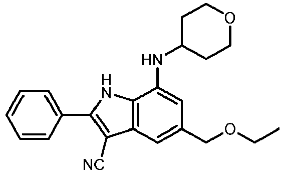

The desired title compound was obtained in the same method as in Example 1 using 7-amino-5-(ethoxymethyl)-2-phenyl-1H-indole-3-carbonitrile obtained in Preparation Example 17 and tetrahydro-4H-pyran-4-one.

¹H NMR (400 MHz, DMSO-d₆); δ 11.94 (bs, 1H), 7.94 (d, 2H), 7.64 (t, 2H), 7.55 (d, 1H), 6.83 (s, 1H), 6.46 (s, 1H), 5.61 (d, 1H), 4.47 (s, 2H), 3.98-3.85 (m, 2H), 3.75-3.60 (m, 1H), 3.60-3.40 (m, 4H) 2.15-1.97 (m, 2H), 1.54-1.38 (m, 2H), 1.15 (t, 3H).

Mass [M + H] = 376 (M +1)

### Example 25: (E)-7-(cyclopentylamino)-5-(ethoxymethyl)-2-phenyl-1H-indole-3-carbaldehyde O-methyl oxime

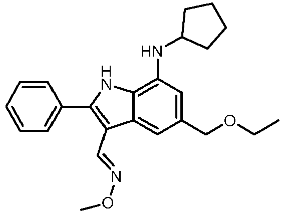

The desired title compound was obtained in the same method as in Example 2 using (E)-7-amino-5-(ethoxymethyl)-2-phenyl-1H-indole-3-carbaldehyde O-methyl-oxime obtained in Preparation Example 18 and cyclopentanone.

¹H NMR (400 MHz, DMSO-d₆); δ 11.34 (bs, 1H), 8.25 (s, 1H), 7.63-7.44 (m, 5H), 7.32 (s, 1H), 6.35 (s, 1H), 5.41 (d, 1H), 4.46 (s, 2H), 3.95-3.85 (m, 1H), 3.88 (s, 3H), 3.46 (q, 2H), 2.10-1.96 (m, 1H), 1.80-1.50 (m, 6H), 1.15 (t, 3H).

Mass [M + H] = 391.8 (M+1)

### Example 26: (E)-7-(bis(tetrahydro-2H-pyran-4-yl)amino)-5-(ethoxymethyl)-2-phenyl-1H-indole-3-carbaldehyde O-methyl Oxime

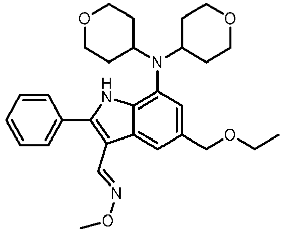

The desired title compound was obtained in the same method as in Example 1 using (E)-7-amino-5-(ethoxymethyl)-2-phenyl-1H-indole-3-carbaldehyde O-methyl-oxime obtained in Preparation Example 18 and 10 equivalents of tetrahydro-4H-pyran-4-one.

¹H NMR (400 MHz, DMSO-d₆); δ 11.48 (bs, 1H), 8.19 (s, 1H), 7.89 (s, 1H), 7.65-7.46 (m, 5H), 7.09 (s, 1H), 4.55 (s, 2H), 3.88 (s, 3H), 3.85-3.75 (m, 4H), 3.55-3.40 (m, 4H), 3.35-3.20 (m, 2H), 1.85-1.70 (m, 4H), 1.40-1.20 (m, 4H), 1.16 (t, 3H).

Mass [M + H] = 491.6 (M +1)

### Example 27: (E)-5-(ethoxymethyl)-2-phenyl-7-(tetrahydro-2H-pyran-4-yl)amino)-1H-indole-3-carbaldehyde O-methyl oxime

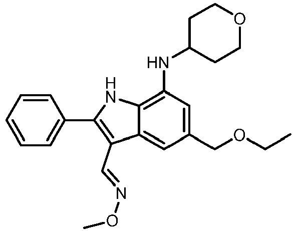

The desired title compound was obtained in the same method as in Example 1 using (E)-7-amino-5-(ethoxymethyl)-2-phenyl-1H-indole-3-carbaldehyde O-methyl-oxime obtained in Preparation Example 18 and 1.0 equivalent of tetrahydro-4H-pyran-4-one.

¹H NMR (400 MHz, DMSO-d₆); δ 11.39 (bs, 1H), 8.26 (s, 1H), 7.65-7.46 (m, 5H), 7.35 (s, 1H), 6.43 (s, 1H), 5.36 (d, 1H), 4.46 (s, 2H), 4.00-3.85 (m, 2H), 3.89 (s, 3H), 3.75-3.60 (m, 1H), 3.60-3.44 (m, 4H), 2.10-1.95 (m, 2H), 1.53-1.40 (m, 2H), 1.15 (t, 3H).

Mass [M + H] = 407.8 (M +1)

### Example 28: N-cyclopentyl-5-(ethoxymethyl)-3-(methoxymethyl)-1-methyl-2-phenyl-1H-indol-7-amine

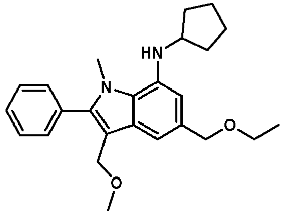

The desired title compound was obtained in the same method as in Example 2 using 5-(ethoxymethyl)-3-(methoxymethyl)-1-methyl-2-phenyl-1H-indol-7-amine obtained in Preparation Example 19 and cyclopentanone.

¹H NMR (400 MHz, DMSO-d₆); δ 7.61-7.44 (m, 5H), 6.97 (s, 1H), 6.45 (s, 1H), 4.90(d, 1H), 4.46 (s, 2H), 4.33 (s, 2H), 3.85-3.75 (m, 1H), 3.82 (s, 3H), 3.46 (q, 2H), 3.18 (s, 3H), 2.05-1.88 (m, 2H), 1.80-1.50 (m, 6H), 1.15 (t, 3H).

Mass [M + H] = 392.8 (M +1)

### Example 29: 5-(ethoxymethyl)-3-(methoxymethyl)-1-methyl-2-phenyl-N-(tetrahydro-2H-pyran-4-yl)-1H-indol-7-amine

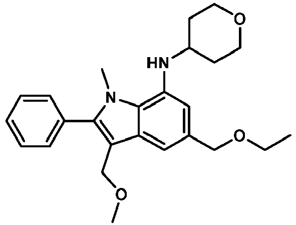

The desired title compound was obtained in the same method as in Example 1 using 5-(ethoxymethyl)-3-(methoxymethyl)-1-methyl-2-phenyl-1H-indol-7-amine obtained in Preparation Example 19 and tetrahydro-4H-pyran-4-one.

¹H NMR (400 MHz, DMSO-d₆); δ 7.60-7.42 (m, 5H), 7.01 (s, 1H), 6.53 (s, 1H), 4.78 (d, 1H), 4.60 (s, 2H), 4.33 (s, 2H), 3.96-3.80 (m, 2H), 3.85 (s, 3H), 3.56-3.40 (m, 6H), 3.19 (s, 3H), 2.02-1.88 (m, 2H), 1.63-1.47 (m, 2H), 1.15 (t, 3H).

Mass [M + H] = 409 (M +1)

### Example 30: methyl (E)-3-(7-(cyclopentylamino)-5-(ethoxymethyl)-2-phenyl-1H-indol-3-yl)acrylate

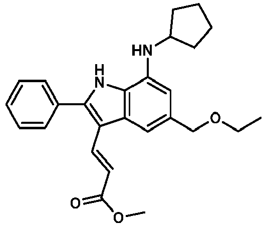

The desired title compound was obtained in the same method as in Example 2 using methyl (E)-3-(7-amino-5-(ethoxymethyl)-2-phenyl-1H-indol-3-yl)-acrylate obtained in Preparation Example 20 and cyclopentanone.

¹H NMR (400 MHz, DMSO-d₆); δ 11.60 (bs, 1H), 7.82 (d, 1H), 7.67-7.50 (m, 5H), 7.10 (s, 1H), 6.41 (s, 1H), 6.38 (d, 1H), 5.48 (d, 1H), 4.51 (s, 2H), 3.96-3.85 (m, 1H), 3.69 (s, 3H), 3.48 (q, 2H), 2.13-1.98 (m, 2H), 1.85-1.50 (m, 6H), 1.16 (t, 3H).

Mass [M + H] = 419 (M+1)

### Example 31: Methyl (E)-3-(5-(ethoxymethyl)-2-phenyl-7-((tetrahydro-2H-pyran-4-yl)amino)-1H-indole-3-yl)acrylate

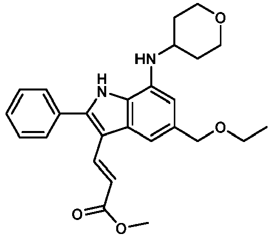

The desired title compound was obtained in the same method as in Example 1 using methyl (E)-3-(7-amino-5-(ethoxymethyl)-2-phenyl-1H-indol-3-yl)-acrylate obtained in Preparation Example 20 and tetrahydro-4H-pyran-4-one.

¹H NMR (400 MHz, DMSO-d₆); δ 11.64 (bs, 1H), 7.81 (d, 1H), 7.69-7.50 (m, 5H), 7.11 (s, 1H), 6.47 (s, 1H), 6.39 (d, 1H), 5.42 (d, 1H), 4.50 (s, 2H), 3.96-3.85 (m, 2H), 3.75-3.63 (m, 1H), 3.68 (s, 3H), 3.56-3.42 (m, 4H), 2.10-1.97 (m, 2H), 1.52-1.35 (m, 2H), 1.15 (t, 3H).

Mass [M + H] = 435 (M +1)

### Example 32: 1-(7-(cyclopentylamino)-5-(ethoxymethyl)-2-phenyl-1H-indol-3-yl)-2,2,2-trifluoroethan-1-one

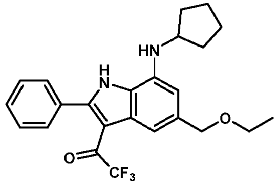

The desired title compound was obtained in the same method as in Example 2 using 1-(7-amino-5-(ethoxymethyl)-2-phenyl-1H-indol-3-yl)-2,2,2-trifluoro-ethan-1-one obtained in Preparation Example 21 and cyclopentanone.

¹H NMR (400 MHz, DMSO-d₆); δ 12.27 (bs, 1H), 7.69-7.48 (m, 5H), 7.28 (s, 1H), 6.44 (s, 1H), 5.54 (d, 1H), 4.50 (s, 2H), 3.96-3.83 (m, 1H), 3.48 (q, 2H), 2.08-1.94 (m, 2H), 1.81-1.45 (m, 6H), 1.16 (t, 3H).

Mass [M + H] =431.2 (M +1)

### Example 33: 1-(5-(ethoxymethyl)-2-phenyl-7-((tetrahydro-2H-pyran-4-yl)amino)-1H-indol-3-yl)-2,2, 2-trifluoro-1-one

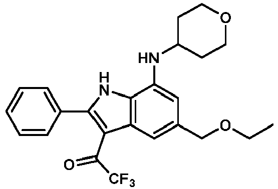

The desired title compound was obtained in the same method as in Example 1 using 1-(7-amino-5-(ethoxymethyl)-2-phenyl-1H-indol-3-yl)-2,2,2-trifluoro-ethan-1-one obtained in Preparation Example 21 and tetrahydro-4H-pyran-4-one.

¹H NMR (400 MHz, DMSO-d₆); δ 12.29 (bs, 1H), 7.69 -7.50 (m, 5H), 7.29 (s, 1H), 6.51 (s, 1H), 5.48 (d, 1H), 4.48 (s, 2H), 3.95-3.83 (m, 2H), 3.74-3.62 (m, 1H), 3.56-3.40 (m, 4H), 2.06-1.95 (m, 2H), 1.52-1.34 (m, 2H), 1.15 (t, 3H).

Mass [M + H] = 447.1 (M +1)

### Example 34: (E)-3-(7-(cyclopentylamino)-5-(ethoxymethyl)-2-phenyl-1H-indol-3-yl)-1-phenylpropan-2-ene-1-one

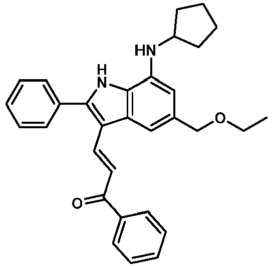

The desired title compound was obtained in the same method as in Example 2 using (E)-3-(7-amino-5-(ethoxymethyl)-2-phenyl-1H-indol-3-yl)-1-phenylprop-2-en-1-one and cyclopentanone.

¹H NMR (400 MHz, DMSO-d₆); δ 11.77 (bs, 1H), 8.08-7.98 (m, 3H), 7.70-7.52 (m, 9H), 7.30 (s, 1H), 6.44 (s, 1H), 5.53 (d, 2H), 4.58 (s, 2H), 4.00-3.90 (m, 1H), 3.52 (q, 2H), 2.10-1.95 (m, 2H), 1.81-1.50 (m, 6H), 1.19 (t, 3H).

Mass [M + H] = 464.9 (M +1)

### Example 35: (E)-3-(5-(ethoxymethyl)-2-phenyl-7-((tetrahydro-2H-pyran-4-yl)amino)-1H-indol-3-yl)-1-phenylpropan-2-en-1-one

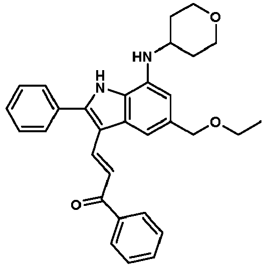

The desired title compound was obtained in the same method as in Example 1 using (E)-3-(7-amino-5-(ethoxymethyl)-2-phenyl-1H-indol-3-yl)-1-phenylprop-2-en-1-one obtained in Preparation Example 22 and tetrahydro-4H-pyran-4-one.

¹H NMR (400 MHz, DMSO-d₆); δ 11.80 (bs, 1H), 8.06-7.95 (m, 3H), 7.70-7.52 (m, 9H), 7.32 (s, 1H), 6.51 (s, 1H), 5.47 (d, 2H), 4.58 (s, 2H), 3.98-3.90 (m, 2H), 3.75-3.62 (m, 1H), 3.56-3.45 (m, 4H), 2.10-1.98 (m, 2H), 1.52-1.40 (m, 2H), 1.18 (t, 3H).

Mass [M + H] = 481.3 (M+1)

### Example 36: 1-(7-(cyclopentylamino)-5-(ethoxymethyl)-2-phenyl-1H-indol-3-yl)-2,2,2-trifluoroethan-1-ol

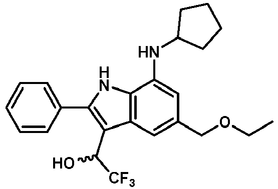

1-(7-(cyclopentylamino)-5-(ethoxymethyl)-2-phenyl-1H-indol-3-yl)-2,2,2-trifluoroethane-1-one (85 mg, 0.20 mmol) obtained in Example 32 was dissolved in THF (1 mL), and NaBH₄ (15 mg, 0.40 mmol) was slowly added at 4°C. The mixture was stirred under the same conditions for 30 minutes, and then BF₃·Et₂O diethyl etherate (73 µL, 0.60 mmol) was added dropwise thereto. After stirring at room temperature for 1 hour, ice water was added thereto, and the mixture was extracted with EA. The organic layer was washed with a saturated NaHCOa solution, dried over MgSO₄, and the filtered filtrate was distilled under reduced pressure. The concentrated residue was purified by column chromatography to obtain 1-(7-cyclopentylamino-5-ethoxymethyl-2-phenyl-1H-indol-3-yl)-2,2,2-trifluoroethanol (58 mg, yield 68%).

¹H NMR (400 MHz, DMSO-d₆); δ 11.07 (bs, 1H), 7.63-7.44 (m, 5H), 7.01 (s, 1H), 6.47 (d, 1H), 6.27 (s, 1H), 5.35-5.25 (m, 1H), 5.25-5.13 (m, 1H), 4.23 (s, 2H), 3.95-3.81 (m, 1H), 3.45 (q, 2H), 2.08-1.94 (m, 2H), 1.80-1.45 (m, 6H), 1.14 (t, 3H).

Mass [M + H] = 433.4 (M +1)

### Example 37: 1-(5-(ethoxymethyl)-2-phenyl-7-((tetrahydro-2H-pyran-4-yl)amino)-1H-indol-3-yl)-2,2, 2-trifluoro-1-ol

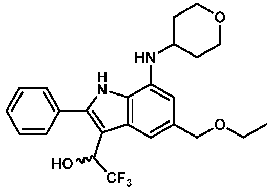

From 1-(5-(ethoxymethyl)-2-phenyl-7-((tetrahydro-2H-pyran-4-yl)amino)-1H-indol-3-yl)-2,2,2-trifluoro-1-one (130 mg, 0.29 mmol) obtained in Example 33, the desired title compound (91 mg, yield 69%) was obtained using the same method as in Example 36.

¹H NMR (400 MHz, DMSO-d₆); δ 11.11 (bs, 1H), 7.65-7.44 (m, 5H), 7.04 (s, 1H), 6.49 (d, 1H), 6.34 (s, 1H), 5.26 (d, 1H), 5.25-5.13 (m, 1H), 4.23 (s, 2H), 3.98-3.88 (m, 2H), 3.75-3.60 (m, 1H), 3.60-3.40 (m, 4H), 2.10-1.98 (m, 2H), 1.52-1.37 (m, 2H), 1.14 (t, 3H).

Mass [M + H] = 449 (M +1)

### Example 38: Methyl 3-(7-(cyclopentylamino)-5-(ethoxymethyl)-2-phenyl-1H-indol-3-yl)propanoate

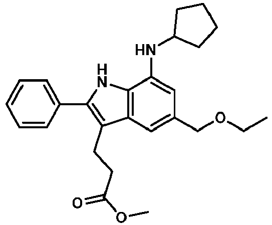

Methyl (E)-3-(7-(cyclopentylamino)-5-(ethoxymethyl)-2-phenyl-1H-indol-3-yl)acrylate (168 mg, 0.40 mmol) obtained in Example 30 was dissolved in THF (4 mL), 10% palladium/carbon (water 51 wt%, 81 mg) was added thereto, and the mixture was stirred under hydrogen overnight. The reaction mixture was filtered through Celite, and the filtrate was distilled under reduced pressure. The concentrated residue was purified by column chromatography to obtain methyl 3-(7-cyclopentylamino)-5-(ethoxymethyl)-2-phenyl-1H-indol-3-yl)-propanoate (109 mg, yield 64%).

¹H NMR (400 MHz, DMSO-d₆); δ 10.69 (bs, 1H), 7.61 (d, 2H), 7.53 (t, 2H), 7.39 (t, 2H), 6.72 (s, 1H), 6.25 (s, 1H), 5.35 (d, 1H), 4.44 (s, 2H), 3.95-3.85 (m, 1H), 3.54 (s, 3H), 3.46 (q, 2H), 3.08 (t, 2H), 2.61 (t, 2H), 2.08-1.95 (m, 2H), 1.80-1.50 (m, 6H), 1.14 (t, 3H).

Mass [M + Na] = 443.1 (M + 23)

### Example 39: Methyl 3-(5-(ethoxymethyl)-2-phenyl-7-((tetrahydro-2H-pyran-4-yl)amino)-1H-indol-3-yl)propanoate

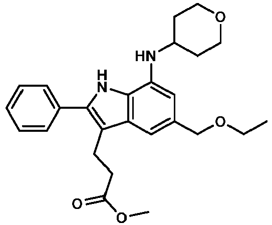

From methyl (E)-3-(5-(ethoxymethyl)-2phenyl-7-((tetrahydro-2H-pyran-4-yl)amino)-1H-indol-3-yl)acrylate (192 mg, 0.44 mmol) obtained in Example 31, the desired title compound (126 mg, yield 65%) was obtained using the same method as in Example 38.

¹H NMR (400 MHz, DMSO-d₆); δ 10.72 (bs, 1H), 7.61 (d, 2H), 7.54 (t, 2H), 7.40 (t, 2H), 6.77 (s, 1H), 6.31 (s, 1H), 5.29 (d, 1H), 4.44 (s, 2H), 3.98-3.90 (m, 2H), 3.73-3.60 (m, 1H), 3.60-3.44 (m, 4H), 3.55 (s, 3H), 3.06 (t, 2H), 2.62 (t, 2H), 2.08-1.98 (m, 2H), 1.52-1.38 (m, 2H), 1.14 (t, 3H).

Mass [M + Na] = 459.1 (M + 23)

### Example 40: 3-(7-(cyclopentylamino)-5-(ethoxymethyl)-2-phenyl-1H-indol-3-yl)propanoic acid

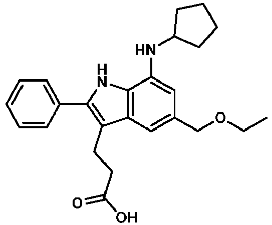

Methyl 3-(7-(cyclopentylamino)-5-(ethoxymethyl)-2-phenyl-1H-indol-3-yl)propanoate (89 mg, 0.21 mmol) obtained in Example 38, and lithium hydroxide (8 mg, 0.32 mmol) were dissolved in a 1:2:5 mixed solution (1 mL) of methanol, water and THF, and the mixture was stirred overnight. Water was added to the reaction mixture, which was adjusted to pH 2~3 with a 1N hydrochloric acid solution, and extracted with EA. The organic layer was washed with water, dried over MgSO₄, and then the filtered filtrate was distilled under reduced pressure. 3-(7-cyclopentylamino-5-ethoxymethyl-2-phenyl-1H-indol-3-yl)-propionic acid (86 mg, yield 100%) was obtained by concentration.

¹H NMR (400 MHz, DMSO-d₆); δ 12.10 (bs, 1H), 10.67 (s, 1H), 7.62 (d, 2H), 7.53 (t, 2H), 7.39 (t, 2H), 6.77 (s, 1H), 6.25 (s, 1H), 5.35 (bs, 1H), 4.44 (s, 2H), 3.96-3.85 (m, 1H), 3.44 (q, 2H), 3.03 (t, 2H), 2.58-2.50 (m, 2H), 2.08-1.95 (m, 2H), 1.83-1.48 (m, 6H), 1.14 (t, 3H)

ESI-MS (*m*/*z*): 407 [M+H]⁺

### Example 41: 5-(2-methoxyethyl)-2-phenyl-N-(tetrahydro-2H-pyran-4-yl)-1H-indol-7-amine

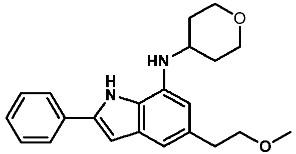

From 5-(2-methoxyethyl)-2-phenyl-1H-indol-7-amine (50 mg, 0.188 mmol) obtained in Preparation Example 26 and tetrahydro-4H-pyran-4H-one (56 mg, 0.564) mmol), the desired title compound (53 mg, yield 84%) was obtained in the same method as in Example 1.

¹H NMR (400 MHz, CDCl₃); δ 8.09 (bs, 1H), 7.70 (d, 2H), 7.46 (t, 2H), 7.30 (t, 1H), 7.01 (s, 1H), 6.77 (s, 1H), 6.46 (s, 1H), 4.10-4.07 (m, 2H), 3.65-3.69 (m, 3H), 3.59 (t, 2H), 2.97 (t, 2H), 3.17-2.14 (m, 2H), 1.67-1.63 (m, 2H).

ESI-MS (*m*/*z*): 351 [M+H]⁺

### Example 42: N-cyclopentyl-5-(2-methoxyethyl)-2-phenyl-1H-indol-7-amine

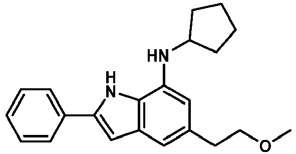

From 5-(2-methoxyethyl)-2-phenyl-1H-indol-7-amine (50 mg, 0.188 mmol) obtained in Preparation Example 26 and cyclopentanone (33 µg, 0.376 mmol), the desired title compound (33 mg, yield 55%) was obtained in the same method as in Example 2.

¹H NMR (400 MHz, CDCl₃); δ 8.06 (bs, 1H), 7.70 (d, 2H), 7.45 (t, 2H), 7.31 (t, 1H), 6.98 (s, 1H), 6.76 (s, 1H), 6.45 (s, 1H), 4.03-3.98 (m, 1H), 3.68 (t, 2H), 3.42 (s, 3H), 2.98 (t, 2H), 2.16-2.10 (m, 2H), 1.90-1.72 (m, 2H), 1.70-1.62 (m, 4H).

ESI-MS (*m*/*z*): 335 [M+H]⁺

### Example 43: Ethyl 2-(7-(cyclopentylamino)-5-(ethoxymethyl)-2-phenyl-1H-indol-3-yl)-1H-imidazole-1-carboxylate

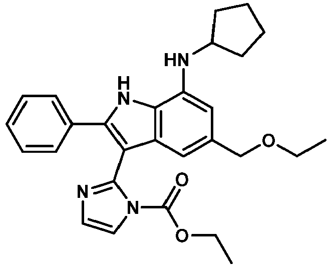

From 25 mg (0.06 mmol) of ethyl 2-(7-amino-5-(ethoxymethyl)-2-phenyl-1H-indol-3-yl)-1H-imidazole-1-carboxylate obtained in Preparation Example 23 and cyclopentanone (8 mg), the desired title compound (17 mg, yield 58%) was obtained in the same method as in Example 2.

¹H NMR (400 MHz, DMSO-d₆); δ 11.18 (bs, 1H), 7.66 (d, 1H), 7.45-7.29 (m, 5H), 7.18 (d, 1H), 6.53 (s, 1H), 6.28 (s, 1H), 5.50 (d, 1H), 4.39 (s, 2H), 3.98-3.88 (m, 3H), 3.42 (q, 2H), 2.15-1.97 (m, 2H), 1.85-1.53 (m, 6H), 1.11 (t, 3H), 0.88 (t, 3H).

Mass [M + H] = 472.8 (M+1)

### Example 44: Ethyl2-(5-(ethoxymethyl)-2-phenyl-7-((tetrahydro-2H-pyran-4-yl)amino)-1H-indol-3-yl)-1H-imi Dazole-1-carboxylate

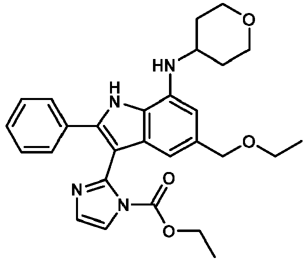

The desired title compound was obtained in the same method as in Example 1 using ethyl 2-(7-amino-5-(ethoxymethyl)-2-phenyl-1H-indol-3-yl)-1H-imidazole-1-carboxylate obtained in Preparation Example 23 and tetrahydro-4H-pyran-4H-one.

¹H NMR (400 MHz, DMSO-d₆); δ 11.21 (s, 1H), 7.67 (d, 1H), 7.45-7.29 (m, 5H), 7.18 (d, 1H), 6.54 (s, 1H), 6.35 (s, 1H), 5.44 (d, 1H), 4.38 (s, 2H), 3.98-3.87 (m, 4H), 3.75-3.63 (m, 1H), 3.51 (t, 2H), 3.41 (q, 2H), 2.15-2.03 (m, 2H), 1.56-1.42 (m, 2H), 1.11 (t, 3H), 0.87 (t, 3H).

Mass [M + H] = 488.8 (M+1)

### Example 45: N-cyclopentyl-5-(ethoxymethyl)-3-(1H-imidazol-2-yl)-2-phenyl-1H-indol-7-amine

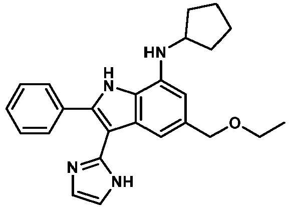

Ethyl 2-(7-(cyclopentylamino)-5-(ethoxymethyl)-2-phenyl-1H-indol-3-yl)-1H-imidazole-1-carboxylate (12 mg, 0.025 mmol) obtained in Example 43 was dissolved in ethanol (3.3 mL), 3M NaOH (85 µL) was added thereto, and the mixture was stirred at room temperature for 3 hours. After neutralization with a 1N hydrochloric acid solution, water was added, and the mixture was extracted with EA. The organic layer was washed with water, dried over MgSO₄, and the filtered filtrate was distilled under reduced pressure. The desired title compound (7 mg, yield 70%) was obtained by concentration.

¹H NMR (400 MHz, DMSO-d₆); δ 11.75 (bs, 1H), 11.07 (s, 1H), 7.60 (d, 2H), 7.45-7.29 (m, 5H), 7.19-6.98 (m, 2H), 6.78 (s, 1H), 6.30 (s, 1H), 5.50 (d, 1H), 4.42 (s, 2H), 3.98-3.87 (m, 1H), 3.44 (q, 2H), 2.15-1.97 (m, 2H), 1.83-1.52 (m, 6H), 1.13 (t, 3H).

Mass [M + H] = 400.8 (M+1)

### Example 46: 5-(ethoxymethyl)-3-(1H-imidazol-2-yl)-2-phenyl-N-(tetrahydro-2H-pyran-4-yl)-1H-indole-7-amine

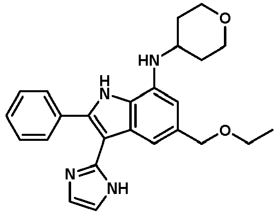

From ethyl 2-(5-(ethoxymethyl)-2-phenyl-7-((tetrahydro-2H-pyran-4-yl)amino)-1H-indol-3-yl)-1H-imidazole-1-carboxylate (17 mg) obtained in Example 44, the desired title compound (12 mg, yield 83%) was obtained in the same method as in Example 45.

¹H NMR (400 MHz, DMSO-d₆); δ 11.81 (bs, 1H), 11.11 (s, 1H), 7.60 (d, 2H), 7.43 (t, 2H), 7.38-7.29 (m, 1H), 7.10 (bs, 2H), 6.80 (s, 1H), 6.37 (s, 1H), 5.43 (d, 1H), 4.42 (s, 2H), 4.01-3.90 (m, 2H), 3.76-3.63 (m, 1H), 3.52 (t, 2H), 3.44 (q, 2H), 2.13-2.03 (m, 2H), 1.56-1.40 (m, 2H), 1.12 (t, 3H).

Mass [M + H] = 416.8 (M+1)

### Example 47: 2-phenyl-5-(propoxymethyl)-N-(tetrahydro-2H-pyran-4-yl)-1H-indol-7-amine

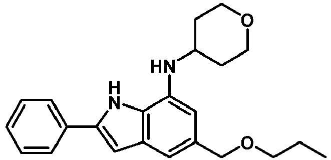

The desired title compound (12 mg, yield 83%) was obtained in the same method as in Example 1 using 2-phenyl-5-(propoxymethyl)-1H-indol-7-amine obtained in Preparation Example 24 and tetrahydro-4H-pyran-4-one.

¹H NMR (400 MHz, DMSO-d₆); δ 10.94 (bs, 1H), 7.81 (d, 2H), 7.48 (t, 2H), 7.31 (t, 1H), 6.77 (s, 2H), 6.31 (s, 1H), 5.36 (d, 1H), 4.42 (s, 2H), 4.00-3.90 (m, 2H), 3.71-3.60 (m, 1H), 3.50 (t, 2H), 3.40-3.30 (m, 2H), 2.13-2.02 (m, 2H), 1.61-1.40 (m, 4H), 0.88 (t, 3H).

Mass [M + H] = 365

### Example 48: 5-((cyclopropylmethoxy)methyl)-2-phenyl-N-(tetrahydro-2H-pyran-4-yl)-1H-indol-7-amine

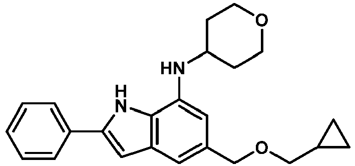

The desired title compound was obtained in the same method as in Example 1 using 5-((cyclopropylmethoxy)methyl)-2-phenyl-1H-indol-7-amine obtained in Preparation Example 25 and tetrahydro-4H-pyran-4-one.

¹H NMR (400 MHz, DMSO-d₆); δ 10.93 (bs, 1H), 7.81 (d, 2H), 7.47 (t, 2H), 7.35-7.26 (m, 1H), 6.76 (s, 2H), 6.30 (s, 1H), 5.35 (d, 1H), 4.44 (s, 2H), 4.00-3.90 (m, 2H), 3.70-3.58 (m, 1H), 3.50 (t, 2H), 3.23 (d, 1H), 2.10-2.00 (m, 2H), 1.54-1.38 (m, 2H), 1.08-0.97 (m, 1H), 0.50-0.40 (m,2H), 0.19-0.09 (m, 2H).

Mass [M + H] = 377.1

### Example 49: 5-(ethoxymethyl)-2-phenyl-N-((tetrahydro-2H-pyran-4-yl)methyl)-1H-indol-7-amine

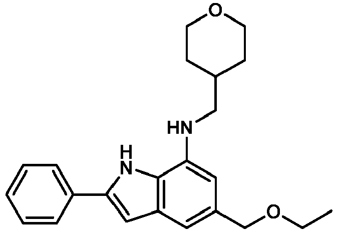

The desired title compound was obtained in the same method as in Example 3 using 5-(ethoxymethyl)-2-phenyl-1H-indol-7-amine obtained in Preparation Example 14 and tetrahydropyran-4-carbaldehyde.

¹H NMR (400 MHz, DMSO-d₆); δ 10.96 (bs, 1H), 7.81 (d, 2H), 7.48 (t, 2H), 7.60-7.25 (m, 1H), 6.77 (s, 2H), 6.22 (s, 1H), 5.45 (bs, 1H), 4.20 (s, 2H), 3.97-3.86 (m, 2H), 3.52-3.31 (m, 5H), 3.12 (bs, 2H), 2.00-1.73 (m, 3H), 1.40-1.25 (m, 2H), 1.15 (t, 3H).

Mass [M + H] = 364.7

### Example 50: 5-(3-methoxypropyl)-2-phenyl-N-(tetrahydro-2H-pyran-4-yl)-1H-indol-7-amine

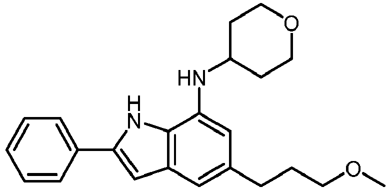

From 5-(3-methoxypropyl)-2-phenyl-1H-indol-7-amine (75 mg, 0.267 mmol) obtained in Preparation Example 27 and tetrahydro-4H-pyran-4H-one (138 mg, 1.38 mmol), the desired title compound (17 mg, yield 17%) was obtained in the same method as in Example 1.

¹H NMR (400 MHz, CDCl₃); δ 8.60 (br, 1H), 7.73 (d, 2H), 7.46 (t, 2H), 7.32 (t, 1H), 7.04 (s, 1H), 6.76 (s, 1H), 6.52 (s, 1H), 4.05 (d, 2H), 3.69 (m, 1H), 3.53 (t, 2H), 3.44 (t, 2H), 3.38 (s, 3H), 2.74 (t, 2H), 2.15 (m, 2H), 1.95 (m, 2H), 1.70 (m, 2H).

Mass [M + H] = 365.33

### Example 51: 5-(3-ethoxypropyl)-2-phenyl-N-(tetrahydro-2H-pyran-4-yl)-1H-indol-7-amine

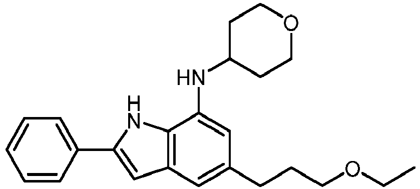

In Step 5 of Preparation Example 27, EtI (ethyl iodide) was used instead of MeI, and the desired title compound was obtained in the same method as in Example 50.

¹H NMR (400 MHz, CDCl₃); δ 8.67 (br, 1H), 7.71 (d, 2H), 7.43 (t, 2H), 7.31 (t, 1H), 7.04 (s, 1H), 6.74 (s, 1H), 6.53 (s, 1H), 4.04 (d, 2H), 3.65 (m, 1H), 3.53 - 3.40 (m, 6H), 2.72 (t, 2H), 2.15 (m, 2H), 1.93 (m, 2H), 1.70 (m, 2H), 1.23 (t, 3H).

### Experimental Example 1: Protective effect with regard to heart cells (H9C2, white mouse cardiomyocytes)

In order to identify the protective effect with regard to heart cells, H9C2 cells were divided into 96-well plates by 1.5 x 10⁴, and cultured for 24 hours. The respective wells were treated with compounds of the examples 3x serial diluted to final concentrations of 30, 10, 3, 1, 0.3, 0.1, 0.03 and 0.01 µM, then cultured for 15 to 20 minutes. After treating with t-BHP with a final concentration of 400µM, culturing was performed for 2 hours. In order to identify the protective effect of each compound, a Cytotoxicity LDH assay kit [Dojindo; CK12] was used to measure the degree of extracellular secretion of LDH. To describe briefly, at the time point 2-hour treatment of the cells with t-BHP was completed, 100µL each of the supernatant in which the cells were placed was collected and moved to a new 96-well plate. 100µL/well of the assay buffer included in the kit (same amount as the sample) was added, followed by wrapping with foil and reacting for 30 minutes at room temperature. Observing the change in color of the sample, 50µL/well of stop solution was added, then an iD3 spectrometer was used to measure absorbance at 490nm and calculate the EC₅₀ values. The EC₅₀ of the compounds of the examples is preferably no more than 1.0 µM, and more preferably no more than 0.3 µM.

Table 1 shows the protective effect of the example compounds against the necrosis-inducing substance t-BHP.

**Table 1: Cell Protection Effect against t-BHP in Heart Cells**

| **[Table 1]** | | | | | |
|---|---|---|---|---|---|
| Example | EC₅₀ (µM) | Example | EC₅₀ (µM) | Example | EC₅₀ (µM) |
| 1 | A | 18 | A | 35 | A |
| 2 | A | 19 | A | 36 | A |
| 3 | A | 20 | A | 37 | B |
| 4 | A | 21 | A | 38 | A |
| 5 | A | 22 | A | 39 | A |
| 6 | A | 23 | A | 40 | C |
| 7 | A | 24 | B | 41 | A |
| 8 | A | 25 | A | 42 | A |
| 9 | A | 26 | C | 43 | B |
| 10 | A | 27 | A | 44 | C |
| 11 | A | 28 | B | 45 | B |
| 12 | A | 29 | B | 46 | C |
| 13 | A | 30 | A | 47 | A |
| 14 | A | 31 | A | 48 | A |
| 15 | A | 32 | A | 49 | A |
| 16 | A | 33 | B | 50 | A |
| 17 | A | 34 | A | 51 | A |
| A indicates EC₅₀ ≤ 0.3 µM, B indicates 0.3 µM < EC₅₀ ≤ 1uM, C indicates EC₅₀ > 1.0 µM | | | | | |

### Experimental Example 2: The Maintenance Effect on Intracellular Calcium Level in Heart Cells (H9C2, white mouse cardiomyocytes) treated with t-BHP

To examine the maintenance effect on calcium level in heart cells, H9C2 cells were divided into a 96-well plate by 1.5 x 10⁴ cells/well and cultured for 24 hours. To measure intracellular calcium level, a FLIPR Calcium 6 assay kit (Molecular devices; #R8190) was used. Following the experimental method provided by the manufacturer, cells were treated with probenecid and calcium-sensitive dye for 1.75 hours, and then the cells were treated with the compound of Example 2 for 0.25 hours (the final concentration of the compound was 0.02 or 0.01 µM). And the cells were treated with t-BHP to a final concentration of 150 µM, then intracellular calcium level was measured every 30 seconds in real time.

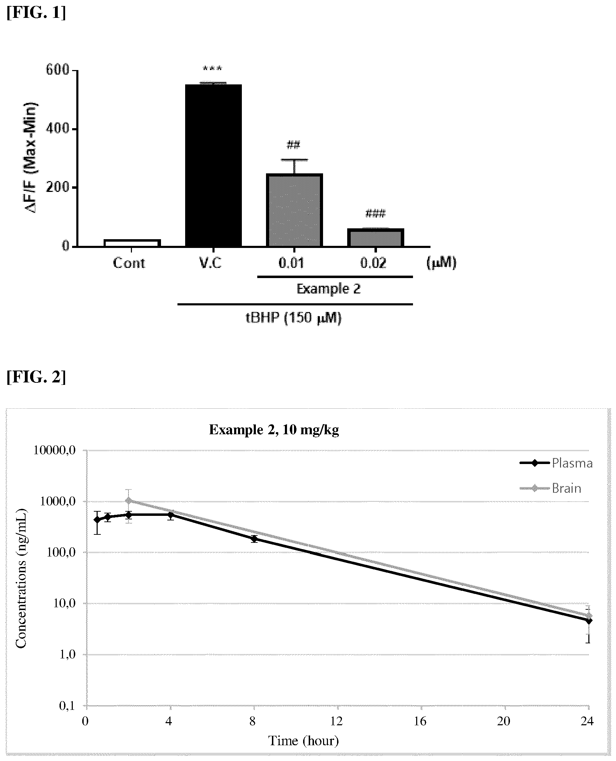

FIG. 1 is a graph regarding the regulatory effect of the compound of Example 2 on calcium level under t-BHP treatment. The ΔF/F(Max-Min) value on the vertical axis is the difference between the maximum and minimum fluorescence intensity values of the calcium-sensitive dye after treatment with t-BHP (150 µM), and this value increases as intracellular calcium level increases. The horizontal axis represents the concentration of the compound of Example 2, and vehicle (V.C.) represents a control group with treatment of DMSO (the same volume of the DMSO in which the compound was diluted). t-BHP increased the intracellular calcium level in the V.C group, whereas the combination of compound of Example 2 (0.01 µM) and t-BHP showed significantly decreased the intracellular calcium level. Moreover, the intracellular calcium level exhibited within normal range under 0.02 µM treatment of the compound of Example 2. Accordingly, it was confirmed that treating with the compound of Example 2 effectively reduced the abnormal increased in intracellular calcium level due to t-BHP treatment.

### Experimental Example 3: Cell death protective effect on fibroblasts (NIH/3T3, mouse embryonic fibroblasts)

In order to identify the protective effect on fibroblasts, NIH/3T3 cells were divided into 96-well plates by 8 × 10³ and cultured for 18 to 24 hours. The compounds of Examples were diluted to final concentrations of 0.1 and 1 µM, treated in each well, and incubated for 15 to 20 minutes. Erastin, glutamate, and RSL3 were treated to have final concentrations of 3 µM, 30 mM, and 3 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, glutamate, and RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 2 showed cell death reduction rates according to the respective concentrations of the compounds of Examples for Erastin, glutamate, and RSL3 as ferroptosis-inducing substances.

**Table 2: Cell death protective effect in fibroblasts (NIH/3T3) against Erastin, glutamate and RSL3**

| [Table 2] | | | | | | |
|---|---|---|---|---|---|---|
| | Erastin, 3 µM | | Glutamate, 30 mM | | RSL3, 3 µM | |
| Example | 0.1 µM | 1.0 µM | 0.1 µM | 1.0 µM | 0.1 µM | 1.0 µM |
| | inhibition (%) | | | | | |
| Ferrostatin-1 | 0 | 96 | 98 | 98 | 100 | 100 |
| 1 | 99 | 99 | 98 | 97 | 98 | 99 |
| 2 | 99 | 100 | 98 | 99 | 100 | 100 |
| 14 | 38 | 100 | 97 | 96 | 100 | 100 |
| 19 | 100 | 100 | 95 | 97 | 100 | 100 |
| 29 | 0 | 99 | 98 | 97 | 100 | 100 |
| 41 | 0 | 0 | 99 | 98 | 100 | 100 |
| 46 | 102 | 99 | 0 | 96 | 0 | 100 |

### Experimental Example 4: Cell death protective effect on adrenal cells (PC-12, white mouse adrenal cells)

In order to identify the protective effect on adrenal cells, PC-12 cells were divided into 96-well plates by 1 × 10⁴ and cultured for 18 to 24 hours. The compounds of Examples were diluted to final concentrations of 0.1 and 1.0 µM in Erastin, 0.01 and 0.1 µM in glutamate, and 0.3 and 3.0 µM in RSL3, treated in each well, and incubated for 15 to 20 minutes. Erastin, glutamate, and RSL3 were treated to have final concentrations of 1 µM, 10 mM, and 0.3 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, glutamate, and RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 3 showed cell death reduction rates according to the respective concentrations of the compounds of Examples for Erastin, glutamate, and RSL3 as ferroptosis-inducing substances.

**Table 3: Cell death protective effect in adrenal cells (PC-12) against Erastin, glutamate, and RSL3**

| [Table 3] | | | | | | |
|---|---|---|---|---|---|---|
| | Erastin, 1 µM | | Glutamate, 10 mM | | RSL3, 0.3 µM | |
| Example | 0.1 µM | 1.0 µM | 0.01 µM | 0.1 µM | 0.1 µM | 1.0 µM |
| | inhibition (%) | | | | | |
| Ferrostatin-1 | 100 | 100 | 29 | 99 | 100 | 100 |
| 1 | 100 | 100 | 77 | 100 | 100 | 100 |
| 2 | 100 | 100 | 96 | 100 | 100 | 100 |
| 14 | 100 | 100 | 45 | 100 | 100 | 100 |
| 19 | 100 | 100 | 100 | 100 | 100 | 100 |
| 29 | 0 | 100 | 22 | 71 | 26 | 100 |
| 41 | 100 | 100 | 76 | 100 | 100 | 100 |
| 46 | 0 | 38 | 4 | 6 | 0 | 100 |

### Experimental Example 5: Cell death protective effect on cardiac cells (H9C2, white mouse cardiac myocytes)

In order to identify the protective effect on cardiac cells, H9C2 cells were divided into 96-well plates by 1 × 10⁴ and cultured for 18 to 24 hours. The compounds of Examples were diluted to final concentrations of 0.1 and 1.0 µM, treated in each well, and incubated for 15 to 20 minutes. Erastin, glutamate, and RSL3 were treated to have final concentrations of 1 µM, 25 mM, and 0.5 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, glutamate, and RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 4 showed cell death reduction rates according to the respective concentrations of the compounds of Examples for Erastin, glutamate, and RSL3 as ferroptosis-inducing substances.

**Table 4: Cell death protective effect in cardiac myocytes (H9C2) against Erastin, glutamate and RSL3**

| [Table 4] | | | | | | |
|---|---|---|---|---|---|---|
| | Erastin, 1 µM | | Glutamate, 25 mM | | RSL3, 0.5 µM | |
| Example | 0.1 µM | 1.0 µM | 0.1 µM | 1.0 µM | 0.1 µM | 1.0 µM |
| | inhibition (%) | | | | | |
| Ferro statin-1 | 100 | 100 | 95 | 97 | 83 | 100 |
| 1 | 100 | 100 | 97 | 98 | 100 | 100 |
| 2 | 100 | 100 | 97 | 98 | 100 | 100 |
| 14 | 100 | 100 | 97 | 99 | 100 | 100 |
| 19 | 100 | 100 | 99 | 100 | 100 | 100 |
| 29 | 1 | 100 | 6 | 95 | 10 | 100 |
| 41 | 100 | 100 | 99 | 98 | 100 | 100 |
| 46 | 4 | 100 | 4 | 94 | 15 | 9 |

### Experimental Example 6: Cell death protective effect on renal fibroblasts (NRK-49F, rat renal fibroblasts)

In order to identify the protective effect on renal fibroblasts, NRK-49F cells were divided into 96-well plates by 1 × 10⁴ and cultured for 18 to 24 hours. The compounds of Examples were diluted to final concentrations of 0.1 and 1.0 µM, treated in each well, and incubated for 15 to 20 minutes. Erastin, glutamate, and RSL3 were treated to have final concentrations of 3 µM, 75 mM, and 3 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, glutamate, and RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 5 showed cell death reduction rates according to the respective concentrations of the compounds of Examples for Erastin, glutamate, and RSL3 as ferroptosis-inducing substances.

**Table 5: Cell death protective effect in renal fibroblasts (NRK-49F) against Erastin, glutamate and RSL3**

| [Table 5] | | | | | | |
|---|---|---|---|---|---|---|
| | Erastin, 3 µM | | Glutamate, 75 mM | | RSL3, 3 µM | |
| Example | 0.1 µM | 1.0 µM | 0.1 µM | 1.0 µM | 0.1 µM | 1.0 µM |
| | inhibition (%) | | | | | |
| Ferro statin-1 | 98 | 97 | 90 | 100 | 97 | 97 |
| 1 | 96 | 95 | 100 | 100 | 98 | 98 |
| 2 | 97 | 94 | 100 | 100 | 98 | 97 |
| 14 | 96 | 93 | 100 | 100 | 97 | 97 |
| 19 | 96 | 95 | 100 | 100 | 97 | 96 |
| 29 | 3 | 93 | 17 | 100 | 0 | 96 |
| 41 | 96 | 94 | 100 | 100 | 97 | 97 |
| 46 | 5 | 64 | 13 | 16 | 0 | 5 |

### Experimental Example 7: Cell death protective effect on retinal epithelial cells (ARPE-19, human retinal epithelial cells)

In order to identify the protective effect on retinal epithelial cells, ARPE-19 cells were divided into 96-well plates by 1 × 10⁴ and cultured for 18 to 24 hours. The compounds of Examples were diluted to final concentrations of 0.03 and 0.3 µM, treated in each well, and incubated for 20 minutes. Erastin, and RSL3 were treated to have final concentrations of 10 µM, and 0.3 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, and RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 6 showed cell death reduction rates according to the respective concentrations of the compounds of Examples for Erastin, and RSL3 as ferroptosis-inducing substances.

**Table 6: Cell death protective effect in retinal epithelial cells (ARPE-19) against Erastin and RSL3**

| [Table 6] | | | | |
|---|---|---|---|---|
| | Erastin, 10 µM | | RSL3, 0.3 µM | |
| Example | 0.03 µM | 0.3 µM | 0.03 µM | 0.03 µM |
| | inhibition (%) | | | |
| Ferrostatin-1 | 96 | 100 | 68 | 100 |
| 1 | 100 | 100 | 100 | 100 |
| 2 | 100 | 100 | 100 | 100 |
| 14 | 100 | 98 | 82 | 98 |
| 19 | 99 | 100 | 100 | 100 |
| 29 | 22 | 100 | 18 | 100 |
| 41 | 100 | 100 | 100 | 100 |
| 46 | 3 | 64 | 5 | 43 |

### Experimental Example 8: Cell death protective effect on lung epithelial cells (MLE-12, rat lung epithelial cells)

In order to identify the protective effect on lung epithelial cells, MLE-12 cells were divided into 96-well plates by 5 × 10³ and cultured for 18 to 24 hours. The compounds of Examples were diluted to final concentrations of 0.03 and 0.3 µM, treated in each well, and incubated for 20 minutes. RSL3 was treated to have final concentrations of 1.0 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 7 showed cell death reduction rates according to the respective concentrations of the compounds of Examples for RSL3 as ferroptosis-inducing substances.

**Table 7: Cell death protective effect in lung epithelial cells (MLE-12) against RSL3**

| [Table 7] | | |
|---|---|---|
| | RSL3, 1 µM | |
| Example | 0.03 µM | 0.3 µM |
| | inhibition (%) | |
| Ferro statin-1 | 25 | 98 |
| 1 | 88 | 100 |
| 2 | 84 | 9 |
| 14 | 50 | 100 |
| 19 | 93 | 100 |
| 29 | 0 | 95 |
| 41 | 95 | 97 |
| 46 | 0 | 7 |

### Experimental Example 9: Pharmacodynamic Comparison of the Compounds of the examples and Comparative Examples

### Measuring the pharmacodynamics of the compound of example 2, N-cyclopentyl-5-(methoxymethyl)-2-phenyl-1H-indol-7-amine

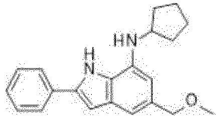

Pharmacodynamic parameters can be represented as maximum concentration in plasma (Cₘₐₓ), time taken until maximum concentration in plasma (Tₘₐₓ), area under the plasma concentration-time curve (AUCₗₐₛₜ and AUC_{inf}), and half-life (t_{1/2}), and the like.

The compound of example 2 was orally administered to ICR mice at a dose of 10 mg/kg, and drug concentration in mouse plasma was measured using HPLC-MS/MS spectroscopy at 0.5, 1, 2, 4, 8 and 24 hours. The mouse brains were removed at 2 or 24 hours, and drug concentration in the brain was measured using HPLC-MS/MS spectroscopy. The area under the plasma concentration v. time curve (AUC_{inf}) was measured to be 4960 ng/mL, and at 2 hours after administration drug concentration in plasma was 546 ng/mL and brain drug concentration was measured at 1043 ng/mL. The brain-plasma drug concentration ratio was 1.91, exhibiting very high exposure in the brain. Further, at 24 hours after administration, for Embodiment 2, drug concentration in plasma was measured to be 4.7 ng/mL and drug concentration in the brain was measured at 5.7 ng/mL. With the brain-plasma drug concentration ratio at 1.23, drug exposure in the brain was still high.

### Measuring the pharmacodynamics of the Comparative Example compound [7-cyclopentylamino]-2-phenyl-1H-indol-5-yl]methanol

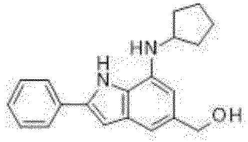

The Comparative Example compound [7-cyclopentylamino]-2-phenyl-1H-indol-5-yl]methanol was synthesized and used in accordance with Korean Registered Patent KR 10-1511771.

The Comparative Example compound was orally administered to ICR mice at a dose of 10 mg/kg, and drug concentration in mouse plasma was measured using HPLC-MS/MS spectroscopy at 0.5, 1, 2, 4, 8 and 24 hours. The mouse brains were removed at 2 or 24 hours, and drug concentration in the brain was measured using HPLC-MS/MS spectroscopy.

For the Comparative Example compound, the area under the plasma concentration v. time curve (AUC_{inf}) was measured to be 5458 ng/mL, and at 2 hours after administration drug concentration in plasma was 1122 ng/mL and brain drug concentration was measured at 95 ng/mL. The brain-plasma drug concentration ratio was 0.08, and further, at 24 hours after administration, no drug concentration was measured in the plasma and brain.

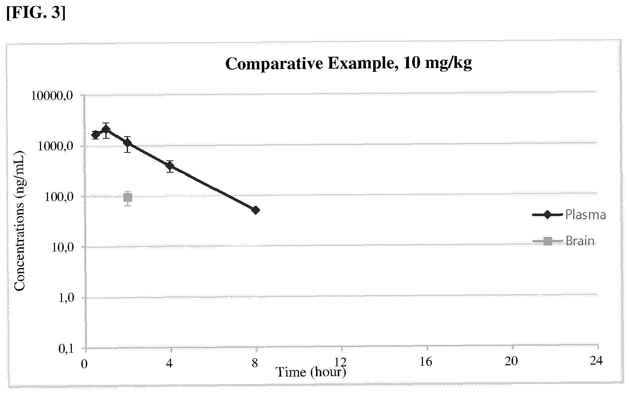

The values for average drug concentration in plasma and the brain v. time following oral administration of the Compound of Example 2 and Comparative Example compound are shown in Table 8, and graphs of these are shown in FIG. 2 and FIG. 3.

**Table 8: Brain/plasma concentration ratios of the compounds of Example 2 and the Comparative Example**

| **[Table 8]** | | | | | | |
|---|---|---|---|---|---|---|
| Test article: | **Example 2** | | | Comparative Example | | |
| Dose (mg/kg): | 10 | | | 10 | | |
| 0.5∼24hr AUCₗₐₛₜ (ng hr/mL) | **4940** | | | **5359** | | |

| Time (hr) | Plasma | Brain | B/P ratio | Plasma | Brain | B/P ratio |
|---|---|---|---|---|---|---|
| **2** | **546.1** | **1043.2** | **1.91** | **1122.4** | **95.1** | 0.08 |
| **24** | **4.7** | **5.7** | **1.23** | **0.0** | **0.0** | **NA** |
| AUCₗₐₛₜ (ng-hr/mL or ng·hr/g) | 6605 | 12581.1 | 1.9 | NA | NA | NA |

| | | | | | | |
|---|---|---|---|---|---|---|
| NT: not tested NA: not available | | | | | | |

### Analysis of the pharmacodynamic results for the compound of Example 2 and the Comparative Example compound

The area under the plasma concentration v. time curve (AUCₗₐₛₜ) for the compounds of Example 2 and the Comparative Example was measured at 4940 ng/mL v. 5359 ng/mL. Similar plasma exposure was exhibited.

However, in the case of the Comparative Example, at 2 hours after administration, drug concentration in plasma was measured at 1122 ng/mL and drug concentration in brain was measured at 95 ng/mL, representing a brain-plasma drug concentration ratio of 0.08. For the compound of example 2, at 2 hours after administration drug concentration in plasma was 546 ng/mL and brain drug concentration was measured at 1043 ng/mL. The brain-plasma drug concentration ratio was 1.91, exhibiting very high exposure in the brain. Further, whereas for the Comparative Example, no drug concentration was measured in the plasma and brain at 24 hours after administration, drug concentration in plasma was measured at 4.7 ng/mL and drug concentration in brain was measured at 5.7 ng/mL for Embodiment 2, giving a brain-plasma drug concentration ratio of 1.23. In-brain drug exposure was still higher than the comparative example.

Therefore, it can be known that the present invention has the characteristics of a compound which maintains fair levels of plasma exposure while having good brain exposure.

## Claims

1. A compound of Formula 1 below, an isomer thereof, a solvate thereof, a hydrate thereof, or a pharmaceutically acceptable salt thereof: 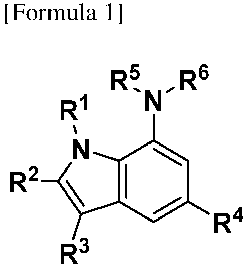 wherein,
R¹ is hydrogen or C₁₋₈ alkyl,
R² is hydrogen, aryl with 5 to 10 atoms, heteroaryl with 5 to 10 atoms, C₁₋₈ alkyl, or C₁₋₈ alkoxy, and R² is unsubstituted or substituted with halogen,
R³ is hydrogen, nitrile, aryl with 5 to 10 atoms, heteroaryl with 5 to 10 atoms, C₁₋₈ alkyl, C₁₋₈ alkoxy, -C(=O)-X, -CH(OH)-X, -CH=CH-C(=O)-X, -CH₂CH₂-C(=O)-X, or - CH=N-X, where the aryl with 5 to 10 atoms, heteroaryl with 5 to 10 atoms, or C₁₋₈ alkyl in R³ is unsubstituted or substituted with a substituent Y,
the substituents X and Y are each independently one or more selected from the group consisting of halogen, hydroxy, C₁₋₈ alkyl, C₁₋₈ haloalkyl, C₁₋₈ alkoxy, C₁₋₃ ester, aryl with 5 to 10 atoms, and heteroaryl with 5 to 10 atoms,
R⁴ is -C₁₋₈ alkylene-O-R⁷, where
R⁷ is C₁₋₈ alkyl or -(CH₂)ₘ-C₃₋₁₀ cycloalkyl, and m is an integer of 1 to 4,
R⁵ and R⁶ are each independently hydrogen, C₁₋₈ alkyl, C₁₋₈ alkoxy, C₃₋₁₀ cycloalkyl, - (CH₂)ₚ-C₃₋₁₀ cycloalkyl, C₃₋₁₀ heterocycloalkyl, -(CH₂)ₚ-C₃₋₁₀ heterocycloalkyl, aryl with 5 to 10 atoms, benzyl or heteroaryl with 5 to 10 atoms, where p is an integer of 1 to 4, and
R⁵ and R⁶ are unsubstituted or substituted with one or more substituents R⁸ selected from the group consisting of halogen, C₁₋₈ alkyl, C₁₋₈ alkoxy, C₁₋₈ haloalkyl, oxo (=O), -C(=O)-C₁₋₈ alkyl, -C(=O)OC₁₋₈ alkyl and -S(=O)₂C₁₋₈ alkyl.

2. The compound according to Claim 1, an isomer thereof, a solvate thereof, a hydrate thereof, or a pharmaceutically acceptable salt thereof, wherein
the compound of Formula 1 is represented by Formula 2 below: 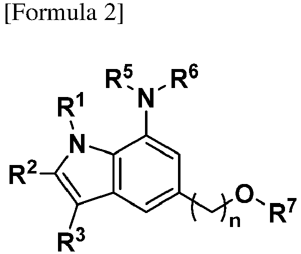
wherein, R¹ to R³ and R⁵ to R⁷ are each as defined in Claim 1, and n is an integer of 1 to 4.

3. The compound according to Claim 1, an isomer thereof, a solvate thereof, a hydrate thereof, or a pharmaceutically acceptable salt thereof, wherein
R¹ is hydrogen or C₁₋₆ alkyl,
R² is hydrogen, aryl with 5 to 8 atoms, heteroaryl with 5 to 8 atoms containing one or more heteroatoms of N, O, and S, C₁₋₆ alkyl, or C₁₋₆ alkoxy, and R² is unsubstituted or substituted with halogen,
R³ is hydrogen, nitrile, aryl with 5 to 8 atoms, heteroaryl with 5 to 8 atoms containing one or more heteroatoms of N, O, and S, C₁₋₆ alkyl, C₁₋₆ alkoxy, -C(=O)-X, -CH(OH)-X, - CH=CH-C(=O)-X, -CH₂CH₂-C(=O)-X, or -CH=N-X, where the aryl with 5 to 8 atoms, heteroaryl with 5 to 8 atoms, or C₁₋₆ alkyl in R³ is unsubstituted or substituted with a substituent Y,
the substituents X and Y are each independently one or more selected from the group consisting of halogen, hydroxy, C₁₋₆ alkyl, C₁₋₆ haloalkyl, C₁₋₆ alkoxy, C₁₋₃ ester, aryl with 5 to 8 atoms, and heteroaryl with 5 to 8 atoms containing one or more heteroatoms of N, O, and S,
R⁴ is -C₁₋₈ alkylene-O-R⁷, where
R⁷ is C₁₋₈ alkyl or -(CH₂)ₘ-C₃₋₇ cycloalkyl, and m is an integer of 1 to 4,
R⁵ and R⁶ are each independently hydrogen, C₁₋₆ alkyl, C₁₋₆ alkoxy, C₃₋₇ cycloalkyl, - (CH₂)ₚ-C₃₋₇ cycloalkyl, C₃₋₇ heterocycloalkyl, -(CH₂)ₚ-C₃₋₇ heterocycloalkyl, aryl with 5 to 8 atoms, benzyl, or heteroaryl with 5 to 8 atoms containing one or more heteroatoms of N, O, and S, where p is an integer of 1 to 4, and
R⁵ and R⁶ are unsubstituted or substituted with one or more substituents R⁸ selected from the group consisting of halogen, C₁₋₆ alkyl, C₁₋₆ alkoxy, C₁₋₆ haloalkyl, oxo (=O), -C(=O)-C₁₋₆ alkyl, -C(=O)OC₁₋₆ alkyl, and -S(=O)₂C₁₋₆ alkyl.

4. The compound according to Claim 1, an isomer thereof, a solvate thereof, a hydrate thereof, or a pharmaceutically acceptable salt thereof, wherein
R² is hydrogen or aryl with 5 to 10 atoms.

5. The compound according to Claim 1, an isomer thereof, a solvate thereof, a hydrate thereof, or a pharmaceutically acceptable salt thereof, wherein
R³ is aryl with 5 to 10 atoms or heteroaryl with 5 to 10 atoms, which is unsubstituted or substituted with one or more substituents Y of C₁₋₃ ester; or
R³ is C₁₋₈ alkyl, which is unsubstituted or substituted with one or more substituents Y selected from the group consisting of hydroxy, C₁₋₈ haloalkyl, and C₁₋₈ alkoxy.

6. The compound according to Claim 1, an isomer thereof, a solvate thereof, a hydrate thereof, or a pharmaceutically acceptable salt thereof, wherein
when R³ is -C(=O)-X, X is C₁₋₈ haloalkyl;
when R³ is -CH=CH-C(=O)-X, X is C₁₋₈ alkoxy, or aryl with 5 to 10 atoms;
when R³ is -CH₂CH₂-C(=O)-X, X is C₁₋₈ alkoxy, or hydroxy; or
when R³ is -CH=N-X, X is C₁₋₈ alkoxy.

7. The compound according to Claim 1, an isomer thereof, a solvate thereof, a hydrate thereof, or a pharmaceutically acceptable salt thereof, wherein
R⁵ and R⁶ are simultaneously C₃₋₇ cycloalkyl; or
any one of R⁵ and R⁶ is hydrogen, and the other is C₁₋₈ alkyl, C₁₋₈ alkoxy, C₃₋₁₀ cycloalkyl, -(CH₂)ₚ-C₃₋₁₀ cycloalkyl, C₃₋₁₀ heterocycloalkyl, -(CH₂)ₚ-C₃₋₁₀ heterocycloalkyl, aryl with 5 to 10 atoms, benzyl, or heteroaryl with 5 to 10 atoms, and p is an integer of 1 to 4, where the heterocycloalkyl and heteroaryl contain one or more heteroatoms of N, O, and S.

8. The compound according to Claim 1, an isomer thereof, a solvate thereof, a hydrate thereof, or a pharmaceutically acceptable salt thereof, wherein
when one or more of R⁵ and R⁶ are C₁₋₈ alkyl, the substituent R⁸ is C₁₋₈ alkoxy;
when one or more of R⁵ and R⁶ are C₃₋₁₀ heterocycloalkyl containing a heteroatom of N, the substituent R⁸ is selected from the group consisting of -C(=O)-C₁₋₆ alkyl, -C(=O)OC₁₋₆ alkyl and -S(=O)₂C₁₋₆ alkyl; or
when one or more of R⁵ and R⁶ are C₃₋₁₀ heterocycloalkyl containing a heteroatom of S, the substituent R⁸ is oxo (=O).

9. The compound according to Claim 1, an isomer thereof, a solvate thereof, a hydrate thereof, or a pharmaceutically acceptable salt thereof, wherein the compound is any one selected from the following compound group: <1>5-(methoxymethyl)-2-phenyl-N-tetrahydropyran-4-yl-1H-indol-7-amine; <2>N-cyclopentyl-5-(methoxymethyl)-2-phenyl-1H-indol-7-amine; <3>5-(methoxymethyl)-2-phenyl-N-(tetrahydropyran-4-ylmethyl)-1H-indol-7-amine; <4>N-(1,1-dioxothian-4-yl)-5-(methoxymethyl)-2-phenyl-1H-indol-7-amine; <5>5-(methoxymethyl)-N-(1-methylsulfonyl-4-piperidyl)-2-phenyl-1H-indol-7-amine; <6>1-[4-[[5-(methoxymethyl)-2-phenyl-1H-indol-7-yl]amino]-1-piperidyl]ethanone; <7>N-(4,4-difluorocyclohexyl)-5-(methoxymethyl)-2-phenyl-1H-indol-7-amine; <8>N-benzyl-5-(methoxymethyl)-2-phenyl-1H-indol-7-amine; <9>tert-butyl 4-[[5-(methoxymethyl)-2-phenyl-1H-indol-7-yl]amino]-piperidine-1-carboxylate; <10> N-isopropyl-5-(methoxymethyl)-2-phenyl-1H-indol-7-amine; <11>5-(methoxymethyl)-N-(1-methyl-4-piperidyl)-2-phenyl-1H-indol-7-amine; <12>N-(2-methoxyethyl)-5-(methoxymethyl)-2-phenyl-1H-indol-7-amine; <13>5-(methoxymethyl)-N-(3-methoxypropyl)-2-phenyl-1H-indol-7-amine; <14>5-(methoxymethyl)-N-(1-oxothian-4-yl)-2-phenyl-1H-indol-7-amine; <15 >5-(tert-butoxymethyl)-2-phenyl-N-tetrahydropyran-4-yl-1H-indol-7- amine; <16>5-(tert-butoxymethyl)-N-cyclopentyl-2-phenyl-1H-indol-7-amine; <17>5-(tert-butoxymethyl)-N,N-dicyclopentyl-2-phenyl-1H-indol-7-amine; <18>5-(methoxymethyl)-3-phenyl-N-tetrahydropyran-4-yl-1H-indol-7-amine; <19>N-cyclopentyl-5-(ethoxymethyl)-2-phenyl-1H-indol-7-amine; <20>5-(ethoxymethyl)-2-phenyl-N-(tetrahydro-2H-pyran-4-yl)-1H-indol-7-amine; <21>N-cyclopentyl-5-(isopropoxymethyl)-2-phenyl-1H-indol-7-amine; <22>5-(isopropoxymethyl)-2-phenyl-N-(tetrahydro-2H-pyran-4-yl)-1H-indol-7-amine; <23>7-(cyclopentylamino)-5-(ethoxymethyl)-2-phenyl-1H-indole-3-carbonitrile; <24>5-(ethoxymethyl)-2-phenyl-7-((tetrahydro-2H-pyran-4-yl)amino)-1H-indole-3-carbonitrile; <25>(E)-7-(cyclopentylamino)-5-(ethoxymethyl)-2-phenyl-1H-indole-3-carbaldehyde O-methyl oxime; <26>(E)-7-(bis(tetrahydro-2H-pyran-4-yl)amino)-5-(ethoxymethyl)-2-phenyl-1H-indole-3-carbaldehyde O-methyl oxime; <27>(E)-5-(ethoxymethyl)-2-phenyl-7-(tetrahydro-2H-pyran-4-yl)amino)-1H-indole-3-carbaldehyde O-methyl oxime; <28>N-cyclopentyl-5-(ethoxymethyl)-3-(methoxymethyl)-1-methyl-2-phenyl-1H-indol-7-amine; <29>5-(ethoxymethyl)-3-(methoxymethyl)-1-methyl-2-phenyl-N-(tetrahydro-2H-pyran-4-yl)-1H-indol-7-amine; <30>methyl (E)-3-(7-(cyclopentylamino)-5-(ethoxymethyl)-2-phenyl-1H-indol-3-yl)acrylate; <31>methyl (E)-3-(5-(ethoxymethyl)-2-phenyl-7-((tetrahydro-2H-pyran-4-yl)amino)-1H-indol-3-yl)acrylate; <32>1-(7-(cyclopentylamino)-5-(ethoxymethyl)-2-phenyl-1H-indol-3-yl)-2,2,2-trifluoroethan-1-one; <33> 1-(5-(ethoxymethyl)-2-phenyl-7-((tetrahydro-2H-pyran-4-yl)amino)-1H-indol-3-yl)-2,2,2-trifluoro-1-one; <34>(E)-3-(7-(cyclopentylamino)-5-(ethoxymethyl)-2-phenyl-1H-indol-3-yl)-1-phenylprop-2-en-1-one; <35>(E)-3-(5-(ethoxymethyl)-2-phenyl-7-((tetrahydro-2H-pyran-4-yl)amino)-1H-indol-3-yl)-1-phenylprop-2-en-1-one; <36>1-(7-(cyclopentylamino)-5-(ethoxymethyl)-2-phenyl-1H-indol-3-yl)-2,2,2-trifluoroethan-1-ol; <37>1-(5-(ethoxymethyl)-2-phenyl-7-((tetrahydro-2H-pyran-4-yl)amino)-1H-indol-3-yl)-2,2,2-trifluoro-1-ol; <38>methyl 3-(7-(cyclopentylamino)-5-(ethoxymethyl)-2-phenyl-1H-indol-3-yl)propanoate; <39>methyl 3-(5-(ethoxymethyl)-2-phenyl-7-((tetrahydro-2H-pyran-4-yl)amino)-1H-indol-3-yl)propanoate; <40>3-(7-(cyclopentylamino)-5-(ethoxymethyl)-2-phenyl-1H-indol-3-yl)propanoic acid; <41>5-(2-methoxyethyl)-2-phenyl-N-(tetrahydro-2H-pyran-4-yl)-1H-indol-7-amine; <42> N-cyclopentyl-5-(2-methoxyethyl)-2-phenyl-1H-indol-7-amine; <43>ethyl 2-(7-(cyclopentylamino)-5-(ethoxymethyl)-2-phenyl-1H-indol-3-yl)-1H-imidazole-1-carboxylate; <44>ethyl 2-(5-(ethoxymethyl)-2-phenyl-7-((tetrahydro-2H-pyran-4-yl)amino)-1H-indol-3-yl)-1H-imidazole-1-carboxylate; <45>N-cyclopentyl-5-(ethoxymethyl)-3-(1H-imidazol-2-yl)-2-phenyl-1H-indol-7-amine; <46>5-(ethoxymethyl)-3-(1H-imidazol-2-yl)-2-phenyl-N-(tetrahydro-2H-pyran-4-yl)-1H-indol-7-amine; <47>2-phenyl-5-(propoxymethyl)-N-(tetrahydro-2H-pyran-4-yl)-1H-indol-7-amine; <48>5-((cyclopropylmethoxy)methyl)-2-phenyl-N-(tetrahydro-2H-pyran-4-yl)-1H-indol-7-amine; <49>5-(ethoxymethyl)-2-phenyl-N-((tetrahydro-2H-pyran-4-yl)methyl)-1H-indol-7-amine; <50>5-(3-methoxypropyl)-2-phenyl-N-(tetrahydro-2H-pyran-4-yl)-1H-indol-7-amine; and <51>5-(3-ethoxypropyl)-2-phenyl-N-(tetrahydro-2H-pyran-4-yl)-1H-indol-7-amine.

10. A method for preparing a compound of Formula 1 comprising steps of:
reacting a compound of Formula 3 and HO-R⁷ to prepare a compound of Formula 4;
reducing the compound of Formula 4; and
preparing the compound of Formula 1 from the reduced compound of Formula 4: 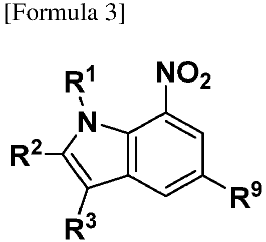 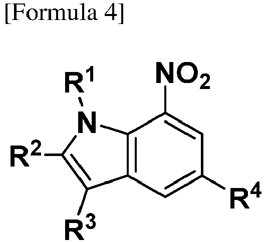 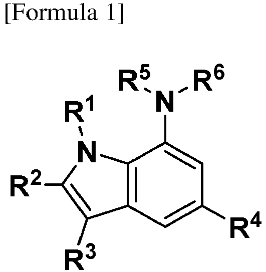
wherein, R⁹ is -C₁₋₈ alkylene-LG, where LG is a leaving group, and R¹ to R⁷ are each the same as defined in Claim 1.

11. A pharmaceutical composition for prevention or treatment of cell necrosis or ferroptosis-related diseases comprising the compound of Formula 1 of Claim 1, and an isomer thereof, a hydrate thereof, a solvate thereof, or a pharmaceutically acceptable salt thereof, as an active ingredient; and a pharmaceutically acceptable carrier.

12. A pharmaceutical composition for prevention or treatment of cell necrosis or ferroptosis-related diseases selected from the following group, the composition comprising the compound of Formula 1 of Claim 1, an isomer thereof, a hydrate thereof, a solvate thereof, or a pharmaceutically acceptable salt thereof, as an active ingredient; and a pharmaceutically acceptable carrier:
Acute or chronic hepatic disease, dementia, Parkinson's disease, Huntington's disease, ischemic disease, diabetes mellitus, pancreatitis, bacterial or viral sepsis, necrotizing procolitis, cystic fibrosis, rheumatoid arthritis, degenerative arthritis, nephropathy, bacterial infection, viral infection, multiple sclerosis, leukemia, lymphoma, neonatal respiratory distress syndrome, asphyxia, tuberculosis, endometriosis, angiasthenia, psoriasis, chilblain, steroid treatment complications, gangrene, pressure sores, hemoglobinuria, burns, hyperthermia, Crohn's disease, celiac disease, compartment syndrome, spinal cord injury, glomerulonephritis, renal ischemia-reperfusion injury, muscular dystrophy, mycoplasma disease, anthrax, Andersen's disease, congenital mitochondrial disease, phenylketonuria, placental infarction, syphilis, osteonecrosis; alcoholism, the exposure to cocaine, antibiotics, anti-cancer agent, NSAID, cyclosporine, chemical toxins, poison gas, agrochemicals, heavy metals, or injury due to the exposure to radioactivity/UV and associated necrosis thereof, acute/chronic renal disease, traumatic brain damage, amyotrophic lateral sclerosis, necrotizing colitis, viral infection, skin disease including psoriasis and allergic dermatitis, and organ preservation/organ transplant, chronic inflammatory pulmonary disease including acute lung injury syndrome / acute pulmonary disease, pneumonia, tuberculosis, asthma, pulmonary hypertension, chronic obstructive pulmonary disease, idiopathic pulmonary fibrosis and cystic fibrosis, demyelinating diseases including demyelination and amyotrophic lateral sclerosis (ALS), hypertension including pulmonary hypertension, stroke, prion disease, epilepsy, ataxia, migraine, reduced cognitive skill, seizure, tremors, psychiatric disorders , insulin resistance, hyperlipidemia, atherosclerosis, inflammatory bowel disease (IBD) including Crohn's Disease and ulcerative colitis, cancers and metastasis of cancer, visual impairment-associated disease including macular degeneration, retinitis pigmentosa, optic neuropathy, cataracts, glaucoma, anemia, cholestasis, hypoparathyroidism, pancytopenia, pancreatic disorder, lactic acidosis, lactacidaemia, hearing loss, short stature, intestinal obstruction, cardiac conduction defect, cardiomyopathy, endometriosis, infertility, early menopause, muscular atrophy diseases including limb girdle/Becker muscular dystrophy (GGMD/BMD) and Duchenne muscular dystrophy (DMD), aging and aging-related diseases, and mucositis.

13. The pharmaceutical composition for prevention or treatment of cell necrosis or ferroptosis-related diseases according to Claim 11, wherein
the disease is neurodegenerative disease, liver disease, kidney disease, stroke, myocardial infarction, ocular disease or lung disease.

14. The pharmaceutical composition for prevention or treatment of cell necrosis or ferroptosis-related diseases according to Claim 13, wherein
the neurodegenerative disease is one or more selected from the group consisting of Alzheimer's disease, Parkinson's disease, epilepsy, Huntington's disease, amyotrophic lateral sclerosis, Friedreich's ataxia, multiple sclerosis, CMT (Charcot-Marie-Tooth) disease, dementia with Lewy bodies, and traumatic brain injury.

15. A method for preventing or treating cell necrosis or ferroptosis-related diseases comprising a step of administering the compound of Formula 1 according to Claim 1, an isomer thereof, a hydrate thereof, a solvate thereof, or a pharmaceutically acceptable salt thereof, to a subject in need thereof in a pharmaceutically effective amount.

16. The method for preventing or treating cell necrosis or ferroptosis-related diseases according to Claim 15, wherein
the disease involves lipid peroxidation.

17. A method for suppressing ferroptosis comprising a step of administering the compound of Formula 1 according to Claim 1, an isomer thereof, a hydrate thereof, a solvate thereof, or a pharmaceutically acceptable salt thereof, to a subject in need thereof in a pharmaceutically effective amount.

18. A method for reducing reactive oxygen species (ROS) in cells comprising a step of contacting the cells with the compound of Formula 1 according to Claim 1, an isomer thereof, a hydrate thereof, a solvate thereof, or a pharmaceutically acceptable salt thereof.

19. A use of the compound of Formula 1 according to Claim 1, an isomer thereof, a solvate thereof, or a pharmaceutically acceptable salt thereof, for prevention or treatment of cell necrosis or ferroptosis-related diseases.
